# EUROPEAN PATENT APPLICATION

(11) **EP 3 892 637 A1**
(43) Date of publication of application: **13.10.2021**
(21) Application number: 19894109.8
(22) Date of filing: 03.12.2019
(51) Int. Cl.: C07K 16/28, A61K 39/395, A61K 35/00

(54) **CD47 ANTIBODY, PREPARATION METHOD THEREFOR AND USES THEREOF**

(30) Priority: 03.12.2018 CN 201811467637
(71) Applicant: Shanghai Pharmaexplorer Co., Ltd., Shanghai 201210 (CN); Pharmaexplorer Limited, Road Town, Tortola (VG)
(72) Inventor: LIU, Lile, Shanghai 201210 (CN); YANG, Tatchi Teddy, Shanghai 201210 (CN); KHAN, Shireen, Shanghai 201210 (CN); DUAN, Qing, Shanghai 201210 (CN); GAO, Jing, Shanghai 201210 (CN); YAO, Jingyun, Shanghai 201210 (CN); XU, Zhiqiang, Shanghai 201210 (CN); MENG, Yali, Shanghai 201210 (CN); WANG, Liang, Shanghai 201210 (CN); PAN, Mingming, Shanghai 201210 (CN); ZHOU, Wenming, Shanghai 201210 (CN); XIA, Ke, Shanghai 201210 (CN); GUO, Cuicui, Shanghai 201210 (CN); TONG, Guozhen, Shanghai 201210 (CN); WANG, Lu, Shanghai 201210 (CN); ZHAO, Jiaxun, Shanghai 201210 (CN); WANG, Dongxu, Shanghai 201210 (CN); DAI, Chaohui, Shanghai 201210 (CN); WANG, Mengying, Shanghai 201210 (CN)
(74) Representative: Rondano, Davide
(86) International application number: PCT/CN2019/122777
(87) International publication number: WO 2020/114399

(57) **Abstract**

An antibody targeting CD47, a preparation method therefor and uses thereof. A novel mouse or human-mouse chimeric antibody targeting CD47. A method for preparing the monoclonal antibody. The monoclonal antibody of the present invention can bind CD47 antigen with high specificity, has high affinity and significant activities, such as anti-tumor activity.

## Description

### Technical field

The present invention belongs to the field of biomedicine, and specifically relates to a CD47 antibody and the preparation method and application thereof.

### Background

CD47, also known as integrin-associated protein (IAP), ovarian cancer antigen OA3, Rh-associated antigen and MER6, is a transmembrane glycoprotein widely expressed on the cell surface and belongs to the immunoglobulin superfamily. The molecular weight of CD47 is between 47-55kD, including an extracellular Ig-like variable domain, five highly hydrophobically extended transmembrane fragments, and a selectively spliced carboxyl terminal cytoplasmic tail region.

SIRPα (signal regulatory protein α) is also a transmembrane protein, which is mainly expressed on the surface of macrophages, dendritic cells and nerve cells. Its extracellular domain contains three immunoglobulin superfamily-like regions, of which the N-terminal region mediates the binding to CD47, and regulates cell migration and phagocytosis, immune self-stabilization and neural network through the contact between cell surface receptors and ligands.

CD47 can generate inhibitory signals by combining with SIRPα to reduce the activity of macrophages and inhibit the non-specific immune system. For example, the decrease of CD47 expression on the surface of red blood cells can enhance the phagocytosis of red blood cells by macrophages in red marrow, which is an important pathogenic factor of hemolytic anemia. High expression of CD47 can inhibit phagocytosis of macrophages. For example, CD47 is temporarily up-regulated before and during normal hematopoietic stem cell migration. In the study of malignant diseases such as leukemia, non-Hodgkin's lymphoma, bladder cancer and breast cancer, it is found that there is an increase in CD47 in tumor cells, and the high expression of CD47 indicates poor clinical prognosis. Tumor cells may evade tumor immunity with the help of "don't eat me" signal. By using anti-CD47 antibody to block CD47-SIRPα pathway, cell phagocytosis is mediated, and tumor cells can be targeted to kill.

Majeti et al. proved *in vivo* and *in vitro* that CD47 monoclonal antibody with the property of blocking can promote macrophages to phagocytize tumor cells, eliminate acute myeloid leukemia (AML) transplanted in mice, and target to clear leukemia stem cells (LSC). In the study of CD47 monoclonal antibody on acute lymphoblastic leukemia (ALL) by Chao et al., it can effectively remove or reduce the tumor of ALL cells in peripheral blood and bone marrow of mice at the original transplantation site, and can also eliminate the tumor spreading in spleen, liver and other sites, thus achieving the curative effect of long-term survival and inhibiting tumor recurrence. The anti-tumor therapy of anti-CD47 monoclonal antibody is also related to other mechanisms, including antibody-dependent cell-mediated cytotoxicity, direct induction of tumor cell apoptosis, activation of killer T cells to kill tumor cells and other factors.

At present, three drugs against CD47 have entered phase I clinical trials, including two monoclonal antibodies (Hu5F9-G4, CC-90002) and one fusion protein (TTI-621), covering a variety of hematological tumors and solid tumors. Hu5F9-G4 and TTI-621 showed different safety in published phase I clinical results. All the 16 patients using Hu5F9-G4 developed anemia of different degrees and hyperbilirubinemia in some cases. However, the occurrence of low platelets was not reported. On the contrary, in the TTI-621 experimental group, 4 of 5 patients had G3 and G4 grade thrombocytopenia symptoms, but all 11 patients had stable hemoglobin and no anemia was reported. In addition, there are factors that overestimate the efficacy in the preclinical model of anti-CD47 therapy, and the efficacy varies greatly among different tumors. There is no denying that CD47 has become a hot target in immunotherapy in recent years. It has many advantages in preventing tumor recurrence and treating advanced cancer and its complications. Therefore, it is urgent to develop safe and specific CD47 therapeutic antibodies.

### Summary of the invention

In order to overcome the current lack of safe and highly specific CD47 antibodies, the present invention provides a CD47 antibody with high affinity and strong specificity, and a preparation method and application thereof.

In the first aspect of the present invention, it provides a heavy chain variable region of an antibody having complementarity determining regions or CDRs selected from the group consisting of:
VH-CDR1 shown in SEQ ID NO.10n+3,
VH-CDR2 shown in SEQ ID NO.10n+4, and
VH-CDR3 shown in SEQ ID NO.10n+5;
wherein, each n is independently 0, 1, 2, 3, 4, 5, 6, or 7;
wherein any one of the above amino acid sequences further comprises a derivative sequence which is obtained through optional addition, deletion, modification and/or substitution of at least one amino acid and is capable of retaining CD47 binding affinity.

In another preferred embodiment, the heavy chain variable region has the amino acid sequence shown in SEQ ID NO. 10 n +1, where n is 0, 1, 2, 3, 4, 5, 6, 7, or 8.

In another preferred embodiment, the heavy chain variable region has the amino acid sequence as shown in SEQ ID NO. 1.

In another preferred embodiment, the heavy chain variable region has the amino acid sequence as shown in SEQ ID NO. 11.

In another preferred embodiment, the heavy chain variable region has the amino acid sequence as shown in SEQ ID NO. 21.

In another preferred embodiment, the heavy chain variable region has the amino acid sequence as shown in SEQ ID NO. 31.

In another preferred embodiment, the heavy chain variable region has the amino acid sequence as shown in SEQ ID NO. 81.

In the second aspect of the present invention, it provides a heavy chain of an antibody having the heavy chain variable region according to the first aspect of the present invention.

In another preferred embodiment, the heavy chain further comprises a heavy chain constant region.

In another preferred embodiment, the heavy chain constant region is of human or murine origin.

In another preferred embodiment, the heavy chain constant region is a human antibody heavy chain IgG4 constant region.

In another preferred embodiment, the heavy chain constant region is a human antibody heavy chain IgG4PE constant region.

In the third aspect of the present invention, it provides a light chain variable region of an antibody having complementarity determining regions or CDRs selected from the group consisting of:
VL-CDR1 shown in SEQ ID NO. 10n +8,
VL-CDR2 shown in SEQ ID NO. 10n +9, and
VL-CDR3 shown in SEQ ID NO. 10n +10;
wherein, each n is independently 0, 1, 2, 3, 4, 5, 6, or 7;
wherein any one of the above amino acid sequences further comprises a derivative sequence which is obtained through optional addition, deletion, modification and/or substitution of at least one amino acid and is capable of retaining CD47 binding affinity.

In another preferred embodiment, the light chain variable region has the amino acid sequence shown in SEQ ID NO. 10 n +6, where n is 0, 1, 2, 3, 4, 5, 6, or 7.

In another preferred embodiment, the light chain variable region has the amino acid sequence shown in SEQ ID NO. 6.

In another preferred embodiment, the light chain variable region has the amino acid sequence shown in SEQ ID NO. 16.

In another preferred embodiment, the light chain variable region has the amino acid sequence shown in SEQ ID NO. 26.

In another preferred embodiment, the light chain variable region has the amino acid sequence shown in SEQ ID NO. 36.

In another preferred embodiment, the light chain variable region has the amino acid sequence shown in SEQ ID NO. 82.

In the fourth aspect of the present invention, there is provided a light chain of an antibody having the light chain variable region according to the third aspect of the present invention.

In another preferred embodiment, the light chain further comprises a light chain constant region.

In another preferred embodiment, the light chain constant region is of human or murine origin.

In another preferred embodiment, the light chain constant region is human antibody light chain kappa constant region.

In the fifth aspect of the present invention, it provides an antibody having:
(1) the heavy chain variable region according to the first aspect of the present invention; and/or
(2) the light chain variable region according to the third aspect of the present invention;
or the antibody has: the heavy chain according to the second aspect of the present invention; and/or the light chain according to the fourth aspect of the present invention,
wherein any one of the above amino acid sequences further comprises a derivative sequence which is obtained through optional addition, deletion, modification and/or substitution of at least one amino acid and is capable of retaining CD47 binding affinity.

In another preferred embodiment, the amino acid sequence of any of the above-mentioned CDRs includes a derivative CDR sequence with 1, 2 or 3 amino acids added, deleted, modified and/or substituted, and the derivative antibody comprising the VH and VL containing the derivative CDR sequence can retain the binding affinity to CD47.

In another preferred embodiment, the ratio (F1/F0) of the binding affinity F1 between the derivatized antibody and CD47 to the binding affinity F0 between the corresponding non-derivatized antibody and CD47 is 0.5-2, preferably 0.7-1.5, and more preferably 0.8-1.2.

In another preferred embodiment, the number of added, deleted, modified and/or substituted amino acids is 1-5 (such as 1-3, preferably 1-2, more preferably 1).

In another preferred embodiment, the derivative sequence with at least one amino acid added, deleted, modified, and/or substituted, which can retain the binding affinity to CD47, is an amino acid sequence having a homology or sequence identity of at least 96%.

In another preferred embodiment, the antibody further comprises a heavy chain constant region and/or a light chain constant region.

In another preferred embodiment, the heavy chain constant region is of human origin, and/or the light chain constant region is of human origin.

In another preferred embodiment, the heavy chain constant region is a human antibody heavy chain IgG4 constant region, and the light chain constant region is a human antibody light chain kappa constant region.

In another preferred embodiment, the heavy chain constant region is a human antibody heavy chain IgG4PE constant region, and the light chain constant region is a human antibody light chain kappa constant region.

In another preferred embodiment, the heavy chain variable region of the antibody further comprises a human-derived framework region, and/or the light chain variable region of the antibody further comprises a human-derived framework region.

In another preferred embodiment, the heavy chain variable region of the antibody further comprises a murine-derived framework region, and/or the light chain variable region of the antibody further comprises a murine-derived framework region.

In another preferred embodiment, the antibody is selected from the group consisting of an animal-derived antibody, a chimeric antibody, a humanized antibody, a fully human antibody, or a combination thereof.

In another preferred embodiment, the ratio (Z1/Z0) of the immunogenicity Z1 of the chimeric antibody in human to the immunogenicity Z0 of the non-chimeric antibody (such as murine-derived antibody) in human is 0-0.5, preferably 0-0.2, more preferably 0-0.05 (e.g. 0.001-0.05).

In another preferred embodiment, the antibody is a partially or fully humanized or fully human monoclonal antibody.

In another preferred embodiment, the antibody is a double chain antibody or a single chain antibody.

In another preferred embodiment, the antibody is a full-length antibody protein or an antigen-binding fragment.

In another preferred embodiment, the antibody is a bispecific antibody or a multispecific antibody.

In another preferred embodiment, the antibody is in the form of a drug conjugate.

In another preferred embodiment, the antibody has one or more properties selected from the group consisting of:
(a) inhibiting tumor cell migration or metastasis;
(b) inhibiting tumor growth.

In another preferred embodiment, the antibody comprises the heavy chain variable region according to the first aspect of the present invention and the light chain variable region according to the third aspect of the present invention;
wherein, the heavy chain variable region and the light chain variable region comprise CDRs selected from the following group:

| VH-CDR1 Sequence number | VH-CDR2 Sequence number | VH-CDR3 Sequence number | VL-CDR1 Sequence number | VL-CDR2 Sequence number | VL-CDR3 Sequence number |
|---|---|---|---|---|---|
| 3 | 4 | 5 | 8 | 9 | 10 |
| 13 | 14 | 15 | 18 | 19 | 20 |
| 23 | 24 | 25 | 28 | 29 | 30 |
| 33 | 34 | 35 | 38 | 39 | 40 |
| 43 | 44 | 45 | 48 | 49 | 50 |
| 53 | 54 | 55 | 58 | 59 | 60 |
| 63 | 64 | 65 | 68 | 69 | 70 |
| 73 | 74 | 75 | 78 | 79 | 80 |

wherein any one of the above amino acid sequences further comprises a derivative sequence which is obtained through optional addition, deletion, modification and/or substitution of at least one amino acid and is capable of retaining CD47 binding affinity.

In another preferred embodiment, the antibody comprises the heavy chain variable region according to the first aspect of the present invention and the light chain variable region according to the third aspect of the present invention; wherein,
the heavy chain variable region comprises the following three complementary determining regions or CDRs:
   VH-CDR1 shown in SEQ ID NO. 3,
   VH-CDR2 shown in SEQ ID NO. 4, and
   VH-CDR3 shown in SEQ ID NO. 5;
the light chain variable region comprises the following three complementary determining regions or CDRs:
   VL-CDR1 shown in SEQ ID NO. 8,
   VL-CDR2 shown in SEQ ID NO. 9, and
   VL-CDR3 shown in SEQ ID NO. 10;
   or
the heavy chain variable region comprises the following three complementary determining regions or CDRs:
   VH-CDR1 shown in SEQ ID NO. 13,
   VH-CDR2 shown in SEQ ID NO. 14, and
   VH-CDR3 shown in SEQ ID NO. 15;
the light chain variable region comprises the following three complementary determining regions or CDRs:
   VL-CDR1 shown in SEQ ID NO. 18,
   VL-CDR2 shown in SEQ ID NO. 19, and
   VL-CDR3 shown in SEQ ID NO. 20;
   or
the heavy chain variable region comprises the following three complementary determining regions or CDRs:
   VH-CDR1 shown in SEQ ID NO. 23,
   VH-CDR2 shown in SEQ ID NO. 24, and
   VH-CDR3 shown in SEQ ID NO. 25;
the light chain variable region comprises the following three complementary determining regions or CDRs:
   VL-CDR1 shown in SEQ ID NO. 28,
   VL-CDR2 shown in SEQ ID NO. 29, and
   VL-CDR3 shown in SEQ ID NO. 30;
   or
the heavy chain variable region comprises the following three complementary determining regions or CDRs:
   VH-CDR1 shown in SEQ ID NO. 33,
   VH-CDR2 shown in SEQ ID NO. 34, and
   VH-CDR3 shown in SEQ ID NO. 35;
the light chain variable region comprises the following three complementary determining regions or CDRs:
   VL-CDR1 shown in SEQ ID NO. 38,
   VL-CDR2 shown in SEQ ID NO. 39, and
   VL-CDR3 shown in SEQ ID NO. 40.

In another preferred embodiment, the heavy chain variable region of the antibody comprises the amino acid sequence shown in SEQ ID NO. 1, 11, 21, 31, 41, 51, 61, or 71; and/or the light chain variable region of the antibody comprises the amino acid sequence shown in SEQ ID NO. 6, 16, 26, 36, 46, 56, 66, or 76.

In another preferred embodiment, the antibody is a humanized antibody, and the heavy chain variable region of the antibody comprises the amino acid sequence shown in SEQ ID NO. 81, and/or the light chain variable region of the antibody comprises the amino acid sequence shown in SEQ ID NO. 82.

In another preferred embodiment, the heavy chain variable region of the antibody comprises the amino acid sequence shown in SEQ ID NO. 1; and the light chain variable region of the antibody comprises the amino acid sequence shown in SEQ ID NO. 6.

In another preferred embodiment, the heavy chain variable region of the antibody comprises the amino acid sequence shown in SEQ ID NO. 11; and the light chain variable region of the antibody comprises the amino acid sequence shown in SEQ ID NO. 16.

In another preferred embodiment, the heavy chain variable region of the antibody comprises the amino acid sequence shown in SEQ ID NO. 21; and the light chain variable region of the antibody comprises the amino acid sequence shown in SEQ ID NO. 26.

In another preferred embodiment, the heavy chain variable region of the antibody comprises the amino acid sequence shown in SEQ ID NO. 31; and the light chain variable region of the antibody comprises the amino acid sequence shown in SEQ ID NO. 36.

In another preferred embodiment, the antibody is selected from the group consisting of:

| Antibody number | Clone number | VH Sequence number | VL Sequence number |
|---|---|---|---|
| 1 | 4D10B11 | 1 | 6 |
| 2 | 29A03NA | 11 | 16 |
| 3 | 20H4G5 | 21 | 26 |
| 4 | 54G8G6 | 31 | 36 |
| 5 | 132D1E5 | 41 | 46 |
| 6 | 25E3B5 | 51 | 56 |
| 7 | 51E2F11 | 61 | 66 |
| 8 | 95E2D10 | 71 | 76. |

In another preferred embodiment, the amino acid sequence of the heavy chain variable region has a sequence homology or identity of at least 80%, 85%, 90%, 91%, 92%, 93%, 94%, 95%, 96%, 97%, 98% or 99% with the amino acid sequence shown in SEQ ID NO. 1, 11, 21, 31, 41, 51, 61, or 71 in the sequence listing.

In another preferred embodiment, the amino acid sequence of the light chain variable region has a sequence homology or identity of at least 80%, 85%, 90%, 91%, 92%, 93%, 94%, 95%, 96%, 97%, 98% or 99% with the amino acid sequence shown in SEQ ID NO. 6, 16, 26, 36, 46, 56, 66, or 76 in the sequence listing.

In the sixth aspect of the present invention, there is provided a recombinant protein comprising:
(i) the heavy chain variable region according to the first aspect of the present invention, the heavy chain according to the second aspect of the present invention, the light chain variable region according to the third aspect of the present invention, the light chain according to the fourth aspect of the present invention, or the antibody according to the fifth aspect of the present invention; and
(ii) an optional tag sequence that assists expression and/or purification.

In another preferred embodiment, the tag sequence comprises a 6His tag.

In another preferred embodiment, the recombinant protein (or polypeptide) comprises a fusion protein.

In another preferred embodiment, the recombinant protein is a monomer, a dimer, or a multimer.

In another preferred embodiment, the recombinant protein comprises:
(i) an antibody selected from the group consisting of,

| Antibody number | Clone number | VH Sequence number | VL Sequence number |
|---|---|---|---|
| 1 | 4D10B11 | 1 | 6 |
| 2 | 29A03NA | 11 | 16 |
| 3 | 20H4G5 | 21 | 26 |
| 4 | 54G8G6 | 31 | 36 |
| 5 | 132D1E5 | 41 | 46 |
| 6 | 25E3B5 | 51 | 56 |
| 7 | 51E2F11 | 61 | 66 |
| 8 | 95E2D10 | 71 | 76 |

and
(ii) an optional tag sequence that assists expression and/or purification.

In the seventh aspect of the present invention, there is provided a polynucleotide encoding a polypeptide selected from the group consisting of:
(i) the heavy chain variable region according to the first aspect of the present invention, the heavy chain according to the second aspect of the present invention, the light chain variable region according to the third aspect of the present invention, the light chain according to the fourth aspect of the present invention, or the antibody according to the fifth aspect of the present invention; and
(2) the recombinant protein according to the sixth aspect of the present invention.

In another preferred embodiment, the polynucleotide encoding the heavy chain variable region is as shown in SEQ ID NO. 2, 12, 22, 32, 42, 52, 62, or 72; and/or, the polynucleotide encoding the light chain variable region is as shown in SEQ ID NO. 7, 17, 27, 37, 47, 57, 67, or 77.

In another preferred embodiment, the polynucleotide encoding the heavy chain variable region sequence and the polynucleotide encoding the light chain variable region sequence are selected from the group consisting of:

| Clone number | Sequence number of the polynucleotide encoding VH | Sequence number of the polynucleotide encoding VL |
|---|---|---|
| 4D10B11 | 2 | 7 |
| 29A03NA | 12 | 17 |
| 20H4G5 | 22 | 27 |
| 54G8G6 | 32 | 37 |
| 132D1E5 | 42 | 47 |
| 25E3B5 | 52 | 57 |
| 51E2F11 | 62 | 67 |
| 95E2D10 | 72 | 77. |

In the eighth aspect of the present invention, there is provided a vector comprising the polynucleotide according to any one of the seventh aspect of the present invention.

In another preferred embodiment, the vector comprises: a bacterial plasmid, a phage, a yeast plasmid, a plant cell virus, a mammalian cell virus such as an adenovirus, retrovirus, or other vectors.

In the ninth aspect of the present invention, there is provided a genetically engineered host cell, wherein the host cell contains the vector according to the eighth aspect of the present invention or the genome thereof is integrated with the polynucleotide according to the seventh aspect of the present invention..

In the tenth aspect of the present invention, there is provided an antibody conjugate comprising:
(i) an antibody moiety, which is selected from the group consisting of: the heavy chain variable region according to the first aspect of the present invention, the heavy chain according to the second aspect of the present invention, the light chain variable region according to the third aspect of the present invention, the light chain according to the fourth aspect of the present invention, the antibody according to the fifth aspect of the present invention, and a combination thereof; and
   (b) a coupling moiety coupled to the antibody moiety, and the coupling moiety is selected from the group consisting of a detectable label, a drug, a toxin, a cytokine, a radionuclide, an enzyme, and a combination thereof.

In another preferred embodiment, the antibody moiety is coupled to the coupling moiety via a chemical bond or linker.

In the eleventh aspect of the present invention, there is provided an immune cell, which expresses or is exposed outside the cell membrane with the antibody according to the fifth aspect of the present invention.

In another preferred embodiment, the immune cell comprises a NK cell, a T cell.

In another preferred embodiment, the immune cell is derived from human or non-human mammals (such as mice).

In the twelfth aspect of the present invention, there is provided a pharmaceutical composition comprising:
(i) an active ingredient, wherein the active ingredient is selected from the group consisting of: the heavy chain variable region according to the first aspect of the present invention, the heavy chain according to the second aspect of the present invention, the light chain variable region according to the third aspect of the present invention, the light chain according to the fourth aspect of the present invention, the antibody according to the fifth aspect of the present invention, the recombinant protein according to the sixth aspect of the present invention, the antibody conjugate according to the tenth aspect of the present invention, the immune cell according to the eleventh aspect of the present invention, and combinations thereof; and
(ii) a pharmaceutically acceptable carrier.

In another preferred embodiment, the pharmaceutical composition is a liquid formulation.

In another preferred embodiment, the pharmaceutical composition is an injection.

In another preferred embodiment, the pharmaceutical composition comprises 0.01-99.99% ofthe antibody according to the fifth aspect of the present invention, the recombinant protein according to the sixth aspect of the present invention, the antibody conjugate according to the tenth aspect of the present invention, the immune cell according to the eleventh aspect of the present invention, or a combination thereof, and 0.01-99.99% of the pharmaceutically acceptable carrier, wherein the percentage is the mass percentage of the pharmaceutical composition.

In the thirteenth aspect of the present invention, there is provided use of an active ingredient, wherein the active ingredient is selected from the group consisting of: the heavy chain variable region according to the first aspect of the present invention, the heavy chain according to the second aspect of the present invention, the light chain variable region according to the third aspect of the present invention, the light chain according to the fourth aspect of the present invention, the antibody according to the fifth aspect of the present invention, the recombinant protein according to the sixth aspect of the present invention, the antibody conjugate according to the tenth aspect of the present invention, the immune cell according to the eleventh aspect of the present invention, and combinations thereof, wherein the active ingredient is used for (a) preparation of a diagnostic reagent or kit; and/or (b) preparation of a medicine for preventing and/or treating diseases associated with abnormal CD47 expression or function.

In another preferred embodiment, the diagnostic reagent is a detection piece or a detection plate.

In another preferred embodiment, the disease associated with CD47 expression or dysfunction is a tumor.

In another preferred embodiment, the tumor is selected from the group consisting of: breast cancer, melanoma, lymphoma, head and neck cancer, colorectal cancer, soft tissue sarcoma, malignant hematoma, metastatic tumor, glioma, pancreatic cancer, gastric cancer, renal cancer, lung cancer, bladder cancer and esophageal cancer.

In another preferred embodiment, the diagnostic reagent or kit is used for:
(1) detecting CD47 protein in a sample; and/or
(2) detecting endogenous CD47 protein in tumor cells; and/or
(3) detecting tumor cells expressing CD47 protein;
while the medicine is used for preventing and/or treating diseases associated with abnormal CD47 expression or function, and the disease associated with abnormal CD47 expression or function is tumor.

In another preferred embodiment, the tumor is selected from the group consisting of breast cancer, melanoma, head and neck cancer, lymphoma, colorectal cancer, soft tissue sarcoma, malignant hematoma, metastatic tumor, glioma, pancreatic cancer, gastric cancer, renal cancer, lung cancer, bladder cancer, and esophageal cancer.

In another preferred embodiment, the antibody is in the form of a drug conjugate (ADC).

In another preferred embodiment, the diagnostic reagent or kit is used for diagnosis of CD47 related diseases.

In another preferred embodiment, the diagnostic reagent or kit is used for detection of CD47 protein in a sample.

In the fourteenth aspect of the present invention, there is provided a method for *in vitro* detection (including diagnostic or non-diagnostic) of CD47 protein in a sample, wherein the method comprises the steps:
(1)contacting the sample with the antibody according to the fifth aspect of the present invention *in vitro*;
(2) detecting whether an antigen-antibody complex is formed, wherein the formation of the complex indicates the presence of CD47 protein in the sample.

In the fifteenth aspect of the invention, there is provided a composition for detecting CD47 protein in a sample *in vitro,* which comprises the antibody according to the fifth aspect of the present invention, the recombinant protein according to the sixth aspect of the present invention, the antibody conjugate according to the tenth aspect of the present invention, the immune cell according to the eleventh aspect of the present invention, or a combination thereof, as an active ingredient.

In the sixteenth aspect of the invention, there is provided a detection plate comprising a substrate (support plate) and a detection strip containing the antibody according to the fifth aspect of the present invention, the recombinant protein according to the sixth aspect of the present invention, the antibody conjugate according to the tenth aspect of the present invention, the immune cell according to the eleventh aspect of the present invention, or a combination thereof.

In the seventeenth aspect of the present invention, there is provided a kit comprising:
(1) a first container, which contains the antibody of the present invention; and/or
(2) a second container, which contains a secondary antibody against the antibody of the present invention;
or,
the kit comprises the detection plate according to the sixteenth aspect of the present invention.

In the eighteenth aspect of the present invention, there is provided a method for preparing a recombinant polypeptide, comprising:
(a) culturing the host cell according to the ninth aspect of the present invention under conditions suitable for expression;
(b) isolating a recombinant polypeptide from the culture, wherein the recombinant polypeptide is the antibody according to the fifth aspect of the present invention or the recombinant protein according to the sixth aspect of the present invention.

In the nineteenth aspect of the present invention, there is provided a pharmaceutical combination comprising:
(i) a first active ingredient comprising the antibody 1 according to the fifth aspect of the present invention, or the recombinant protein according to the sixth aspect of the present invention, or the antibody conjugate according to the tenth aspect of the present invention, or the immune cell according to the eleventh aspect of the present invention, or the pharmaceutical composition according to the twelfth aspect of the present invention, or a combination thereof;
(ii) a second active ingredient comprising a second antibody, or a chemotherapeutic agent.

In another preferred embodiment, the second antibody is selected from the group consisting of a CTLA4 antibody, and a PD-1 antibody.

In another preferred embodiment, the second antibody is a PD-1 antibody.

In another preferred embodiment, the chemotherapeutic agent is selected from the group consisting of docetaxel, carboplatin, and a combination thereof.

In the twentieth aspect of the invention, there is provided use of a combination for preparation of a medicine for the treatment of diseases associated with abnormal CD47 expression or function, wherein the combination comprises the antibody according to the fifth aspect of the present invention, or the recombinant protein according to the sixth aspect of the present invention, or the antibody conjugate according to the tenth aspect of the present invention, or the immune cell according to the eleventh aspect of the present invention, and/or the pharmaceutical composition according to the twelfth aspect of the present invention as well as a second antibody or a chemotherapeutic agent.

In another preferred embodiment, the second antibody is selected from the group consisting of a CTLA4 antibody, and a PD-1 antibody.

In another preferred embodiment, the second antibody is a PD-1 antibody.

In a twenty-first aspect of the present invention, there is provided a method for the treatment of diseases associated with abnormal CD47 expression or function, which comprises administering an effective amount of the antibody according to the fifth aspect of the present invention, the recombinant protein according to the sixth aspect of the present invention, the antibody conjugate according to the tenth aspect of the present invention, the immune cell according to the eleventh aspect of the present invention, the pharmaceutical composition of the twelfth aspect of the present invention, or a combination thereof, to a subject in need.

In another preferred embodiment, the disease associated with abormal CD47 expression or dysfunction is a tumor.

In another preferred embodiment, the tumor is selected from the group consisting of breast cancer, melanoma, head and neck cancer, lymphoma, colorectal cancer, soft tissue sarcoma, malignant hematoma, metastatic tumor, glioma, pancreatic cancer, gastric cancer, renal cancer, lung cancer, bladder cancer, and esophageal cancer.

In another preferred embodiment, the method further comprises: administering a safe and effective amount of a second antibody to the subject before, during, and/or after administering the first active ingredient.

In another preferred embodiment, the second antibody is selected from the group consisting of a PD-1 antibody and a CTLA4 antibody.

In another preferred embodiment, the second antibody is a PD-1 antibody.

It should be understood that within the scope of the present invention, each technical features of the present invention described above and in the following (as examples) may be combined with each other to form a new or preferred technical solution, which needs not be described one by one, due to space limitations.

### Description of Drawings

Figure 1 shows the binding activity of SIRPα-hFc protein and biotin-labeled CD47-hFc.
Figure 2 shows detection of the binding of antibodies to human CD47 expressing cells by Flow cytometry (FACS).
Figure 3 shows detection of the binding of antibodies to cynomolgus CD47 expressing cells by Flow cytometry (FACS).
Figure 4 shows detection of the binding of antibodies to mouse CD47 expressing cells by Flow cytometry (FACS).
Figure 5 shows CD47 antibody blocks the binding reaction between CD47 protein and its receptor SIRPα.
Figure 6 shows CD47 antibody mediates the phagocytosis of human peripheral blood primary macrophages on Jurkat cells.
Figure 7 shows CD47 antibody mediates the phagocytosis of mouse bone marrow macrophages on CHOK1-mCD47.
Figure 8a and Figure 8b show the hemagglutination activity of CD47 antibody.
Figure 9 shows apoptosis of primary CD3+ T cells induced by CD47 antibody.
Figure 10 shows detection of the binding of murine antibodies to CD47 protein by enzyme-linked immunosorbent assay (ELISA).
Figure 11 shows detection of the binding of murine antibodies to human CD47 expressing cells by Flow cytometry (FACS).
Figure 12 shows detection of the binding of murine antibodies to cynomolgus CD47 expressing cells by Flow cytometry (FACS).
Figure 13 shows that CD47 murine antibody blocks the binding reaction between CD47 protein and its receptor SIRPα.
Figure 14 shows that CD47 murine antibody mediates phagocytosis of human peripheral blood primary macrophages on Jurkat cells.
Figure 15a, Figure 15b, Figure15c and Figure15d show the hemagglutination activity of CD47 murine antibody.
Figure 16 shows apoptosis of primary CD3+ T cells induced by CD47 murine antibody.
Figure 17 shows detection of the binding of chimeric antibodies to human CD47 expressing cells by Flow cytometry (FACS).
Figure 18 shows detection of the binding of chimeric antibodies to cynomolgus CD47 expressing cells by Flow cytometry (FACS).
Figure 19 shows that CD47 chimeric antibody blocks the binding reaction between CD47 protein and its receptor SIRPα.
Figure 20 shows that CD47 chimeric antibody mediates phagocytosis of human peripheral blood primary macrophages on Jurkat cells.
Figure 21a and Figure 21b show the hemagglutination activity of CD47 chimeric antibody.
Figure 22 shows apoptosis of primary CD3+ T cells induced by CD47 chimeric antibody.
Figure 23 shows effect of CD47 antibody on body weight of B-hSIRP/hCD47 humanized mice.
Figure 24 shows the changes of blood routine indexes on the first day after grouping.
Figure 25 shows the changes of blood routine indexes on the third day after grouping.
Figure 26 shows the changes of blood routine indexes on the sixth day after grouping.
Figure 27 shows the changes of blood routine indexes on the 13th day after grouping.
Figure 28 shows the changes of blood biochemical indexes on the 6th day after grouping.

### Detailed description

Through extensive and intensive research, using human CD47 protein, cell lines stably expressing CD47 protein, and plasmids containing cDNA encoding CD47 protein as immunogens, using hybridoma technology and phage technology, the present inventors accidentally obtained a group of mouse-derived or human-mouse chimeric CD47 antibodies with completely new amino acid sequences. The CD47 antibody of the present invention can effectively block the binding of CD47 protein and its receptor SIRPα (can inhibit the binding of CD47 and its receptor SIRP in a dose-dependent manner), promote tumor cells to be phagocytized by macrophages without causing significant hemagglutination reaction *in vitro* and obvious blood toxicity *in vivo,* it does not cause red blood cell failure, and will reduce potential safety hazards such as hemolysis in future animal models and clinical trials. Many reported CD47 antibodies will cause significant death of activated T cells, including a reference antibody Hu5F9-G4. However, the immune response generated by stress of activated T cells is also a main way to kill tumor cells. The CD47 antibody of the present invention has higher safety, does not cause hemagglutination of red blood cells, has no killing effect on activated normal T cells, and does not cause abnormal reduction of red blood cells or platelets in the toxicity detection *in vivo;* and has no obvious apoptosis-promoting effect on activated primary T cells. The antibody of the present invention can be applied to the preparation of drugs for treating tumors, autoimmune diseases and the like. The present invention has been completed on the basis of this.

### Terms

In the present invention, "VH-CDR1" and "CDR-H1" can be used interchangeably, and both refer to CDR1 of heavy chain variable region; "VH-CDR2" and "CDR-H2" can be used interchangeably and both refer to CDR2 of heavy chain variable region; "VH-CDR3" and "CDR-H3" can be used interchangeably and both refer to CDR3 of heavy chain variable region. "VL-CDR1" and "CDR-L1" can be used interchangeably, and both refer to CDR1 of light chain variable region; "VL-CDR2" and "CDR-L2" can be used interchangeably and both refer to CDR2 of light chain variable region; "VL-CDR3" and "CDR-L3" can be used interchangeably and both refer to CDR3 of light chain variable region.

### Antibody

As used herein, the term "antibody" or "immunoglobulin" is a heterotetrameric glycoprotein of about 150,000 Da having the same structural characteristics, which consists of two identical light chains (L) and two identical heavy chains (H). Each light chain is linked to a heavy chain via a covalent disulfide bond, and different immunoglobulin isotypes have different numbers of disulfide bonds between the heavy chains. There are also regularly spaced intrachain disulfide bonds in each heavy and each light chain. Each heavy chain has a variable region (VH) at one end, followed by a plurality of constant regions. Each light chain has a variable region (VL) at one end and a constant region at the other end; the constant region of a light chain pairs with the first constant region of a heavy chain, and the variable region of a light chain pairs with the variable region of a heavy chain. Special amino acid residues form an interface between the variable regions of a light chain and a heavy chain.

As used herein, the term "variable" means that antibodies are different from each other in terms of sequence in certain parts of variable regions, which is responsible for the binding and specificity of various specific antibodies to their specific antigens. However, the variability is not distributed evenly throughout the variable regions of an antibody. It is concentrated in three segments called complementarity determining regions (CDRs) or hypervariable regions in the light and heavy chain variable regions. The conserved parts of variable regions are called framework regions (FRs). The variable regions of the natural heavy and light chains each contain four FR regions, which are roughly in the β-folded configuration, connected by the three CDRs that form the connecting loop, and in some cases may form a partly β folded structure. The CDRs in each chain are closely linked together via the FR regions, and together with the CDRs of the other chain, form the antigen binding site of an antibody (see Kabat et al., NIH Publ. No. 91-3242, Vol. I, pp. 647-669 (1991)). The constant regions are not directly involved in the binding of an antibody to an antigen, however, they exhibit different effector functions, for example, involved in the antibody-dependent cytotoxicities of an antibody.

The light chains of vertebrate antibodies (immunoglobulins) can be classified into one of two distinct classes (referred to as κ and λ) based on the amino acid sequence of their constant regions. Immunoglobulins can be classified into different classes depending on the amino acid sequences of their heavy chain constant regions. There are mainly five classes of immunoglobulins: IgA, IgD, IgE, IgG, and IgM, some of which can be further classified into subclasses (isotypes), such as IgG1, IgG2, IgG3, IgG4, IgA, and IgA2. The heavy chain constant regions corresponding to different classes of immunoglobulins are called α, δ, ε, γ, and µ, respectively. The subunit structures and three-dimensional configurations of different classes of immunoglobulins are well known for those skilled in the art.

In general, the antigen binding characteristics of an antibody can be described by three specific regions located in the heavy and light chain variable regions, called complementarity determining regions (CDRs), which divide the variable region into four framework regions (FRs); the amino acid sequences of the four FRs are relatively conservative and are not directly involved in the binding reaction. These CDRs form a ring structure, and approach to each other in the steric structure by virtue of the β-sheets formed by the FRs between them, and the CDRs on the heavy chain and the CDRs on the corresponding light chain constitute the antigen-binding site of an antibody. By comparison of the amino acid sequences of antibodies of the same type, it can be determined which amino acids form FRs or CDRs.

The present invention includes not only an intact antibody, but also the fragments of the antibody having an immunological activity or a fusion protein formed by the antibody and another sequence. Therefore, the present invention also includes fragments, derivatives and analogs of the antibody.

In the present invention, antibodies include murine, chimeric, humanized or fully human antibodies as prepared by techniques well known to those skilled in the art. Recombinant antibodies, such as chimeric and humanized monoclonal antibodies, including human and non-human portions, can be obtained by standard DNA recombination techniques, all of which are useful antibodies. A chimeric antibody is a molecule in which different portions are derived from different animal species, for example, a chimeric antibody having a variable region from a monoclonal antibody from a mouse and a constant region from a human immunoglobulin (see, for example, U.S. Pat. Nos. 4,816,567 and 4,816,397, which are incorporated herein by reference in its entirety). A humanized antibody refers to an antibody molecule derived from a non-human species, which has one or more complementarity determining regions (CDRs) derived from a non-human species and framework regions derived from a human immunoglobulin molecule (see U.S. Pat. No. 5,585,089, which is incorporated herein by reference in its entirety). These chimeric and humanized monoclonal antibodies can be prepared by recombinant DNA techniques well known in the art.

In the present invention, an antibody may be monospecific, bispecific, trispecific, or multispecific.

In the present invention, the antibody of the present invention further includes a conservative variant thereof, which refers to a polypeptide formed by substitution of at most 10, preferably at most 8, more preferably at most 5, and most preferably at most 3 amino acids with amino acids having similar or analogous property, as compared to the amino acid sequence of the antibody of the present invention. These conservatively variant polypeptides are preferably produced by amino acid substitution according to Table 16.

**Table 16**

| Initial residue | Representative substitution | Preferred substitution |
|---|---|---|
| Ala (A) | Val; Leu; Ile | Val |
| Arg (R) | Lys; Gln; Asn | Lys |
| Asn (N) | Gln; His; Lys; Arg | Gln |
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gln; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

### Anti-CD47 antibody

In the present invention, the antibody is an anti-CD47 antibody. The present invention provides an antibody with high specificity and high affinity against CD47, which comprises a heavy chain and a light chain, wherein the heavy chain contains a heavy chain variable region (VH) amino acid sequence, and the light chain contains a light chain variable region (VL) amino acid sequence.

Preferably,
the heavy chain variable region (VH) has a complementarity determining region or CDR selected from the group consisting of:
   VH-CDR1 shown in SEQ ID NO.10n+3,
   VH-CDR2 shown in SEQ ID NO.10n+4, and
   VH-CDR3 shown in SEQ ID NO.10n+5;
   wherein, each n is independently 0, 1, 2, 3, 4, 5, 6, or 7;
the light chain variable region (VL) has a complementarity determining region CDR selected from the group consisting of:
   VL-CDR1 shown in SEQ ID NO. 10n+8,
   VL-CDR2 shown in SEQ ID NO. 10n+9, and
   VL-CDR3 shown in SEQ ID NO. 10n+10;
   wherein, each n is independently 0, 1, 2, 3, 4, 5, 6, or 7;
wherein any one of the above amino acid sequences further comprises a derivative sequence which is obtained through optional addition, deletion, modification and/or substitution of at least one amino acid and is capable of retaining CD47 binding affinity.

Preferably, the heavy chain variable region (VH) comprises the following three complementary determining regions or CDRs:
VH-CDR1 shown in SEQ ID NO.10n+3,
VH-CDR2 shown in SEQ ID NO.10n+4, and
VH-CDR3 shown in SEQ ID NO.10n+5;
the light chain variable region (VL) comprises the following three complementary determining regions or CDRs:
VL-CDR1 shown in SEQ ID NO. 10n +8,
VL-CDR2 shown in SEQ ID NO. 10n +9, and
VL-CDR3 shown in SEQ ID NO. 10n +10;
each n is independently 0, 1, 2 or 3; preferably n is 0 or 1;
wherein any one of the above amino acid sequences further comprises a derivative sequence which is obtained through optional addition, deletion, modification and/or substitution of at least one amino acid and is capable of retaining CD47 binding affinity.

In another preferred embodiment, the sequence with at least one amino acid added, deleted, modified and/or substituted in any of the above amino acid sequences is preferably an amino acid sequence having a homology or sequence identity of at least 80%, preferably at least 85%, more preferably at least 90%, most preferably at least 95% to the above amino acid sequence.

Method for determining sequence homology or identity that are well known to the ordinary skilled in the art includes, but are not limited to: Computer Molecular Biology, edited by Lesk, A.M., Oxford University Press, New York, 1988; Biocomputing; Biocomputing: Informatics and Genome Projects, edited by Smith, D.W., Academic Press, New York, 1993; Computer Analysis of Sequence Data, Part I, edited by Griffin, A.M. and Griffin, H.G., Humana Press, New Jersey, 1994; Sequence Analysis in Molecular Biology, von Heinje, G., Academic Press, 1987, and Sequence Analysis Primer, edited by Gribskov, M. and Devereux, J., M Stockton Press, New York, 1991 and Carillo, H. & Lipman, D., SIAM J. Applied Math ., 48:1073(1988). The preferred method for determining identity is to obtain the greatest match between the sequences tested. Methods for determining identity are compiled into publicly available computer programs. Preferred computer program method for determining identity between two sequences includes, but are not limited to, the GCG software package (Devereux, J. et al., 1984), BLASTP, BLASTN, and FASTA (Altschul, S, F. et al., 1990). The BLASTX program is available to the public from NCBI and other sources (BLAST Handbook, Altschul, S. et al., NCBI NLM NIH Bethesda, Md. 20894; Altschul, S. et al., 1990). The well-known Smith Waterman algorithm can also be used to determine identity.

Preferably, the antibody described herein is one or more of an antibody full-length protein, an antigen-antibody binding domain protein fragment, a bispecific antibody, a multispecific antibody, a single chain antibody fragment (scFv), a single domain antibody (sdAb), and a single-domain antibody, as well as a monoclonal antibody or a polyclonal antibody prepared from the above antibodies. The monoclonal antibody can be developed by a variety of approaches and technologies, including hybridoma technology, phage display technology, single lymphocyte gene cloning technology, etc. The mainstream is to prepare monoclonal antibodies from wild-type or transgenic mice through hybridoma technology.

The antibody full-length protein is a conventional antibody full-length protein in the art, which comprises a heavy chain variable region, a light chain variable region, a heavy chain constant region, and a light chain constant region. The heavy chain variable region and light chain variable region of the protein and human heavy chain constant region and human light chain constant region constitute a fully humanized antibody full-length protein. Preferably, the antibody full-length protein is IgG1, IgG2, IgG3 or IgG4.

The antibody of the present invention may be a double-chain or single-chain antibody, and may be selected from an animal-derived antibody, a chimeric antibody and a humanized antibody, more preferably a humanized antibody and a human-animal chimeric antibody, more preferably a fully humanized antibody.

The antibody derivatives of the present invention may be single chain antibodies, and/or antibody fragments, such as: Fab, Fab', (Fab')2 or other known antibody derivatives in the art, etc., as well as any one or several of IgA, IgD, IgE, IgG and IgM antibodies or other subtypes.

The single-chain antibody is a conventional single-chain antibody in the art, which comprises a heavy chain variable region, a light chain variable region and a short peptide of 15-20 amino acids.

In the present invention, the animal is preferably a mammal, such as a mouse.

The antibody of the present invention may be a chimeric antibody, a humanized antibody, a CDR grafted and/or modified antibody targeting CD47 (such as human CD47).

In above content of the present invention, the number of the added, deleted, modified and/or substituted amino acids, preferably does not exceed 40%, more preferably does not exceed 35%, more preferably is 1-33%, more preferably is 5-30%, more preferably is 10-25%, and more preferably is 15-20% of the total number of the amino acids of the initial amino acid sequence.

In the above content of the present invention, more preferably, the number of the added, deleted, modified and/or substituted amino acids, may be 1-7, more preferably 1-5, more preferably 1-3, and more preferably 1-2.

In another preferred embodiment, the heavy chain variable region of the antibody contains the amino acid sequence shown in SEQ ID NO. 1, 11, 21, 31, 41, 51, 61, or 71.

In another preferred embodiment, the light chain variable region of the antibody contains the amino acid sequence shown in SEQ ID NO. 6, 16, 26, 36, 46, 56, 66, or 76.

In another preferred embodiment, the amino acid sequences of the heavy chain variable region and/or the light chain variable region of the antibody targeting CD47 are shown in the following Table 17:

**Table 17**

| Antibody number | VH Sequence number | VL Sequence number |
|---|---|---|
| 1 | 1 | 6 |
| 2 | 11 | 16 |
| 3 | 21 | 26 |
| 4 | 31 | 36 |
| 5 | 41 | 46 |
| 6 | 51 | 56 |
| 7 | 61 | 66 |
| 8 | 71 | 76. |

In another preferred embodiment, the antibodies targeting CD47 are 4D10B11, 29A03NA, 20H4G5, 54G8G6, 132D1E5, 25E3B5, 51E2F11, 95E2D10, 158B3G6, 2G9C7, 92D9G2, 95B9E7, 89A4H1, 95F7E5, 126G2B1, 128D8D6, and 144B4E6. In another preferred embodiment, the antibody targeting CD47 is 4D10B11. In another preferred embodiment, the antibody targeting CD47 is 29A03NA.

### Recombinant protein

The present invention also provides a recombinant protein comprising one or more of heavy chain CDR1 (VH-CDR1), heavy chain CDR2 (VH-CDR2) and heavy chain CDR3 (VH-CDR3) of a CD47 antibody, and/or one or more of light chain CDR1 (VL-CDR1), light chain CDR2 (VL-CDR2) and light chain CDR3 (VL-CDR3) of a CD47 antibody,
the sequences of the heavy chain CDR1-3 are as follows:
   VH-CDR1 shown in SEQ ID NO: 10n+3,
   VH-CDR2 shown in SEQ ID NO. 10n+4,
   VH-CDR3 shown in SEQ ID NO: 10n+5;
the sequences of the light chain CDR1-3 are as follows:
   VL-CDR1 shown in SEQ ID NO: 10n+8,
   VL-CDR2 shown in SEQ ID NO: 10n+9, and
   VL-CDR3 shown in SEQ ID NO: 10n+10;
each n is independently 0, 1, 2, 3, 4, 5, 6, or 7; preferably n is 0 or 1;
wherein any one of the above amino acid sequences further comprises a derivative sequence which is obtained through optional addition, deletion, modification and/or substitution of at least one amino acid and is capable of retaining CD47 binding affinity.

In another preferred embodiment, the sequence with at least one amino acid added, deleted, modified and/or substituted in any of the above amino acid sequences is preferably an amino acid sequence having a homology or sequence identity of at least 80%, preferably at least 85%, more preferably at least 90%, most preferably at least 95% to the above amino acid sequence.

In another preferred embodiment, the recombinant protein of the present invention comprises a heavy chain variable region of a CD47 antibody and/or a light chain variable region of a CD47 antibody, the heavy chain variable region of the antibody comprising the amino acid sequence shown in SEQ ID NO. 1, 11, 21, 31, 41, 51, 61, or 71; the light chain variable region of the antibody comprising the amino acid sequence shown in SEQ ID NO. 6, 16, 26, 36, 46, 56, 66, or 76.

In another preferred embodiment, the recombinant protein of the present invention comprises a heavy chain variable region of a CD47 antibody and a light chain variable region of a CD47 antibody, the heavy chain variable region of a CD47 antibody comprising the amino acid sequence shown in SEQ ID NO. 1, 11, 21, 31, 41, 51, 61, or 71, and the light chain variable region of a CD47 antibody comprising the amino acid sequence shown in SEQ ID NO. 6, 16, 26, 36, 46, 56, 66, or 76.

In another preferred embodiment, the amino acid sequence numbers of the recombinant protein and the heavy chain CDR1-3 and light chain CDR1-3 comprised therein are as shown in Table 18:

**Table 18 Amino acid sequence numbers of heavy chain CDR1-3 and light chain CDR1-3**

| Recomb inant protein number | Heavy chain protein | | | | Light chain protein | | | |
|---|---|---|---|---|---|---|---|---|
| | Variable region | VH-CDR1 | VH-CDR2 | VH-CDR3 | Variable region | VL-CDR1 | VL-CDR2 | VL-CDR3 |
| 1 | 1 | 3 | 4 | 5 | 6 | 8 | 9 | 10 |
| 2 | 11 | 13 | 14 | 15 | 16 | 18 | 19 | 20 |
| 3 | 21 | 23 | 24 | 25 | 26 | 28 | 29 | 30 |
| 4 | 31 | 33 | 34 | 35 | 36 | 38 | 39 | 40 |
| 5 | 41 | 43 | 44 | 45 | 46 | 48 | 49 | 50 |
| 6 | 51 | 53 | 54 | 55 | 56 | 58 | 59 | 60 |
| 7 | 61 | 63 | 64 | 65 | 66 | 68 | 69 | 70 |
| 8 | 71 | 73 | 74 | 75 | 76 | 78 | 79 | 80 |

wherein any one of the above amino acid sequences further comprises a derivative sequence which is obtained through optional addition, deletion, modification and/or substitution of at least one amino acid and is capable of retaining CD47 binding affinity.

Preferably, the recombinant protein further comprises an antibody heavy chain constant region and/or an antibody light chain constant region, wherein the antibody heavy chain constant region is conventional in the art, preferably a rat antibody heavy chain constant region or a human antibody heavy chain constant region, more preferably a human antibody heavy chain constant region. The antibody light chain constant region is conventional in the art, preferably a rat antibody light chain constant region or a human antibody light chain constant region, more preferably a human antibody light chain constant region.

The recombinant protein is a conventional protein in the art. Preferably, it is one or more of an antibody full-length protein, an antigen-antibody binding domain protein fragment, a bispecific antibody, a multispecific antibody, a single chain antibody fragment (scFv), a single domain antibody (sdAb) and a single-domain antibody, as well as a monoclonal antibody or a polyclonal antibody made from the above antibodies. The monoclonal antibody can be developed by a variety of approaches and technologies, including hybridoma technology, phage display technology, single lymphocyte gene cloning technology, etc. The mainstream is to prepare monoclonal antibodies from wild-type or transgenic mice through hybridoma technology.

The antibody full-length protein is a conventional antibody full-length protein in the art, which comprises a heavy chain variable region, a light chain variable region, a heavy chain constant region, and a light chain constant region. The heavy chain variable region and light chain variable region of the protein and human heavy chain constant region and human light chain constant region constitute a fully human antibody full-length protein. Preferably, the antibody full-length protein is IgG1, IgG2, IgG3 or IgG4.

The single-chain antibody is a conventional single-chain antibody in the art, which comprises a heavy chain variable region, a light chain variable region and a short peptide of 15-20 amino acids.

The antigen-antibody binding domain protein fragments are conventional antigen-antibody binding domain protein fragments in the art, which comprise a light chain variable region, a light chain constant region, and an Fd segment of heavy chain constant region. Preferably, the antigen-antibody binding domain protein fragments are Fab and F (ab').

The single domain antibody is a conventional single domain antibody in the art, which comprises a heavy chain variable region and a heavy chain constant region.

The single-domain antibody is a conventional single-domain antibody in the art, which only comprises a heavy chain variable region.

Wherein, the preparation method of the recombinant protein is a conventional preparation method in the art. Preferably, the preparation method is: isolating and obtaining the protein from an expression transformant that recombinantly expresses the protein or obtaining the protein by artificially synthesizing a protein sequence. The method of isolating and obtaining the protein from an expression transformant that recombinantly expresses the protein is preferably as follows: cloning a nucleic acid molecule encoding the protein carrying a point mutation into a recombinant vector, and transforming the obtained recombinant vector into a transformant to obtain a recombinant expression transformant, and by culturing the obtained recombinant expression transformant, the recombinant protein can be obtained by separation and purification.

### Nucleic Acid

The present invention also provides a nucleic acid which encodes the heavy chain variable region or light chain variable region of the above-mentioned antibody (e.g., anti-CD47 antibody) or recombinant protein or anti-CD47 antibody.

Preferably, the nucleotide sequence of the nucleic acid encoding the heavy chain variable region is as shown in SEQ ID NO. 2, 12, 22, 32, 42, 52, 62, or 72 in the sequence listing; and/or, the nucleotide sequence of the nucleic acid encoding the light chain variable region is as shown in SEQ ID NO. 7, 17, 27, 37, 47, 57, 67, or 77 in the sequence listing.

More preferably, the nucleotide sequence of the nucleic acid encoding the heavy chain variable region is as shown in SEQ ID NO. 2, 12, 22, 32, 42, 52, 62, or 72 in the sequence listing; and the nucleotide sequence of the nucleic acid encoding the light chain variable region is as shown in SEQ ID NO. 7, 17, 27, 37, 47, 57, 67, or 77 in the sequence listing.

The preparation method of the nucleic acid is a conventional preparation method in the art. Preferably, it comprises the following steps: obtaining the nucleic acid molecule encoding the above-mentioned protein by gene cloning technology, or obtaining the nucleic acid molecule encoding the above-mentioned protein by the method of artificial full-length sequence synthesis.

Those skilled in the art know that the base sequence encoding the amino acid sequence of the protein can be replaced, deleted, changed, inserted or added appropriately to provide a polynucleotide homolog. The homolog of the polynucleotide of the present invention can be prepared by replacing, deleting or adding one or more bases of the gene encoding the protein sequence within the scope of maintaining the activity of the antibody.

### Vector

The present invention also provides a recombinant expression vector comprising the nucleic acid.

Wherein the recombinant expression vector can be obtained by conventional methods in the art, that is, by connecting the nucleic acid molecule of the present invention to various expression vectors, thus being constructed. The expression vector is one of a variety of conventional vectors in the art, as long as it can carry the above-mentioned nucleic acid molecule. The vector preferably includes: various plasmids, cosmids, phage or virus vectors and the like.

The present invention also provides a recombinant expression transformant comprising the above-mentioned recombinant expression vector.

Wherein, the preparation method of the recombinant expression transformant is a conventional preparation method in the art, preferably comprising: being obtained by transforming the recombinant expression vector into a host cell. The host cell is one of a variety of conventional host cells in the art, as long as the recombinant expression vector can replicate itself stably and the nucleic acid carried can be effectively expressed. Preferably, the host cell is *E.coli* TG1 or *E.coli* BL21 cell (for expressing single-chain antibodies or Fab antibodies), or HEK293 or CHO cell (for expressing full-length IgG antibodies). The above-mentioned recombinant expression plasmid is transformed into a host cell to obtain the preferred recombinant expression transformant of the present invention. Wherein the transformation method is a conventional transformation method in the art, preferably a chemical transformation method, a heat shock method or an electrotransformation method.

### Antibody preparation

The sequence of the DNA molecule for the antibody or a fragment thereof according to the present invention can be obtained by conventional techniques, for example, methods such as PCR amplification or genomic library screening. In addition, the sequences encoding light chain and heavy chain can be fused together, to form a single-chain antibody.

Once a relevant sequence is obtained, the relevant sequence can be obtained in bulk using a recombination method. This is usually carried out by cloning the sequence into a vector, transforming a cell with the vector, and then separating the relevant sequence from the proliferated host cell by conventional methods.

In addition, a relevant sequence can be synthesized artificially, especially when the fragment is short in length. Usually, several small fragments are synthesized first, and then are linked together to obtain a fragment with a long sequence.

At present, it is possible to obtain a DNA sequence encoding the antibody of the present invention (or fragments thereof, or derivatives thereof) completely by chemical synthesis. The DNA sequence can then be introduced into a variety of existing DNA molecules (or, for example, vectors) and cells known in the art. In addition, mutations can also be introduced into the protein sequences of the present invention by chemical synthesis.

The present invention further relates to a vector comprising said suitable DNA sequence and a suitable promoter or a control sequence. These vectors can be used to transform suitable host cells to enable them to express protein.

The host cell can be a prokaryotic cell, such as a bacterial cell; or a lower eukaryotic cell, such as a yeast cell; or a higher eukaryotic cell, such as a mammalian cell. Preferred animal cells include, but are not limited to, CHO-S, HEK-293 cells.

In general, under conditions suitable for expression of the antibody according to the present invention, the host cell obtained is cultured. Then, the antibody of the present invention is purified by using conventional immunoglobulin purification steps, for example, the conventional separation and purification means well known to those skilled in the art, such as protein A-Sepharose, hydroxyapatite chromatography, gel electrophoresis, dialysis, ion exchange chromatography, hydrophobic chromatography, molecular sieve chromatography or affinity chromatography.

The monoclonal antibody obtained can be identified by conventional means. For example, the binding specificity of a monoclonal antibody can be determined by immunoprecipitation or an *in vitro* binding assay (such as radioimmunoassay (RIA) or enzyme-linked immunosorbent assay (ELISA)). The binding affinity of a monoclonal antibody can be determined by, for example, the Scatchard analysis (Munson et al., Anal. Biochem., 107: 220 (1980)).

The antibody according to the present invention can be expressed in a cell or on the cell membrane, or is secreted extracellularly. If necessary, the recombinant protein can be separated and purified by various separation methods according to its physical, chemical, and other properties. These methods are well known to those skilled in the art. The examples of these methods comprise, but are not limited to, conventional renaturation treatment, treatment by protein precipitant (such as salt precipitation), centrifugation, cell lysis by osmosis, ultrasonic treatment, supercentrifugation, molecular sieve chromatography (gel chromatography), adsorption chromatography, ion exchange chromatography, high performance liquid chromatography (HPLC), and any other liquid chromatography, and the combination thereof.

### Antibody-drug conjugate (ADC)

The present invention also provides an antibody-drug conjugate (ADC) based on the antibody according to the present invention.

Typically, the antibody-drug conjugate comprises the antibody and an effector molecule, wherein the antibody is conjugated to the effector molecule, and chemical conjugation is preferred. Preferably, the effector molecule is a therapeutically active drug. In addition, the effector molecule may be one or more of a toxic protein, a chemotherapeutic drug, a small-molecule drug or a radionuclide.

The antibody according to present invention and the effector molecule may be coupled by a coupling agent. Examples of the coupling agent may be any one or more of a non-selective coupling agent, a coupling agent utilizing a carboxyl group, a peptide chain, and a coupling agent utilizing a disulfide bond. The non-selective coupling agent refers to a compound that results in a linkage between an effector molecule and an antibody via a covalent bond, such as glutaraldehyde, etc. The coupling agent utilizing a carboxyl group may be any one or more of cis-aconitic anhydride coupling agents (such as cis-aconitic anhydride) and acyl hydrazone coupling agents (the coupling site is acyl hydrazone).

Certain residues on an antibody (such as Cys or Lys, etc.) are used to link a variety of functional groups, including imaging agents (such as chromophores and fluorophores), diagnostic agents (such as MRI contrast agents and radioisotopes), stabilizers (such as poly(ethylene glycol)) and therapeutic agents. An antibody can be conjugated to a functional agent to form a conjugate of the antibody-functional agent. A functional agent (e.g. a drug, a detection reagent, a stabilizer) is conjugated (covalently linked) to an antibody. A functional agent can be linked to an antibody either directly or indirectly via a linker.

Antibodies can be conjugated to drugs to form antibody-drug conjugates (ADCs). Typically, an ADC comprises a linker between a drug and an antibody. The linker can be a degradable or non-degradable linker. Typically, degradable linkers are easily degraded in an intracellular environment, for example, the linker is degraded at the target site, thereby releasing the drug from the antibody. Suitable degradable linkers include, for example, enzyme-degradable linkers, including peptidyl-containing linkers that can be degraded by protease (e.g. lysosomal protease or endosomal protease) in a cell, or sugar linkers, for example, glucuronide-containing linkers that can be degraded by glucuronidase. Peptidyl linkers may include, for example, dipeptides, such as valine-citrulline, phenylalanine-lysine or valine-alanine. Other suitable degradable linkers include, for example, pH sensitive linkers (e.g. linkers that are hydrolyzed at a pH of below 5.5, such as hydrazone linkers) and linkers that are degraded under reducing conditions (e.g. disulfide-bond linkers). A non-degradable linker typically releases a drug under conditions that the antibody is hydrolyzed by protease.

Prior to linkage to an antibody, a linker has a reactive group capable of reacting with certain amino acid residues, and the linkage is achieved by the reactive group. A thiol-specific reactive group is preferred, and includes, for example, a maleimide compound, a halogenated (e.g. iodo-, bromo- or chloro-substituted) amide; a halogenated (e.g. iodo-, bromo- or chloro-substituted) ester; a halogenated (e.g. iodo-, bromo- or chloro-substituted) methyl ketone, a benzyl halide (e.g. iodide, bromide or chloride); vinyl sulfone, pyridyl disulfide; a mercury derivative such as 3,6-di-(mercurymethyl)dioxane, wherein the counter ion is CH₃COO⁻, Cl⁻ or NO₃⁻; and polymethylene dimethyl sulfide thiosulfonate. The linker may include, for example, a maleimide linked to an antibody via thiosuccimide.

A drug may be any cytotoxic drug which inhibits cell growth or immunosuppression. In an embodiment, an antibody is linked to a drug via a linker, and the drug has a functional group that can form a bond with the linker. For example, a drug may have an amino group, a carboxyl group, a thiol group, a hydroxyl group, or a ketone group that can form a bond with a linker. When a drug is directly linked to a linker, the drug has a reactive group before being linked to an antibody.

Useful drugs include, for example, anti-tubulin drugs, DNA minor groove binding agents, DNA replication inhibitors, alkylating agents, antibiotics, folic acid antagonists, antimetabolites, chemotherapy sensitizers, topoisomerase inhibitors, vinca alkaloids, etc. Examples of particularly useful cytotoxic drugs include, for example, DNA minor groove binding agents, DNA alkylating agents, and tubulin inhibitors; typical cytotoxic drugs include, for example, auristatins, camptothecins, docamycinDokamycin/duocarmycins, etoposides, maytansines and maytansinoids (e.g. DM1 and DM4), taxanes, benzodiazepines or benzodiazepine containing drugs (e.g. pyrrolo[1,4]benzodiazepines (PBDs), indolinobenzodiazepines and oxazolidinobenzodiazepines), and vinca alkaloids.

In the present invention, a drug-linker can be used to form an ADC in a simple step. In other embodiments, a bifunctional linker compound can be used to form an ADC in a two-step or multi-step process. For example, a cysteine residue is reacted with the reactive moiety of a linker in a first step, and then the functional group on the linker is reacted with a drug in the subsequent step, so as to form an ADC.

In general, the functional group on a linker is selected so that it can specifically react with the suitable reactive group on a drug moiety. As a non-limiting example, an azide-based moiety can be used to specifically react with the reactive alkynyl group on a drug moiety. The drug is covalently bound to the linker by 1,3-dipolar cycloaddition between the azide and alkynyl group. Other useful functional groups include, for example, ketones and aldehydes (suitable for reacting with hydrazides and alkoxyamines), phosphines (suitable for reacting with azides); isocyanates and isothiocyanates (suitable for reacting with amines and alcohols); and activated esters, for example, N-hydroxysuccinimide esters (suitable for reacting with amines and alcohols). These and other linkage strategies, for example, those described in "Bioconjugation Technology" (2nd Edition (Elsevier)), are well known to those skilled in the art. Those skilled in the art could understand that when a complementary pair of reactive functional groups are selected for a selective reaction between a drug moiety and a linker, each member of the complementary pair can be used for the linker, and can also be used for the drug.

The present invention further provides a method for preparing an ADC, which may further comprise: under conditions sufficient to form an antibody-drug conjugate (ADC), binding an antibody to a drug-linker compound.

In certain embodiments, the method according to the present invention comprises: under conditions sufficient to form an antibody-linker conjugate, binding an antibody to a bifunctional linker compound. In these embodiments, the method according to the present invention further comprises: under conditions sufficient to covalently link the drug moiety to the antibody via a linker, binding the antibody-linker conjugate to the drug moiety.

In some embodiments, an antibody-drug conjugate (ADC) has a formula as follows: 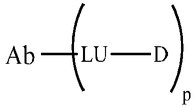 wherein:
Ab is an antibody,
LU is a linker;
D is a drug;
And the subscript p is a value selected from 1 to 8.

### Application

The present invention also provides use of the antibody, the antibody conjugate ADC, the recombinant protein, and/or immune cell of the present invention, for example for the preparation of diagnostic preparations or the preparation of drugs.

Preferably, the drug is used for prevention and/or treatment of diseases associated with abnormal CD47 expression or function.

In the present invention, the diseases associated with abnormal CD47 expression or function are conventional diseases associated with abnormal CD47 expression or function in the art. Preferably, the disease associated with abnormal CD47 expression or function is a tumor/cancer.

In the present invention, the cancer is a conventional cancer in the art, preferably breast cancer, melanoma, head and neck cancer, lymphoma, colorectal cancer, soft tissue sarcoma, malignant hematoma, metastatic tumor, glioma, pancreatic cancer, gastric cancer, renal cancer, lung cancer, bladder cancer, and esophageal cancer.

Use of the antibody, the ADC, the recombinant protein, and/or the immune cell of the present invention includes (but is not limited to):
(i) for diagnosis, prevention and/or treatment of tumorigenesis, tumor growth and/or metastasis, especially a tumor with high expression of CD47. The tumor includes, but is not limited to, breast cancer, melanoma, head and neck cancer, lymphoma, colorectal cancer, soft tissue sarcoma, malignant hematoma, metastatic tumor, glioma, pancreatic cancer, gastric cancer, renal cancer, lung cancer, bladder cancer, and esophageal cancer.

### Use for detection and kit

The antibody or ADC thereof of the present invention can be used for detection, for example, for detecting samples, thereby providing diagnostic information.

In the present invention, the samples (specimens) used include cells, tissue samples and biopsy specimens. The term "biopsy" used in the present invention shall include all kinds of biopsy known to those skilled in the art. Therefore, the biopsy used in the present invention may include, for example, excision samples of tumors, tissue samples prepared by endoscopic methods or organ puncture or needle biopsy.

The samples used in the present invention include fixed or preserved cell or tissue samples.

The present invention also provides a kit comprising the antibody (or fragment thereof) of the present invention. In a preferred embodiment of the present invention, the kit further includes a container, an instruction for use, buffer, and the like. In a preferred embodiment, the antibody of the present invention can be immobilized on a detection plate.

### Pharmaceutical composition

The invention further provides a composition. In the preferred examples, the composition is a pharmaceutical composition comprising the antibody, or an active fragment, a fusion protein or an ADC thereof, or a corresponding immune cell, and a pharmaceutically acceptable carrier. In general, these substances may be formulated in a non-toxic, inert and pharmaceutically acceptable aqueous carrier medium, wherein the pH is generally about 5-8, preferably, pH is about 6-8, though the pH value may be varied depending on the nature of the substances to be formulated and the condition to be treated.

The formulated pharmaceutical composition may be administered by conventional routes, including (but not limited to): intratumoral, intraperitoneal, intravenous, or topical administration. Typically, the administration route of the pharmaceutical composition of the present invention is preferably injection or oral administration. The injection administration preferably includes intravenous injection, intramuscular injection, intraperitoneal injection, intradermal injection, or subcutaneous injection. The pharmaceutical composition is in one of a variety of conventional dosage forms in the art, preferably in solid, semi-solid or liquid form, and can be an aqueous solution, a non-aqueous solution or a suspension, and more preferably tablets, capsules, granules, injection or infusion, etc.

The antibody of the present invention can also be used for cell therapy by expressing the nucleotide sequence in a cell, for example, the antibody is used for chimeric antigen receptor T cell immunotherapy (CAR-T) and the like.

The pharmaceutical composition of the present invention is a pharmaceutical composition for prevention and/or treatment of diseases associated with abnormal CD47 expression or function.

The pharmaceutical composition of the present invention can be directly used for binding to a CD47 protein molecule, and thus can be used for preventing and treating diseases such as tumors.

The pharmaceutical composition according to the present invention comprises a safe and effective amount (e.g. 0.001-99 wt %, preferably 0.01-90 wt %, preferably 0.1-80 wt %) of the monoclonal antibody according to the present invention (or a conjugate thereof) and a pharmaceutically acceptable carrier or excipient. Such carriers include, but are not limited to, saline, buffer solution, glucose, water, glycerin, ethanol or the combination thereof. The pharmaceutical preparation should be matched to the method of administration. The pharmaceutical composition of the present invention can be prepared in the form of injection, for example, prepared by a conventional method using physiological saline or an aqueous solution containing glucose and other adjuvants. Pharmaceutical compositions such as injections and solutions are preferably prepared under sterile conditions. The dosage of active ingredient is therapeutically effective amount, for example from about 1 microgram per kilogram body weight to about 5 milligrams per kilogram body weight per day. Further, the polypeptide of the present invention can also be used in combination with the other therapeutic agents.

In the present invention, preferably, the pharmaceutical composition of the present invention further comprises one or more pharmaceutical carriers. The pharmaceutical carrier is a conventional pharmaceutical carrier in the art, and the pharmaceutical carrier can be any suitable physiologically or pharmaceutically acceptable pharmaceutical excipient. The pharmaceutical excipient is a conventional pharmaceutical excipient in the art, and preferably includes pharmaceutically acceptable excipients, fillers or diluents. More preferably, the pharmaceutical composition comprises 0.01-99.99% of the above-mentioned protein and 0.01-99.99% of the pharmaceutically acceptable carrier, wherein the percentage is the mass percentage of the pharmaceutical composition.

In the present invention, preferably, the administration amount of the pharmaceutical composition is an effective amount, and the effective amount is an amount that can alleviate or delay the progression of the disease, and the degenerative or traumatic condition. The effective amount can be determined on an individual basis and will be partly based on consideration of the symptoms to be treated and the results sought. Those skilled in the art can determine the effective amount by using the above-mentioned factors such as individual basis and using no more than conventional experiments.

When a pharmaceutical composition is used, a safe and effective amount of the immunoconjugate is administered to a mammal, wherein the safe and effective amount is usually at least about 10 micrograms per kilogram of body weight, and in most cases does not exceed about 50 mg/kg body weight, preferably the dose is about 10 micrograms/kg body weight to about 20 mg/kg body weight. Of course, the particular dose should also depend on various factors, such as the route of administration, patient healthy status, which are well within the skills of an experienced physician.

The present invention provides use of the above-mentioned pharmaceutical composition in the preparation of a medicine for preventing and/or treating diseases associated with abnormal CD47 expression or function. Preferably, the disease associated with abnormal CD47 expression or function is a tumor/cancer.

### Method and composition for detecting CD47 protein in a sample

The present invention also provides a method for detecting CD47 protein in a sample (for example, detecting cells over-expressing CD47), which comprises the following steps: contacting the above-mentioned antibody with a sample to be tested *in vitro,* and detecting whether the above-mentioned antibody binds to the sample to be tested, to form an antigen-antibody complex.

The meaning of overexpression is conventional in the art, which refers to the overexpression of RNA or protein of CD47 protein in the sample to be tested (due to increased transcription, post-transcriptional processing, translation, post-translational processing and protein degradation changes), and local overexpression and increased functional activity (such as in the case of increased enzymatic hydrolysis of the substrate) due to changes in protein transport mode (increased nuclear localization).

In the present invention, the detection method for detecting whether an antigen-antibody complex is formed is a conventional detection method in the art, preferably a flow cytometry (FACS) detection.

The present invention provides a composition for detecting CD47 protein in a sample, which comprises the above-mentioned antibody, recombinant protein, antibody conjugate, immune cell, or a combination thereof as an active ingredient. Preferably, it also comprises a compound composed of the functional fragments of the above-mentioned antibody as an active ingredient.

On the basis of conforming to common knowledge in the art, the above-mentioned preferred conditions can be combined arbitrarily to obtain preferred embodiments of the present invention.

### The main advantages of the invention are:

(1) The CD47 antibody of the present invention is mouse-derived or human-mouse chimeric, which has high affinity, has high affinity with human CD47 protein, and has similar binding ability with cynomolgus monkey-derived CD47 protein.
(2) The CD47 antibody of the present invention can inhibit the binding of CD47 to its receptor SIRPα; it can inhibit the binding of CD47 to its receptor SIRPα in a dose-dependent manner.
(3) The CD47 antibody of the present invention can promote tumor cells to be phagocytized by macrophages.
(4) Some antibodies of the present invention do not cause significant red blood cell hemagglutination reaction, and some antibodies have no killing effect on activated primary T cells.
(5) The antibody of the present invention has good tolerance in the in vivo safety evaluation and does not cause significant red blood cell failure.
(6) The CD47 antibody has important value in the application of preparing tumor prevention and treatment drugs, including leukemia, lymphoma, breast cancer, melanoma, glioma, pancreatic cancer, cervical cancer, intestinal cancer, gastric cancer, renal cancer, lung cancer, bladder cancer and, or esophageal cancer.

The invention is further illustrated by the following specific examples. It is to be understood that these examples are for illustrative purposes only and are not intended to limit the scope of the invention. The experimental methods without detailed conditions in the following examples are generally in accordance with the conditions described in the conventional conditions such as Sambrook. J et al. "Guide to Molecular Cloning Laboratory" (translated by Huang Peitang et al., Beijing: Science Press, 2002), or in accordance with the conditions recommended by the manufacturer (for example, product manuals). Percentages and parts are by weight unless otherwise stated. The experimental materials and reagents used in the following examples are commercially available unless otherwise specified.

The room temperature described in the examples is a conventional room temperature in the art, and is generally 10-30°C.

Unless otherwise specified, the PBS described in the examples is PBS phosphate buffer, pH 7.2.

### EXAMPLE 1 Preparation of CD47 antibody

### (1) Preparation of Immunogen A

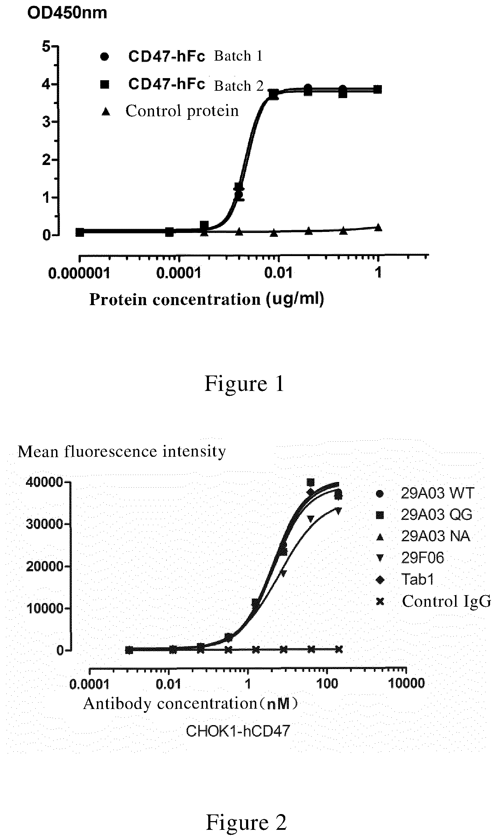

The sequence encoding amino acid sequence 19-141 (Gln19-Glu141) of the extracellular domain of the human CD47 protein (wherein the protein sequence number of the human CD47 protein in the UniProtKB database is Q08722) was cloned into the pCPC vector (purchased from Invitrogen, V044-50) to prepare recombinant plasmid in accordance with the established standard molecular biology methods (Sambrook, J., Fritsch, E. F., and Maniatis, T. (1989). Molecular Cloning: A Laboratory Manual, Second Edition (Plainview, New York: Cold Spring Harbor Laboratory Press)). The prepared recombinant plasmid was transiently transfected into HEK293 cells (purchased from Invitrogen) [polyetherimide (PEI) purchased from Polysciences] and expanded. After 4 days, the cell culture medium was collected, and the cell components were removed by centrifugation to obtain a culture supernatant containing CD47 protein. After imidazole with a final concentration of 10mM was added to the culture supernatant, the sample was filtered with a 0.22 µm filter membrane and loaded into a nickel column. At the same time, the ultraviolet absorption value (A280nm) was monitored by an ultraviolet (UV) detector. After loading, Buffer A (20mM imidazole was adde to 1×PBS), Buffer B [0.1% (v/v) triton × 100 and 0.1% (v/v) triton × 114 were added to Buffer A] were added to balance, and then eluted with Buffer C [250mM imidazole was added to 1×PBS]. The collected samples were further purified with molecular sieve superdex 200 (purchased from GE Healthcare) to obtain high purity human CD47 protein (hCD47) extracellular domain protein, which was dialyzed with PBS at 4 °C overnight. The dialyzed protein was aseptically filtered at 0.22 µm, then packed and stored at -80 °C, which is called immunogen A. Immunogen A protein needed a series of quality control tests before use, such as tests of protein concentration, purity, molecular weight, biological activity, etc. The results are shown in Figure 1 and Table 1.

**Table 1 Binding activity of SIRPα-hFc protein to biotin-labeled CD47-hFc**

| OD₄₅₀ₙₘ | SIRPα-hFc (1µg/mL) | | | | | | | |
|---|---|---|---|---|---|---|---|---|
| Protein concentration (µg/ml) | 1 | 0.2 | 0.04 | 0.008 | 0.0016 | 0.0003 | 0.0001 | 0.00001 |
| Biotin-labeled CD47 batch 1 | 3.84 | 3.85 | 3.88 | 3.71 | 1.06 | 0.19 | 0.09 | 0.07 |
| Biotin-labeled CD47 batch 2 | 3.84 | 3.71 | 3.78 | 3.70 | 1.27 | 0.25 | 0.10 | 0.07 |
| Control protein (non-CD47 fusion protein) | 0.19 | 0.11 | 0.10 | 0.06 | 0.08 | 0.07 | 0.07 | 0.06 |

Table 1 shows that the binding of CD47 to SIRPα at the protein level varies with the concentration of CD47, wherein the control protein is a non-CD47 fusion protein, and the data in the table is OD₄₅₀ₙₘ value.

Wherein, the biological activity of immunogen A was detected by ELISA, specifically:
The SIRPα protein with hFc tag (SIRPα-hFc) was diluted to 1µg/ml with PBS, added to ELISA microplate at 100µL/well, and incubated at 4 °C overnight. The preparation method of the SIRPα-hFc was the same as the preparation method of the immunogen A, wherein the amino acid sequence information of the SIRPα extracellular domain protein (Glu31-Arg370) was referred to the Uniprot database, numbered P78324. After blocking with ELISA blocking solution (PBS phosphate buffer containing 1% BSA, pH7.4, and the percentage is mass percentage) at 37 °C for two hours, added gradient diluted biotin-labeled CD47-hFc (i.e. immunogen A, the preparation method of biotin-labeled CD47-hFc was as follows: reacting CD47-hFc with biotinylation reagent. The biotinylation reagent was purchased from Sigma, trade number B3295. The operation steps of the reaction with the biotinylation reagent can refer to the instructions of the biotinylation reagent), incubated at 37 °C for 1 hour. Streptavidin-labeled horseradish peroxidase (purchased from Sigma trade number S2438) was added and incubated for 30 minutes at room temperature; 100µl/well of TMB substrate was added and incubated at room temperature for 15 minutes. After that, 50 µL of IN hydrochloric acid was added to terminate the developing reaction, and the OD₄₅₀ₙₘ was read with an ELISA plate reader.

### (2) Preparation of Immunogen B

The nucleotide sequence encoding the full-length amino acid sequence of human CD47 was cloned into pIRES vector (purchased from Clontech) and the plasmid was prepared. After the HEK293 cell line and the NIH3T3 cell line (both purchased from Invitrogen) were transfected with plasmid (PEI, purchased from Polysciences), cells were selectively cultured in DMEM medium containing 10% (w/w) fetal bovine serum of 0.5 µg/ml for 2 weeks. Subcloning was conducted in a 96-well culture plate by limited dilution method, and the plate was placed at 37 °C, 5% (v/v) CO₂. After about 2 weeks, some monoclonal wells were selected and amplified into 6-well plates. The amplified clones were screened by flow cytometry using known CD47 antibodies (Hu5F9-G4). The culture expanding of the monoclonal cell line with better growth, higher fluorescence intensity was continued and the cell was frozen in liquid nitrogen, thus obtaing immunogen B. The specific selection results are shown in Table 2. In Table 2, positive cells (%) refer to the percentage of number of positive cells in the total number of cells.

**Table 2 FACS screening detection results of HEK293 cells transfected with CD47 protein**

| Number | Transfected cell clone number | CD47 antibody | | IgG subtype control | |
|---|---|---|---|---|---|
| | | Positive cells (%) | Mean fluorescence intensity | Positive cells (%) | Mean fluorescence intensity |
| 1 | 293F-hCD47 1D10 | 78.51 | 416.86 | 2.38 | 3.19 |
| 2 | 293F-hCD47 1B4 | 94.21 | 508.96 | 1.60 | 2.56 |
| 3 | 293F-hCD47 2E2 | 95.31 | 535.32 | 2.42 | 2.79 |
| 4 | 293F-hCD47 1B8 | 92.78 | 479.45 | 3.08 | 3.11 |
| 5 | 293F-hCD47 1C8 | 97.34 | 705.06 | 0.81 | 2.15 |
| 6 | 293F-hCD47 1A8 | 96.39 | 904.86 | 2.19 | 3.20 |
| 7 | 293F-hCD47 1E8 | 95.14 | 784.57 | 1.29 | 2.79 |
| 8 | 293F-hCD47 1C4 | 94.14 | 804.23 | 2.55 | 3.84 |
| 9 | 293F-hCD47 1B5 | 97.01 | 796.36 | 0.85 | 2.72 |
| 10 | 293F-hCD47 1F6 | 86.98 | 492.52 | 2.35 | 2.94 |
| 11 | 293F-hCD47 1F10 | 87.35 | 622.66 | 3.85 | 3.64 |
| 12 | 293F-hCD47 1F11 | 85.94 | 476.33 | 3.78 | 3.73 |
| 13 | 293F-hCD47 1B11 | 96.27 | 743.47 | 4.70 | 2.87 |
| 14 | 293F-hCD47 1B6 | 93.69 | 953.12 | 5.70 | 3.22 |
| 15 | 293F-hCD47 1F8 | 93.33 | 949.77 | 3.13 | 2.66 |
| 16 | 293F-hCD47 1C2 | 97.05 | 469.90 | 1.85 | 2.47 |

Table 2 indicates that a series of HEK293 cell lines with CD47 positive expression have been prepared.

### (3) Preparation of Immunogen C

The cDNA of CD47 full-length amino acid sequence was cloned into pCDNA3.1 vector (purchased from Invitrogen), and coated on 1.0 um gold colloidal bullet. Immunization was conducted with Helios Gene Gun System (Bio-rad, Cat. No. 165-2431). Among them, the methods of coating 1.0 µm gold colloid bullets and immunization refer to Helios gene gun instructions. Immunogen C was obtained after immunization.

### (4) Preparation of hybridoma cells and antibody screening

(1) Immunogen A Balb/c and SJL mice (purchased from Shanghai Slack) aged 6-8 weeks were used to immunize, and the mice were fed under SPF conditions after receiving. Immunogen A was emulsified with Freund's complete adjuvant and injected intraperitoneally with 0.25 mL and 50 µg protein per mouse for primary immunization. Immunogen A was emulsified with Freund's incomplete adjuvant and injected intraperitoneally with 0.25 mL and 50 µg immunogen A per mouse for booster immunization. The interval between the primary immunization and the first booster immunization was 2 weeks, and the interval between each booster immunization after that was 3 weeks. Blood was collected 7 days after each booster immunization, and antibody titer and specificity in serum were detected by ELISA (Table 3). The blank control in Table 3 is 1% (w/w) BSA, where the batch refers to the mouse serum on the seventh day after the second booster immunization, and the data in the table is the value of OD₄₅₀ₙₘ. After two booster immunizations, the ELISA titer was usually 1: 10000.

**Table 3 Detection of serum antibody titer in a SJL mice immunized with CD47 protein by ELISA**

| OD₄₅₀ₙₘ | Serum dilution | | | | | | |
|---|---|---|---|---|---|---|---|
| Batch | 1:100 | 1:10³ | 1:10⁴ | 1:10⁵ | 1:10⁶ | 1:10⁷ | Blank control |
| 916 (TB2) | 3.47 | 3.68 | 3.53 | 2.61 | 0.78 | 0.28 | 0.21 |
| 917 (TB2) | 3.47 | 3.48 | 3.55 | 2.39 | 0.63 | 0.25 | 0.22 |
| 918 (TB2) | 3.49 | 3.48 | 3.55 | 2.43 | 0.70 | 0.28 | 0.23 |
| 919 (TB2) | 3.56 | 3.66 | 3.50 | 2.24 | 0.60 | 0.28 | 0.25 |
| 920 (TB2) | 3.32 | 3.66 | 3.56 | 2.60 | 0.72 | 0.30 | 0.22 |
| 921 (TB2) | 3.41 | 3.67 | 3.56 | 2.55 | 0.68 | 0.27 | 0.21 |
| 922 (TB2) | 3.32 | 3.58 | 3.36 | 2.19 | 0.58 | 0.26 | 0.25 |
| 923 (TB2) | 3.33 | 3.58 | 3.51 | 2.60 | 0.71 | 0.29 | 0.25 |
| 924 (TB2) | 3.24 | 3.57 | 3.35 | 1.74 | 0.49 | 0.25 | 0.24 |
| 925 (TB2) | 3.43 | 3.47 | 3.34 | 1.79 | 0.48 | 0.28 | 0.32 |

(2) Immunogen B Balb/c and SJL mice (purchased from Shanghai Slack) aged 6-8 weeks were used to immunize, and the mice were fed under SPF conditions after receiving. HEK293 cell line was transfected with a pIRES plasmid containing the nucleotide sequence encoding the full-length amino acid sequence of human CD47 (see step (2) of example 1) to obtain HEK293 and NIH3T3 stable cell lines containing human CD47 (transfected using X-treme GENE HP DNA Transfusion Reagent, purchased from Roche Company, catalog number Cat # 06 366 236 001, and operated according to the instructions). In a T-75 cell culture flask, the cells were expanded to 90% confluence, the medium was sucked out, the cells were washed twice with DMEM basic medium (purchased from Invitrogen), and then treated with enzyme-free cell dissociation solution (purchased from Invitrogen) at 37°C until the cells could fall off from the dish wall, and the cells were collected. Cells were washed twice with DMEM basal medium and counted, and then diluted with phosphate buffer (pH 7.2) to 2×10⁷ cells per ml. Each mouse was intraperitoneally injected with 0.5 ml of cell suspension during each immunization. The interval between the first and the second immunization was 2 weeks. After that, the intervals between each subsequent immunization were 3 weeks. Except for the first immunization, blood was collected one week after each immunization, and the antibody titer and specificity in the serum were detected by FACS. After the second booster immunization, the serum antibody titer detected by FACS reached more than 1: 1000.
(3) Immunogen C Balb/c and SJL mice (purchased from Shanghai Slack) aged 6-8 weeks were used to immunize, and the mice were fed under SPF conditions after receiving. All mice were immunized with the Helios gene gun through the abdomen for 4 times, 4 shots each time, 1.0 µg cDNA amount per shot. The interval between the primary immunization and the first booster immunization was 2 weeks, and the interval between each booster immunization after that was 3 weeks. Blood was collected one week after each booster immunization, and the antibody titer in serum was detected by ELISA or FACS. After the second booster immunization, the titer of serum antibody detected by FACS reached more than 1: 1000, and the titer of ELISA was over 1: 10000.

Before the completion of steps (1) to (3), each selected mouse was intraperitoneally injected with 100 micrograms of purified CD47-hFc (for mice immunized with immunogen A and immunogen C) or HEK293 or NIH3T3 stable cell lines containing human CD47 (for mice immunized with immunogen B) for the last immunization. After 5 days, the mice were sacrificed and spleen cells were collected. NH₄OH was added to a final concentration of 1% (w/w) to lyse the mixed red blood cells in the spleen cells to obtain a spleen cell suspension. Cells were washed by centrifugation at 1000 revolutions per minute in DMEM basal medium 3 times, and then mixed with mouse myeloma cells SP2/0 (purchased from ATCC) at a ratio of 5: 1 according to the number of viable cells. Cell fusion was performed using PEG (polyethylene glycol) fusion methods. The fused cells were diluted into DMEM medium containing 20% fetal bovine serum and 1×HAT, wherein the percentage was the mass percentage. Then the cell solution was added 1×10⁵/200 microliters per well to a 96-well cell culture plate, and put in a 5% CO₂, 37°C incubator, wherein the percentage was the volume percentage. After 14 days, ELISA and Acumen (microwell plate cell detection method) were used to screen the supernatants in cell fusion plate. The positive clones with OD450nm>1.0 in ELISA and MFI value>100 in Acumen were expanded to a 24-well plate, in the medium of DMEM (Invitrogen) containing 10% (w/w) of HT fetal bovine serum, cultured at 37°C and 5% (v/v) CO₂. After 3 days of culture, the expanded culture medium in the 24-well plate was centrifuged. The supernatant was collected for the antibody subtype analysis. The binding activity to CD47 protein and CD47 positive cells was determined by ELISA and FACS (see Example 3A and Example 3B respectively for the detection method of binding activity). The blocking activity of antibody sample on CD47 receptor was determined by ligand receptor binding experiment (or the detection method of binding activity, please refer to Example 4 respectively).

According to the results of the 24-well plate screening, hybridoma cells with OD450nm >1.0 in the ELISA experiment, with MFI value >1.0 in the FACS experiment and with the blocking inhibition rate on CD47 receptor by hybridoma cell culture supernatant reached 60% in the ligand receptor binding assay were selected as eligible positive clones. Eligible hybridoma cells were subcloned in a 96-well plate by limiting dilution, and cultured in DMEM medium containing 10% (w/w) FBS (purchased from invitrogen) under conditions of 37°C, 5% (v/v) CO₂. Ten days after subcloning, ELISA and Acumen were used for preliminary screening, and a single positive monoclone was selected and amplified into 24-well plate for further culture. After 3 days, the positive antigen binding was determined by FACS and the biological activity was evaluated by CD47 receptor ligand binding experiment (the evaluation criteria were OD450nm > 1.0 in ELISA assay, MFI value > 50 in FACS assay, and the blocking inhibition rate of hybridoma cell culture supernatant on CD47 receptor reached 60% in ligand receptor binding assay).

According to the detection results of the 24-well plate samples, the optimal clones were selected and placed and cultured in DMEM medium containing 10% (w/w) FBS (purchased from invitrogen) under conditions of 37°C, 5% (v/v) CO₂ for expanding. And the hybridoma cells of the present invention were obtained by freezing in liquid nitrogen, which can be used for subsequent antibody production and purification.

### EXAMPLE 2 Production and purification of murine antibody

The antibody concentration produced by hybridoma cells was relatively low, only about 1-10 µ g/ml, and the concentration varied greatly. Moreover, the various proteins produced by cell culture in the medium and the fetal bovine serum components contained in the medium had varying degrees of interference to many biological activity analysis methods, small-scale (1-5mg) antibody production and purification was required.

The hybridoma cells obtained in Example 1 were inoculated into a T-75 cell culture flask and production medium (Hybridoma serum free medium, purchased from Invitrogen company) was used for domestication and passage for 3 generations. When the cells grew well, they were inoculated into the cell culture spinner flask. 500mL of production medium was added to each 2 liter culture spinner flask, and the inoculated cell density was 1.0×10⁵ cells/mL. The bottle was tightly capped and placed in the spinner in the 37°C incubator at a speed of 3 rpm. After 14 days of continuous spinning culture, the cell culture fluid was collected, filtered to remove the cells, and filtered with a 0.45µm filter membrane until the culture supernatant was clarified. The clarified culture supernatant can be purified immediately or frozen at -30°C.

The monoclonal antibodies in the clarified culture supernatant (300mL) of the hybridoma cells were purified with a 2mL protein G column (purchased from GE Healthcare). The protein G column was first equilibrated with an equilibration buffer (PBS phosphate buffer, pH7.2), and then the clarified culture supernatant was loaded onto the protein G column, with a flow rate controlled at 3mL/min. After the sample was loaded, protein G column was washed with the equilibration buffer. The volume of the equilibration buffer was 4 times the volume of the protein G column bed. The CD47 antibody bound to the protein G column was eluted with the eluent (0.1M glycine hydrochloride buffer, pH2.5), and the elution was monitored with an ultraviolet detector (A280 ultraviolet absorption peak). The eluted antibodies were collected, 10% 1.0 M Tris-HCl buffer was added to neutralize pH, wherein the percentage was the volume percentage. Then immediately dialyzed with PBS phosphate buffer overnight, and the fluid was changed once on the next day and the dialysis continued for 3 hours. The dialyzed CD47 antibody was collected, aseptically filtered with a 0.22µm filter, and stored aseptically, thus obtaining purified CD47 antibody.

The purified CD47 antibody was tested and analyzed for protein concentration (A280/1. 4), purity and endotoxicity (Lonza kit), and the results were shown in Table 4.

**Table 4 Detection and analysis of purified CD47 antibodies**

| Clone number | Antibody purity | Protein concentration (mg/ml) | Endotoxin (EU/mg) |
|---|---|---|---|
| 2G9C7 | > 90% | 0.90 | 0.36 |
| 4D10B11 | > 90% | 0.88 | 0.51 |
| 25E3B5 | > 90% | 0.44 | 1.00 |
| 20H4G5 | > 90% | 1.03 | 0.43 |
| 51E2F11 | > 90% | 0.36 | 7.75 |
| 54G8G6 | > 90% | 0.68 | 2.06 |
| 92D9G2 | > 90% | 0.96 | 1.40 |
| 95B9E7 | > 90% | 0.96 | 1.17 |
| 95E2D10 | > 90% | 0.83 | 1.35 |
| 89A4H1 | > 90% | 0.69 | 1.09 |
| 95F7E5 | > 90% | 0.55 | 0.82 |
| 126G2B1 | > 90% | 0.60 | 0.56 |
| 132D1E5 | > 90% | 0.32 | 0.35 |
| 128D8D6 | > 90% | 0.77 | 0.16 |
| 158B3G6 | > 90% | 0.45 | 0.28 |
| 159E8F5 | > 90% | 0.39 | 0.32 |
| 160E9D4 | > 90% | 0.36 | 0.35 |
| 144B4E6 | > 90% | 0.59 | 1.32 |

The results showed that the final product concentration, purity and endotoxin concentration of antibody were normal.

### EXAMPLE 3 Phage display library construction and antibody screening

Spleen cells obtaining: Mice immunized with NIH3T3-hCD47, 293-hCD47 cells and pCp-hCD47 as antigens were sprint immunized with NIH3T3-hCD47, 293-hCD47 cells and hCD47-ECD-hFc protein respectively. After 3 days, the spleen cells of the mice were isolated to prepare an immune bank. The isolated spleen cells were resuspended in DMEM medium, centrifuged at 2000rpm, 4 °C for 10min, and the supernatant was discarded. Cell precipitation was lysed with RNAiso plus, and the ratio was that 1 ml RNAiso plus (purchased from Takara, Item No. 9108) was added to the spleen of a mouse, incubated at room temperature for 5min, and then stored at-80 °C.

RNA extraction: The frozen mouse spleen cells were thawed at room temperature and swirled for 5min. 0.2 ml of 1-Bromo-3-chloropropane (purchased from Sigma, catalogue number: B9673-200ml) was added to every 1ml of RNAiso plus sample, shaken violently for 15 seconds, and then incubated at room temperature for 5min. The sample was centrifuged at 4 °C, 12000g for 10min, the aqueous phase was transfered to a new tube, and 0.7 ml of isopropanol was added to precipitate RNA. After incubated at room temperature for 10min, centrifugation was performed at 12000g for 10min at 4 °C, and the supernatant was discarded. The RNA precipitate was washed once with 1ml of 75% ethanol (without RNAase), centrifuged at 12000g for 5min at 4 °C, and the supernatant was discarded. After drying the RNA precipitate for 15min, the RNA was dissolved with 40ul of DEPC-containing water (purchased from Invitrogen, 46-2224), gently mixed and left at room temperature for 5min. The RNA of all samples were mixed in half, and the concentration of the obtained RNA library was determined, and the result was 2175.6 ng/ul.

Preparation of cDNA library: The reverse transcription reaction system was prepared with reference to the reverse transcription kit 5^{∗}PrimeScriptT MRT Master Mix (purchased from Takara, catalogue number RR036A) as shown in the table below, and thermal cycling was performed. The setting conditions were 37 °C for 20min, 85 °C for 20s and 4 °C for continuous. The obtained reverse transcription products were mixed and divided into two parts. One part was stored at-80 °C and the other was stored at 4 °C for subsequent experiments.

| Reagent | Volume (ul) | Master Mix (^{∗} 3) |
|---|---|---|
| 5^{∗}Mix | 20 | 60 |
| RNA | 6.9 | 21 |
| H₂O | Supplemented to 100 | |

Amplification and purification of VH and VL libraries: primer design for amplification refers to Journal of Immunological Methods 201 (1997), 35-5. The forward and reverse amplification primers of heavy chain and light chain were mixed respectively, and the PCR reaction was prepared as follows:

| | |
|---|---|
| cDNA | 5ul |
| 2^{∗}Taq Mix (purchased from Vazyme, catalogue number P212-01/02/03) | 25ul |
| Primer Mix F (VH or VL, 100uM) | 1ul |
| Primer Mix R (VH or VL, 100uM) | 1ul |
| H₂O | 18ul |

| | |
|---|---|
| Total | 50ul |

The PCR program was set as follows:

| | |
|---|---|
| 94°C | 1min 30s |
| 94°C | 1min |
| 63°C | 30s |
| 58°C | 50s |
| 72°C | 1min |
| 94°C | 1min |
| 63°C | 1min |
| 72°C | 1min |
| 72°C | 5min |
| 4°C | Continuous |

At the end of PCR, the amplified products were purified by gel, and the obtained VH and VL libraries were assembled into scFvs by SOE (splicing overlap extension) PCR. The obtained PCR products were purified (QIAquick gel)/PCR purification kit).

Preparation of phagemids: pCAN vector and scFv were digested with Sfi enzyme (purchased from NEB, catalogue number R0123S), and the digested product was recovered by gel. The obtained pCAN vector and scFv were subjected to enzymatic linkage reaction with T4 ligase (purchased from NEB), and the obtained ligation product was purified with a purification kit (purchased from Qiagen, catalogue number: 28014) to prepare an immune phage library.

Preparation of immune phage library: 500ng of the purified DNA was mixed with 200ul of TG1 competent *Escherichia coli* and electroporation was performed. The electroporation products were shaken and cultured in 1ml YT culture for 1h at 37 °C. After 10ul of cell suspension was diluted with a 10-fold gradient (10-4, 10-5, 10-6), the storage capacity was detected by coating a plate. The remaining bacterial liquid was collected by centrifugation, and the bacterial precipitate was resuspended and coated on a flat plate, cultured overnight. The next day, the bacterial clones in the culture plate were scraped off, the precipitate was collected by centrifugation, resuspended in 4ml 2^{∗}YT medium (containing 40% glycerol), and the obtained immune library was frozen at -80 °C.

### Panning:

The His-tagged hCD47 protein was used for positive screening of the immune library obtained above. The CD47-His protein was diluted to 20 ug/ml, and 1ml was coated on four immune tubes, respectively, which were blocked with 2% MPBS at room temperature for 2 hours. The other four were directly coated with PBS as negative screening. 1 ml of phage library and 3 ml of 2% mPBS were mixed, and 1 ml of the above mixture was added to each tube for 2 hours at room temperature. The CD47-his coated tubes were emptied, the negatively selected phage library was added, the tubes were sealed with paraffin wax, and slowly shaken at room temperature for 2 hours. 40 ul of TG1 was inoculated in 4ml of 2YT culture medium and cultured overnight at 37 °C until OD600 reading exceeded 0.5. After being treated with trypsin, 1ml of liquid in the tube was transferred to 4ml of TG1 in logarithmic growth phase, and incubated at 37 °C for 30min to obtain the culture solution of TG1. The TG1 culture solution was gradiently diluted, spreaded on a plate, and incubated overnight at 37°C. The number of CD47-His bound and control tube clones were calculated, and 30 clones were randomly selected for sequencing. Then a total of three rounds of panning would be carried out. A total of 40,000 clones were obtained after the first round of panning.

Monoclones were selected from the plates of the above-mentioned panning strategy and cultured in 96-well plates, each well of which contained 200 µL 2YT medium with antibiotics, and were cultured overnight at 37 °C with shaking at 1000 rpm. 10µL of the overnight cultured supernatant was taken and added to 4mL of antibiotic-containing medium, and cultured for 1.5-2.5 hours at 37°C with shaking at 250 rpm. IPTG was added to a final concentration of ImM, and the cells were cultured with shaking at 30°C for 16 hours, and centrifuged at 4000 rpm for 10 minutes, thus obtaining the single-chain antibodies from the supernatant.

Firstly, the binding activity of the screened scFv antibody to hCD7-ECD-hFc was determined by ELISA method, and it could block the binding of CD47 to SIRPa-hFc. Some clones used FACS method to determine the binding activity of the scFv antibody to CHOK1/hCD47 and cells. Specific clones that only bound to CHOK1/hCD47 cells were selected. The binding activity of all the above-mentioned specific clones to CHOK1/cynoCD47 and CHOK1/mCD47 cells was determined by FACS method, and the still positive clones were sequenced to obtain clones with different heavy chain CDR3 sequences.

Production and purification of phage-derived lead antibody: Amplification of heavy chain and light chain variable regions: According to the sequencing results of positive clones, the variable regions of light chain and heavy chain were amplified by PCR respectively. A 50 µL reaction system was configured, including 0.5µL of plasmids extracted from the transfected positive clone *E. coli* TG1, 10pmol of each primer, 25µL of Q5 high-fidelity DNA polymerase, and water to make up to 50µL. PCR program was set, comprising pre-denaturation 95°C for 5min, denaturation 95°C for 30s, annealing 55°C for 30s, extension 68°C for 30s, and further extension at 68°C for 1 min after 25 cycles. And the PCR product was obtained. The DNA polymerase used in PCR was purchased from NEB, catalog number E0555L. 5µl of PCR product was taken for agarose gel electrophoresis detection, and the recovery kit was used to purify the positive samples. Wherein, the recovery kit was QIAquick Gel extraction kit, purchased from Qiagen, catalog number 28706.

Preparation of human IgG4 antibody: Ligation reaction was carried out: the reaction system was with a volume of 20µL, containing 3µL of fragments to be inserted, 2µL of digested expression vector, 2µL of recombinase Exnase, and 4µL of buffer, and reacted at 37°C for half an hour to obtain the ligation product, which was the constructed recombinant vector. Wherein, the recombinase was purchased from Vazyme, catalog number C112-01/02; and the buffer was the buffer used in the purchase of the recombinase. The heavy chain variable region was directionally cloned into the expression vector containing sequences encoding a signal peptide and human antibody heavy chain IgG4 (S228P) constant region (wherein, the expression vector was purchased from Invitrogen, and the recombination step was a conventional step). The light chain variable region was directionally cloned into the expression vector containing a signal peptide and the human antibody light chain kappa constant region (wherein, the expression vector was purchased from Invitrogen, and the recombination step was a conventional step). 10µL of the ligation product was added to 100µL of competent cells (Ecos 101competent cells, purchased from Yeastern, catalog number FYE607), and ice bathed for 30 minutes. Then heat shock in a 42°C water bath was performed for 90 seconds, and cells were put back on ice for 2 minutes, added with 800µL of antibiotic-free 2YT medium, and incubated on a 37°C shaker at 200 rpm for 45 minutes. Then 200µL of the culture was taken and coated onto LB solid medium containing 100µg/mL ampicillin, and cultured overnight in a 37°C incubator. The next day, the primers pTT-EF1a-F and pSV40 for the expression vector were used for configuration of a 30µL PCR system, to perform colony PCR. The colony PCR system was: 1µL of either primer, 10µL of PCR pre-mixture (purchased from Novoprotein), maked up to 20µL. A pipette tip was used to dip the colony into the PCR reaction system and pipette, and 0.5µl was aspirated onto another piece of 100µg/mL ampicillin LB solid petri dish to store the strain. After the PCR reaction, 5µL of the reaction solution was taken out for agarose gel electrophoresis detection, and the positive samples were sequenced and analyzed [see Kabat, "Sequences of Proteins of Immunological Interest," National Institutes of Health, Bethesda, Md. (1991)].

After colony PCR verification, expression vectors with the correct sequences of the recombinant antibody heavy and light chain were transiently transfected into FreeStyleTM 293-F cells (purchased from Invitrogen) to produce antibodies. Among them, clones 29A03VL and 29A03VL were used to construct mutant vectors, and the NG site of CDRL1 region was mutated into QG or NA, and the letters WT, QG or NA were added after the clone number respectively to represent. During transfection, the density of 293-F cells should be 1-1.5×10⁶ cells/mL, and 100mL of cells required 100µg of the above-mentioned constructed recombinant vector (wherein the quality ratio of the recombinant heavy chain vector and light chain vector was 2:3) and 200µg of the transfection reagent polyethyleneimine (PEI). The recombinant vector and PEI were added to 5mL culture medium respectively, and the mixture was allowed to stand at room temperature for 5 minutes. After filtration with a 0.22µm filter, the mixture of recombinant vector and PEI was allowed to stand at room temperature for 15 minutes. Then the above mixture was slowly added to the cells, and cultured in a 37°C, 8% (v/v) CO₂ incubator at 120 rpm. After 7 days, the cell culture solution was centrifuged at 3500g for 30 minutes, and the supernatant was collected and filtered with a 0.22µm filter. A 1mL protein A column (purchased from GE Healthcare) was used to purify the monoclonal antibody from 200mL of clear supernatant. The protein A column was first equilibrated with a equilibration buffer (PBS phosphate buffer, pH7.2), and then the supernatant was loaded onto the protein A column, with a flow rate controlled at 3mL/min. After the sample was loaded, protein A column was washed with the equilibration buffer. The volume of the equilibration buffer was 20 times the volume of the protein A column bed. The monoclonal antibody bound to the protein A column was eluted with the eluent (0.1M glycine hydrochloride buffer, pH3.0), and the elution was monitored with an ultraviolet detector (A280 ultraviolet absorption peak). The eluted antibody was collected, added with 10% (v/v) 1.0M Tris-HCl buffer to neutralize the pH. Then immediately dialysis was performed overnight with PBS phosphate buffer. The dialyzed monoclonal antibody was collected, aseptically filtered with a 0.22µm filter, and stored aseptically, thus obtaining purified CD47 antibody as the lead antibody. The leading antibody was tested and analyzed for protein concentration (A280/1.4), purity, and endotoxicity (Lonza kit). The results are shown in Table 6 below.

**Table 6 Detection analysis of CD47 antibody**

| Clone number | Antibody purity | Protein concentration (mg/ml) | Endotoxin (EU/mg) |
|---|---|---|---|
| 29A03 WT | > 90% | 0.40 | 0.24 |
| 29A03 QG | > 90% | 0.32 | 0.25 |
| 29A03 NA | > 90% | 0.42 | 0.20 |
| 29A04 | > 90% | 0.43 | 0.67 |
| 29G09 WT | > 90% | 0.44 | 0.31 |
| 29G09 QG | > 90% | 0.83 | 0.34 |
| 29G09 NA | > 90% | 0.39 | 0.59 |
| 31F01 | > 90% | 0.18 | 0.72 |
| 29B07 | > 90% | 0.20 | 0.36 |
| 29F06 | > 90% | 0.70 | 0.16 |
| 29G06 | > 90% | 0.60 | 0.33 |
| 31A06 | > 90% | 1.00 | 0.26 |
| 31C11 | > 90% | 1.00 | 0.04 |
| 31E01 | > 90% | 1.40 | 0.03 |

The results in Table 6 show that the antibody concentration, purity and endotoxin are all normal.

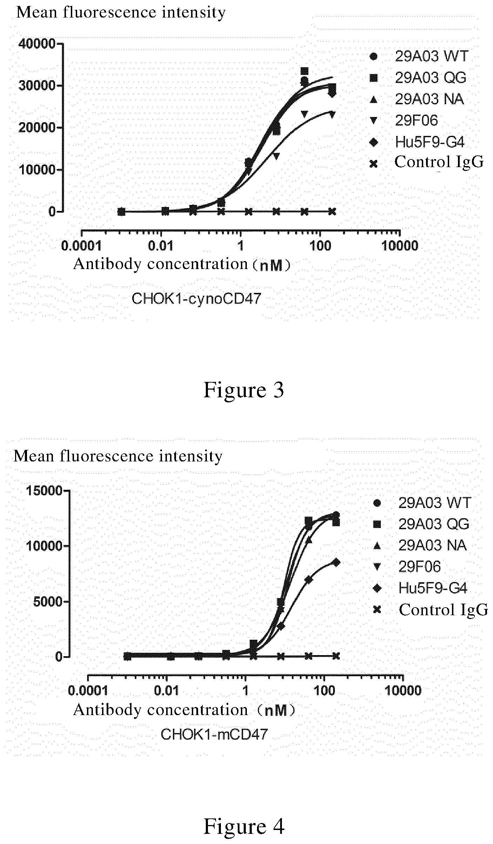

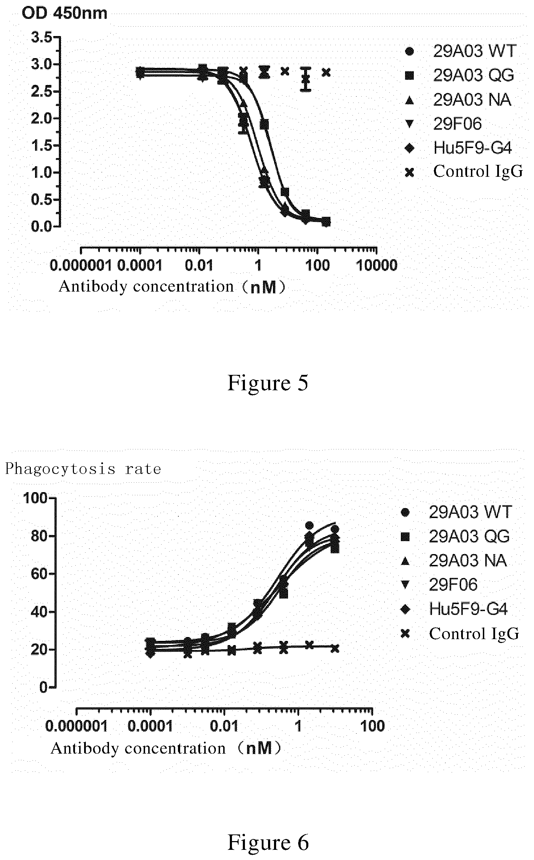

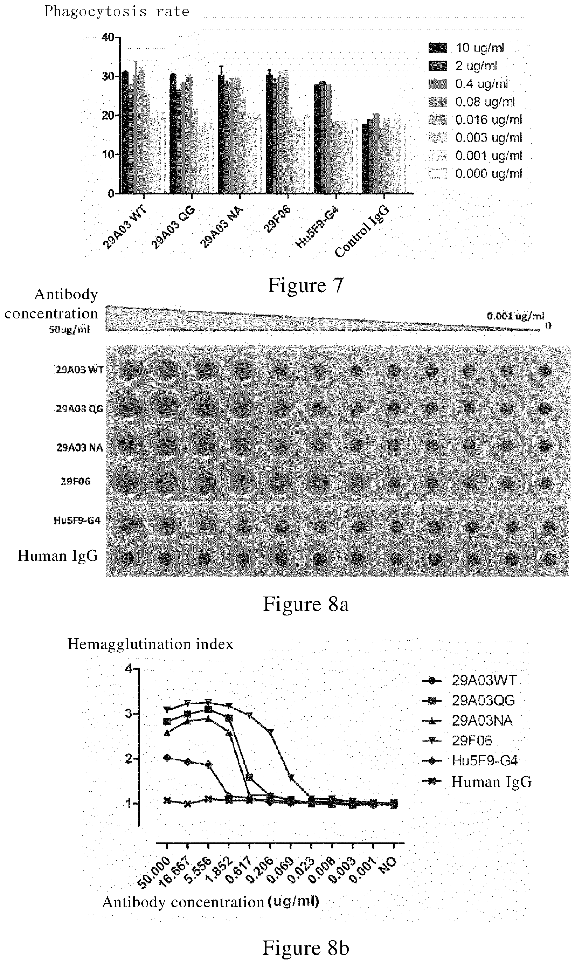

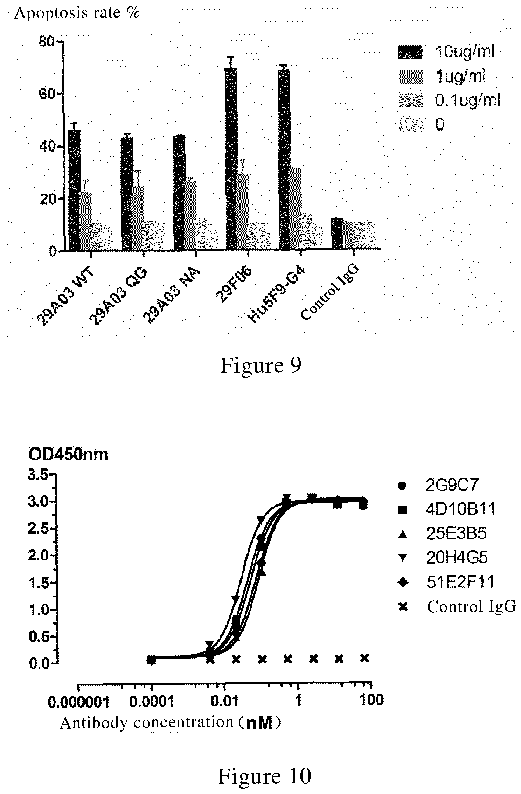

Activity identification of lead Antibody
A. The binding of antibodies to CD47-expressing cells is detected by Flow cytometry (FACS) (the method is the same as in Example 4), and the results are shown in Figure 2, Figure 3 and Figure 4. The results show that the detected antibody can bind to CD47 of human, monkey or mouse origin on the cell surface. Wherein, the control IgG is human IgG.
B. CD47 antibody blocks binding reaction of the CD47 protein to its receptor SIRPα (the method is the same as in Example 5), the results are shown in Figure 5, and it is shown that the detection antibody can block the binding of the CD47 receptor to its receptor SIRPα. Wherein, the control IgG is human IgG.
C. Phagocytosis of primary human peripheral blood macrophages on Jurkat cells was mediated by CD47 antibody (the method is the same as in Example 6), and the results are shown in Figure 6. The results show that the detected antibody can promote macrophage phagocytosis on Jurkat cells, and has a phagocytic effect similar to Hu5F9-G4.
D. Phagocytosis of mouse bone marrow macrophages on CHOK1-mCD47 was mediated by CD47 antibody
   The hind limbs of C57BL/6 mice (purchased from Slack) were removed, muscles were removed, bone marrow cells were collected, evenly spread on a 6-well plate, mouse macrophage colony stimulating factor (murine M-CSF, purchased from Peprotech) was added, and the fluid was changed every other day and stimulated for 7-10 days. Then it was digested with trypsin and inoculated into a 96-well cell culture plate. Different concentrations of CD47 antibody and CHOK1-mCD47cells labeled with CFSE (purchased from Sigma) were added in turn, and incubated at 37 °C for 4 hours. Next, the supernatant was discarded, the unphagocytized CHOK1-mCD47cells were washed away, the macrophages were digested with tyrisin, and APC-conjugated anti-mouse CD11b antibody (purchased from eBioscience) was added to label the macrophages. After incubated at 4 °C for 1 hour, washed twice with PBS, scattering and fluorescence detection of cells were carried out by flow cytometry. In the four-quadrant scatter plot, CD11b and CFSE double positive indicated macrophages that have engulfed CHOK1-mCD47, CD11b positive and CFSE negative indicated macrophages that did not phagocytosis. The ratio of the former cell number to the sum of the two is the proportion of phagocytic cells, and the percentage is expressed as the phagocytosis rate, as shown in Figure 7. Phagocytosis rate is expressed as the proportion of macrophages that phagocytize CHOK1-mCD47cells to the total macrophages. The results in Figure 7 show that CD47 antibody obtained in Example 2 can cross-react with murine CD47 and promote phagocytosis of macrophages on CHOK1-mCD47cell.
E. Hemagglutination activity of CD47 antibody (the method is the same as that of Example 7). The results are shown in Figure 8a and Figure 8b. The results show that the detected antibody is similar to Hu5F9-G4 and causes red blood cell hemagglutination.
F. Experiment of apoptosis of primary CD3+ T cell induced by CD47 antibody (the method is the same as that of Example 8), and the results are shown in Figure 9. The results show that low concentration of detected antibody does not cause apoptosis of activated T cells, and Hu5F9-G4 cause a more obvious phenomenon of T cell apoptosis.

### EXAMPLE 4 Detection of lead antibody

A. Detection of the binding activity of antibodies to CD47 protein by Enzyme-linked immunosorbent assay (ELISA)

The purified CD47 antibody obtained in Example 2 was tested for its binding reaction with human CD47-hFc protein.

His-tagged CD47 recombinant protein, CD47-His, was prepare according to that preparation method of immunogen A in Example 1, diluted to a final concentration of 1.0 µg/mL with PBS, and then added to a 96-well ELISA plate at 100 µl per well. The plate was sealed with plastic film and incubated at 4 °C overnight. The plate was washed twice with plate washing solution [PBS+0. 01% (v/v) Tween20] the next day, and blocking solution [PBS+0. 01% (v/v) Tween20 +1% (w/w) BSA] was added to block it at room temperature for 2 hours. The blocking solution was poured out and 100 µl of the purified CD47 antibody obtained in Example 2 was added per well. After incubation at 37 °C for 2 hours, the plates were washed three times with plate washing solution [PBS+0.01% (v/v) Tween 20]. HRP (horseradish peroxidase) labeled secondary antibody (purchased from Sigma) was added, incubated at 37 °C for 2 hours, and then washed three times with plate washing solution [PBS+0. 01% (v/v) Tween 20]. 100µl of TMB substrate was added to each well. After incubated at room temperature for 30 minutes, the plate was added with 100µl of stop solution (1.0N HCl) to each well. An ELISA plate reader (SpectraMax 384plus, purchased from Molecular Device) was used to read the A450nm value. The results are shown in Figure 10, and Table 7.

**Table 7 Binding reaction of CD47 antibody to human CD47-His protein detected by ELISA**

| OD450nm | Antibody concentration (nM) | | | | | | | |
|---|---|---|---|---|---|---|---|---|
| Clone number | 66.667 | 13.333 | 2.667 | 0.533 | 0.107 | 0.021 | 0.004 | 0 |
| 2G9C7 | 2.87 | 2.91 | 3.01 | 2.96 | 2.29 | 0.80 | 0.21 | 0.05 |
| 4D10B11 | 2.91 | 2.91 | 3.03 | 2.94 | 2.13 | 0.69 | 0.18 | 0.05 |
| 25E3B5 | 2.89 | 2.96 | 3.02 | 2.91 | 1.68 | 0.46 | 0.14 | 0.05 |
| 20H4G5 | 2.90 | 2.94 | 3.01 | 3.02 | 2.61 | 1.15 | 0.31 | 0.05 |
| 51E2F11 | 2.95 | 2.97 | 3.00 | 2.95 | 1.83 | 0.53 | 0.16 | 0.05 |
| 54G8G6 | 2.86 | 2.94 | 3.00 | 2.99 | 2.10 | 0.62 | 0.18 | 0.05 |
| 92D9G2 | 2.87 | 2.94 | 3.01 | 2.93 | 2.05 | 0.60 | 0.18 | 0.05 |
| 95B9E7 | 2.90 | 2.99 | 3.03 | 2.95 | 2.25 | 0.69 | 0.20 | 0.05 |
| 95E2D10 | 2.93 | 2.98 | 3.01 | 2.96 | 2.07 | 0.58 | 0.17 | 0.05 |
| 89A4H1 | 2.92 | 2.98 | 3.05 | 2.97 | 2.13 | 0.66 | 0.18 | 0.05 |
| 95F7E5 | 3.02 | 2.99 | 3.05 | 2.82 | 2.05 | 0.57 | 0.16 | 0.05 |
| 126G2B1 | 2.98 | 3.04 | 3.07 | 3.04 | 1.70 | 0.50 | 0.13 | 0.04 |
| 132D1E5 | 2.88 | 2.95 | 3.07 | 2.96 | 1.83 | 0.49 | 0.14 | 0.06 |
| 128D8D6 | 2.98 | 3.00 | 3.06 | 2.99 | 2.27 | 0.84 | 0.21 | 0.05 |
| 158B3G6 | 2.88 | 2.95 | 3.07 | 2.96 | 1.83 | 0.49 | 0.14 | 0.06 |
| 159E8F5 | 2.94 | 3.07 | 3.12 | 3.01 | 1.77 | 0.50 | 0.16 | 0.06 |
| 160E9D4 | 3.06 | 3.05 | 3.08 | 3.02 | 1.74 | 0.51 | 0.15 | 0.05 |
| 144B4E6 | 3.02 | 3.01 | 3.08 | 3.02 | 1.64 | 0.43 | 0.14 | 0.05 |
| IgG control | 0.06 | 0.05 | 0.05 | 0.05 | 0.05 | 0.05 | 0.05 | 0.06 |

Table 7 shows that the purified antibody binds to the CD47 recombinant protein at the ELISA level. Wherein, the IgG control is mouse IgG and the data in the table is OD₄₅₀ₙₘvalues.

B. Detection of the binding of antibodies to CD47 expressing cells by Flow cytometry (FACS)

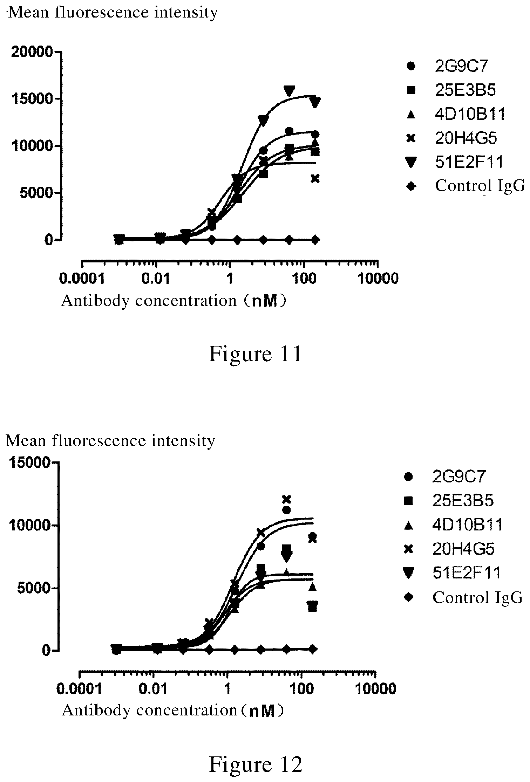

CHOK1 stable cell line containing human CD47 (herein referred to as CHOK1-hCD47 stable cell line) was obtain by transfecting the pIRES plasmid containing the nucleotide sequence encoding the full-length amino acid sequence of human CD47 described in step (2) of Example 1 into the CHOK1 cell line. The pIRES plasmid with the full-length gene of monkey-derived CD47 (which was prepared in the same way as the pCpC vector with human IgG Fc fragment (hFc) in Step (1) "Preparation of Immunogen A" of Example 1, wherein the database accession number of the amino acid sequence of the extracellular domain of the cynomolgus-derived CD47 protein (Leu25-Gln167) is B0LAJ3) was transfected into a CHOK1 cell line to obtain a CHOK1 stable cell line containing cynomolgus CD47 (herein referred to as a CHOK1-cCD47 stable cell line). The CHOK1-hCD47 stable cell lines and CHOK1-cCD47 stable cell lines were expanded to 90% confluence in a T-75 cell culture flask, the medium was exhausted, washed twice with HBSS buffer (Hanks balanced salt solution) (purchased from Invitrogen), and then treated and collected with enzyme-free cell dissociation solution (Versene solution: purchased from Life technology). The cells were washed twice with HBSS buffer. After counted, the cells were diluted with HBSS to 2×10⁶ cells per ml, added with 1% goat serum blocking solution, wherein the percentage was the mass percantage, incubated on ice for 30 minutes, and then washed twice with HBSS by centrifugation. The collected cells were suspended in the FACS buffer (HBSS +1% BSA, wherein the percentage was the mass percantage) to 2×10⁶ cells/ml, added as 100 microliters per well to a 96-well FACS reaction plate, and the purified CD47 antibody sample to be tested obtained in Example 2 was added with 100 microliters per well, incubated on ice for 2 hours. The plate was washed twice with the FACS buffer by centrifugation, added with 100 microliters of fluorescent (Alexa 488)-labeled secondary antibodies (purchased from Invitrogen) per well, and incubated on ice for 1 hour. The plate was washed 3 times with FACS buffer by centrifugation, added with 100µl fixative solution [4% (v/v) Paraformaldehyde] per well to suspend the cells. 10 minutes later, it was washed twice with FACS buffer by centrifugation. The cells were suspended with 100 microliters of FACS buffer, FACS (FACS Calibur, purchased from BD) was used for detection and the results were analyzed. The results are shown in Figure 11, Figure 12 and Tables 8-9.

**Table 8 FACS detection of the binding reaction of CD47 antibody with CHOK1-hCD47**

| Mean fluorescence intensity value | Antibody concentration (nM) | | | | | | | |
|---|---|---|---|---|---|---|---|---|
| Clone number | 200 | 40 | 8 | 1.6 | 0.32 | 0.064 | 0.0128 | 0 |
| 2G9C7 | 11224.8 | 11604.6 | 9507.4 | 5431.4 | 1431.4 | 417.2 | 115.3 | 22.5 |
| 4D10B11 | 10463.9 | 8897.6 | 8448.8 | 4854.9 | 1712.7 | 480.5 | 113.2 | 21.9 |
| 25E3B5 | 9420.9 | 9794.8 | 7020.1 | 4426.6 | 1564.3 | 386.5 | 120.5 | 22.9 |
| 20H4G5 | 6546.6 | 9524.3 | 8465.9 | 5164.4 | 2976.2 | 758.6 | 232.8 | 23.1 |
| 51E2F11 | 14592.7 | 15856.5 | 12658.1 | 6456.4 | 1999.7 | 541.3 | 174.5 | 22.6 |
| 54G8G6 | 16685.9 | 17678.7 | 13541 | 6175.8 | 1940.1 | 561.3 | 215.6 | 23.1 |
| 92D9G2 | 16396.4 | 16180.3 | 12188.1 | 4499.1 | 1911.3 | 531.5 | 101.2 | 24.8 |
| 95B9E7 | 16107 | 17280 | 12114.3 | 6192.2 | 2054.2 | 549.5 | 125.5 | 23.3 |
| 95E2D10 | 15845.5 | 17266.7 | 12339.4 | 5135.1 | 2486.5 | 408.5 | 220.8 | 23.3 |
| 89A4H1 | 16887.7 | 17702.8 | 13438.1 | 5511.9 | 1989.4 | 534.5 | 190.2 | 22.2 |
| 95F7E5 | 13101.2 | 14290.4 | 11060.7 | 6199.9 | 1807.9 | 444.8 | 121.7 | 21.5 |
| 126G2B1 | 16294.1 | 13858.4 | 11665.6 | 5827.8 | 1831.2 | 491.9 | 152.9 | 22.4 |
| 128D8D6 | 14338.3 | 14276.2 | 10918.8 | 5364.5 | 1461.3 | 403.4 | 114.7 | 23.6 |
| 132D1E5 | 7100.2 | 8231.1 | 6306.8 | 3747.6 | 1328 | 317.6 | 88.4 | 18.1 |
| 158B3G6 | 13628.2 | 12933.8 | 10921.2 | 5808.1 | 1758.7 | 416.9 | 130.5 | 22 |
| 159E8F5 | 13020.5 | 13170.9 | 11215.2 | 6001 | 1517.9 | 516.3 | 158.8 | 23.6 |
| 160E9D4 | 6880.6 | 6451.1 | 5553.1 | 3252.5 | 1060.4 | 285.1 | 88.8 | 21.9 |
| 144B4E6 | 14878.5 | 15770.9 | 11886.1 | 5246.6 | 1676.7 | 450.4 | 156.7 | 23 |
| IgG control | 30.8 | 23.9 | 22 | 22.9 | 21.6 | 21.9 | 21.4 | 20.5 |

**Table 9 FACS detection of the binding reaction of CD47 antibody with CHOK1-cCD47**

| Mean fluorescence intensity value | Antibody concentration (nM) | | | | | | | |
|---|---|---|---|---|---|---|---|---|
| Clone number | 200 | 40 | 8 | 1.6 | 0.32 | 0.064 | 0.0128 | 0 |
| 2G9C7 | 11198.7 | 13038.6 | 11823.3 | 6681.6 | 2528.5 | 630.5 | 240.3 | 75.4 |
| 4D10B11 | 8867.6 | 13944.6 | 12095.9 | 7194.2 | 2536.4 | 667 | 218.3 | 75.5 |
| 25E3B5 | 7041.2 | 9756.8 | 13221.8 | 7395 | 4545.9 | 1451.2 | 421.8 | 83.5 |
| 20H4G5 | 16529 | 13392 | 16399.9 | 9450.6 | 3313.5 | 997.7 | 343 | 81.2 |
| 51E2F11 | 9458.7 | 13348.3 | 10648.9 | 7194.5 | 2883.8 | 287.7 | 245.1 | 72.4 |
| 54G8G6 | 17191.4 | 21220.1 | 21343.5 | 9572.2 | 3248.3 | 907.7 | 277.7 | 76.8 |
| 92D9G2 | 16591.4 | 13333.6 | 9908.2 | 6657.6 | 2132.3 | 731.7 | 231.9 | 75 |
| 95B9E7 | 17251.1 | 17972.4 | 17825.3 | 7901.5 | 3185.5 | 824.5 | 225.4 | 78 |
| 95E2D10 | 21211.7 | 17452.3 | 21378.3 | 7994.6 | 3044.1 | 734.2 | 223.4 | 76 |
| 89A4H1 | 9696.3 | 9141.4 | 11935.2 | 7921.7 | 2337.8 | 687.7 | 190.5 | 74.6 |
| 95F7E5 | 8608.8 | 9250.5 | 11117.7 | 7676.6. | 2250.3 | 719.5 | 254.9 | 76.4 |
| 126G2B1 | 9620 | 7499.4 | 9920.9 | 8699.4 | 2541.4 | 946.3 | 297.6 | 110.8 |
| 128D8D6 | 9809.9 | 8557.7 | 12895.5 | 8604.1 | 2379.7 | 735.4 | 239.9 | 76.9 |
| 132D1E5 | 6965.6 | 5610.6 | 8738.9 | 6056.5 | 2062.7 | 689.4 | 224.4 | 72.5 |
| 158B3G6 | 11084 | 11740.1 | 13104.9 | 9051.4 | 2625 | 868.6 | 287.9 | 84 |
| 159E8F5 | 12659 | 9418.1 | 15215.4 | 9983.8 | 2526.8 | 838.6 | 260.9 | 75.7 |
| 160E9D4 | 6410.3 | 4540.7 | 6818.3 | 4367.8 | 1412.7 | 480.4 | 187.3 | 74.1 |
| 144B4E6 | 8980.3 | 9501.6 | 13913.1 | 7374.7 | 2296.6 | 751.5 | 196 | 77 |
| IgG control | 92.5 | 78.7 | 77.2 | 78 | 78.5 | 78 | 77.1 | 75.5 |

Tables 8-9 illustrate that the antibody to be tested can bind to the CD47 protein on the cell surface. Wherein, the IgG control is murine IgG, and the data in the table is the average fluorescence intensity values of the cell populations measured by MFI.

### EXAMPLE 5 CD47 antibody blocks the binding reaction of CD47 protein to its receptor SIRPα

The receptor ligand binding assay of CD47 protein detected that CD47 antibody blocked the binding of CD47 protein to its receptor SIRPα.

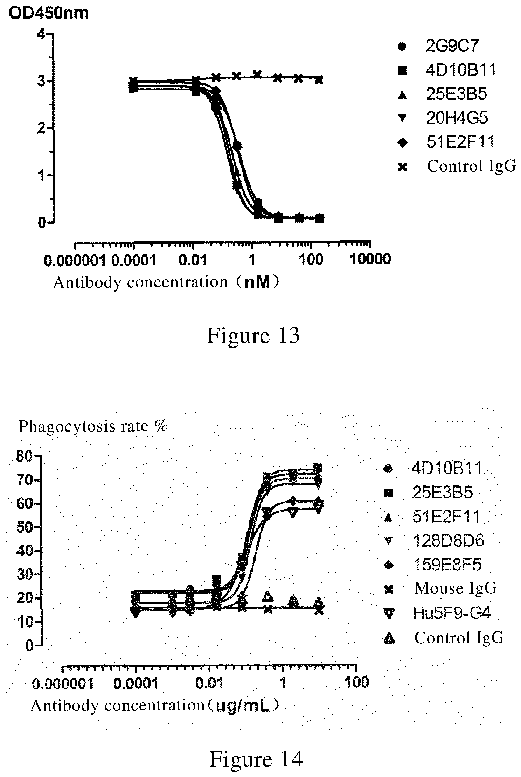

The SIRPα extracellular domain protein (SIRP-hFc) purified in Example 1 was diluted with PBS to a final concentration of 1.0 µg/mL and then added to a 96-well ELISA plate at 100 µl per well. The plate was sealed with plastic film and incubated at 4 °C overnight. The plate was washed twice with plate washing solution [PBS+0. 01% (v/v) Tween20] on the next day, and blocking solution [PBS+0. 01% (v/v) Tween20 +1% (w/w) BSA] was added to block it at room temperature for 2 hours. The blocking solution was discarded, and the plate was added with 50µl of the purified CD47 antibody sample to be tested obtained in Example 2 to each well, and then added with biotin-labeled CD47 extracellular domain protein (CD47-ECD) 50 microliters per well, mixed well. After incubation at 37 °C for 2 hours, the plate was washed three times with the plate washing solution [PBS+0. 01% (v/v) Tween 20]. HRP (horseradish peroxidase) labeled avidin diluent (purchased from Sigma) was added as 100 microliters per well, and after the plate was incubated for 2 hours at 37°C, it was washed 3 times with washing solution [PBS+0.01% (v/v) Tween 20]. 100µl of TMB substrate was added to each well. After incubated at room temperature for 30 minutes, the plate was added with 100µl of stop solution (1.0N HCl) to each well. An ELISA plate reader (SpectraMax 384plus, purchased from Molecular Device) was used to read the A450nm value. The results are shown in Table 10, and Figure 13.

**Table 10 Inhibition of CD47 antibody on binding of CD47 protein to its receptor SIRPα**

| OD450nm | Antibody concentration (nM) | | | | | | | |
|---|---|---|---|---|---|---|---|---|
| Clone number | 200 | 40 | 8 | 1.6 | 0.32 | 0.064 | 0.0128 | 0 |
| 2G9C7 | 0.07 | 0.07 | 0.10 | 0.41 | 1.63 | 2.66 | 2.87 | 2.88 |
| 4D10B11 | 0.06 | 0.06 | 0.06 | 0.15 | 0.77 | 2.45 | 2.76 | 2.84 |
| 25E3B5 | 0.06 | 0.06 | 0.07 | 0.18 | 1.06 | 2.50 | 2.79 | 2.91 |
| 20H4G5 | 0.07 | 0.07 | 0.08 | 0.16 | 0.68 | 2.29 | 2.81 | 2.90 |
| 51E2F11 | 0.07 | 0.08 | 0.10 | 0.32 | 1.57 | 2.77 | 2.94 | 2.95 |
| 54G8G6 | 0.07 | 0.06 | 0.09 | 0.13 | 0.31 | 1.76 | 2.89 | 2.90 |
| 92D9G2 | 0.06 | 0.06 | 0.17 | 0.53 | 1.64 | 2.80 | 2.94 | 2.93 |
| 95B9E7 | 0.06 | 0.09 | 0.06 | 0.14 | 0.74 | 2.45 | 2.90 | 2.91 |
| 95E2D10 | 0.06 | 0.06 | 0.09 | 0.52 | 2.00 | 2.84 | 2.96 | 2.94 |
| 89A4H1 | 0.06 | 0.09 | 0.09 | 0.20 | 1.15 | 2.59 | 2.93 | 2.94 |
| 95F7E5 | 0.06 | 0.07 | 0.11 | 0.58 | 1.93 | 2.88 | 2.92 | 2.93 |
| 126G2B1 | 0.06 | 0.07 | 0.11 | 0.41 | 1.75 | 2.84 | 2.91 | 2.94 |
| 128D8D6 | 0.09 | 0.06 | 0.08 | 0.39 | 1.64 | 2.84 | 2.96 | 2.94 |
| 132D1E5 | 0.08 | 0.08 | 0.07 | 0.16 | 0.79 | 2.55 | 2.92 | 2.89 |
| 158B3G6 | 0.07 | 0.07 | 0.07 | 0.14 | 0.67 | 2.42 | 2.89 | 2.90 |
| 159E8F5 | 0.06 | 0.06 | 0.07 | 0.17 | 0.93 | 2.63 | 2.91 | 2.90 |
| 160E9D4 | 0.06 | 0.06 | 0.08 | 0.20 | 1.08 | 2.83 | 3.00 | 2.99 |
| 144B4E6 | 0.07 | 0.06 | 0.08 | 0.15 | 0.86 | 2.62 | 2.98 | 2.98 |
| IgG control | 3.00 | 3.03 | 3.04 | 3.11 | 3.09 | 3.06 | 3.01 | 2.99 |

Table 10 and Figure 13 illustrate that CD47 antibody can block the binding of the CD47 receptor to its receptor SIRPα. Wherein, the IgG control is mouse IgG and the data in the table is OD450 values.

### EXAMPLE 6 CD47 antibody mediates phagocytosis of human peripheral blood primary macrophages on Jurkat cell

The isolated and purified human peripheral blood mononuclear cells (PBMC, purchased from AllCells) were evenly spread on a 6-well plate, and macrophage colony stimulating factor (M-CSF, purchased from peprotech) was added. The solution was changed every other day and stimulated for 7-10 days. Then it was digested with trypsin and inoculated into a 96-well cell culture plate. Different concentrations of CD47 antibody and Jurkat cells labeled with CFSE (purchased from Sigma) were added in turn, and incubated at 37 °C for 4 hours. Next, the supernatant was discarded, the unphagocytized Jurkat cells were washed away, the macrophages were digested with tyrisin, and APC-conjugated CD14 antibody (purchased from eBioscience) was added to label the macrophages. After incubated at 4 °C for 1 hour, washed twice with PBS, scattering and fluorescence detection of cells were carried out by flow cytometry. In the four-quadrant scatter plot, CD14 and CFSE double positive indicated macrophages that have engulfed Jurkat, CD14 positive and CFSE negative indicated macrophages that did not phagocytosis. The ratio of the former cell number to the sum of the two is the proportion of phagocytic cells, and the percentage is expressed as the phagocytosis rate, as shown in Figure 7. Wherein Hu5F9-G4 is the reference antibody (US9382320B2), the phagocytosis rate is expressed as the proportion of macrophages that phagocytize Jurkat cells to the total macrophages. In Figure 14, the CD47 antibody obtained from Example 2 can promote phagocytosis of macrophages on Jurkat cells, and has a phagocytic effect similar to that of Hu5F9-G4.

### EXAMPLE 7 Hemagglutination activity of CD47 antibody

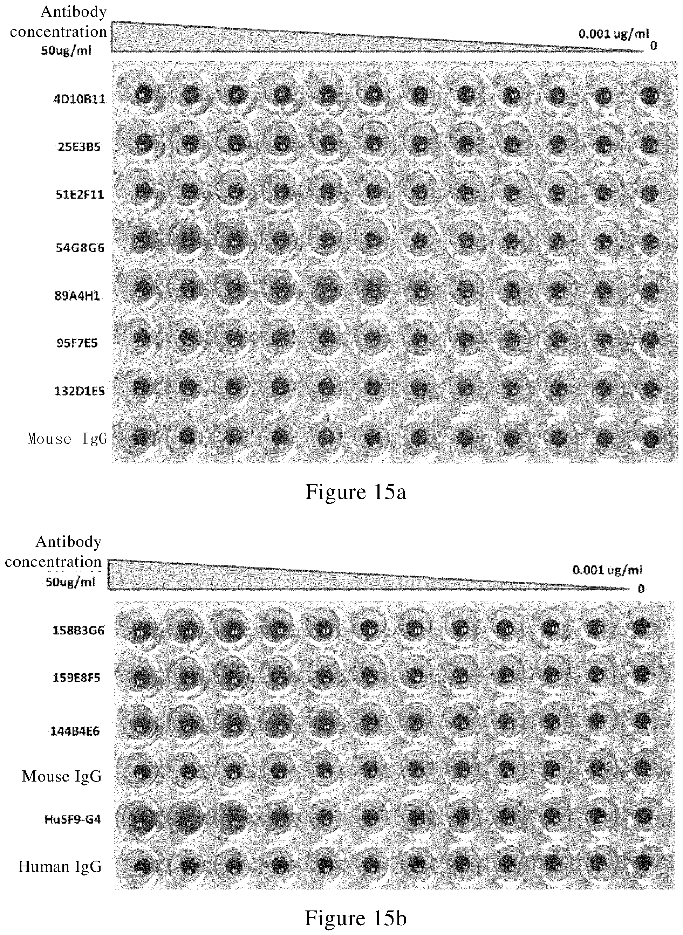

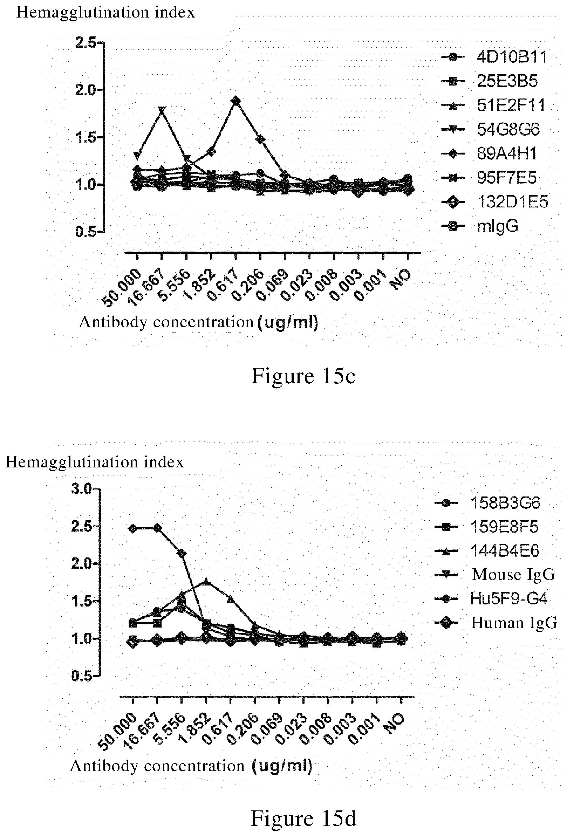

In order to evaluate the ability of CD47 antibody to initiate hemagglutination reaction, human red blood cell RBC was diluted to 5% in PBS, the same volume of CD47 antibody was added, titrated to a round bottom 96-well plate, and incubated at 37 °C for 2-4 hours. The results are shown in Figure 8a and Figure 8b. As shown in Figure 8a and Figure 8b, the presence of unfixed RBCs indicates signs of hemagglutination, and the appearance of RBCs with hemagglutination is blurred compared with the clear red dots of RBCs without hemagglutination. The quantitative results of the hemagglutination experiment are shown, and the hemagglutination index is determined by quantifying the area of RBC beads in the presence of antibodies and standardizing the data measured in the absence of antibodies. The results are shown in Figure 15a, Figure 15b, Figure 15c and Figure 15d. The results show that 54G6G8 and 89A4H1 can cause hemagglutination, while other antibodies can not cause hemagglutination.

### EXAMPLE 8 Apoptosis assay of primary CD3+ T cells induced by CD47 antibody

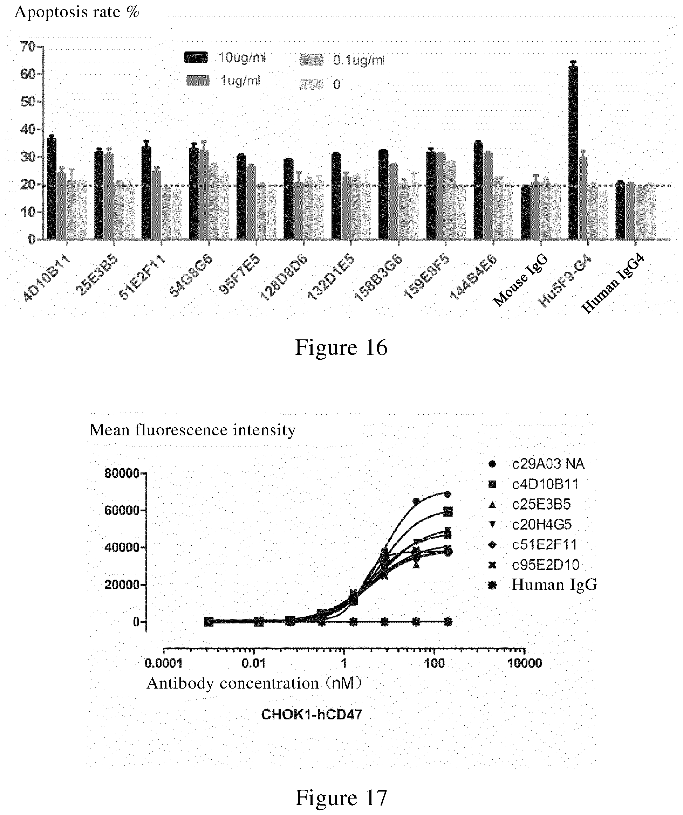

In order to evaluate the ability of CD47 antibody to induce apoptosis of normal T cells, CD3+ T in peripheral blood cells was isolated, spread on 96-well plates coated with CD47 antibody with different concentrations at 4 °C overnight, incubated at 37 °C for 20-24 hours. The cells were collected, washed twice, then labeled with Annexin-V-FITC (purchased from invitrogen) at room temperature for 10-15 minutes, and then detected apoptosis by flow cytometry. The results are shown in Figure 16. Among them, Hu5F9-G4 is the reference antibody (US9382320B2), and the apoptosis rate is the proportion of Annexin-V positive cells. The results in Figure 16 show that addition of 1 µg/ml of CD3 antibody to activate T cells when the CD47 antibody is added, it is found that Hu5F9-G4 can significantly induce apoptosis of activated T cells, while the CD47 antibody obtained in Example 2 has no significant killing effect on unactivated T cells.

### EXAMPLE 9 Amino acid sequence determination of the light and heavy chain variable region and preparation of mouse-human chimeric antibody

Isolation of total RNA: After the supernatant obtained from the subclonal culture of Example 1 was tested for antigen binding (that is, after the verification and activity determination in Examples 3-6), 5×10⁷ hybridoma cells were collected by centrifugation, added with 1mL Trizol and mixed well and transferred to a 1.5mL centrifuge tube, and allowed to stand at room temperature for 5 minutes. The tube was added with 0.2mL chloroform, shaked for 15 seconds, let stand for 2 minutes, and centrifuged at 12000g at 4°C for 5 minutes. The supernatant was taken and transferred to a new 1.5mL centrifuge tube. 0.5 mL of isopropanol was added, and the liquid in the tube was gently mixed, and let stand at room temperature for 10 minutes. After centrifuged at 12000g for 15 minutes at 4°C, the supernatant was discarded. 1 mL of 75% ethanol (the percentage was volume percentage) was added, and the precipitate was gently washed, centrifuged at 12000g at 4°C for 5 minutes. The supernatant was discarded, and the precipitate was dried, and added with DEPC-treated H₂O for dissolution (55°C water bath to promote dissolution for 10 minutes). The total RNA was obtained.

Reverse transcription and PCR: 1µg of total RNA was taken, and a 20ul system was configured, added with reverse transcriptase and reacted at 42°C for 60 minutes, and the reaction was terminated at 7°C for 10 minutes. 50µl PCR system was configured, comprising 1µl cDNA, 25pmol of each primer, 1µl DNA polymerase and a matching buffer system, 250µmol dNTPs. PCR program was set, comprising pre-denaturation 95°C 3min, denaturation 95°C 30s, annealing 55°C 30s, extension 72°C 35s, and further extension at 72°C for 5 min after 35 cycles. And the PCR product was obtained. Wherein, the kit used for reverse transcription was PrimeScript RT Master Mix, purchased from Takara, catalog number RR036; the kit used for PCR was Q5 ultra-fidelity enzyme, purchased from NEB, catalog number M0492.

Cloning and sequencing: 5µ1 of PCR product was taken for agarose gel electrophoresis detection, and the column recovery kit was used to purify the positive samples. Wherein, the recovery kit was NucleoSpin® Gel & PCR Clean-up, purchased from MACHEREY-NAGEL, catalog number 740609. Ligation reaction: 10µl of reaction system containing sample 50ng, T vector 50ng, ligase 0.5µl, and buffer 1µl was reacted for half an hour at 16°C to obtain the ligation product. Wherein, the ligation kit was T4 DNA ligase, purchased from NEB, catalog number M0402. 5µl of ligation product was taken and added into 100µl of competent cells (Ecos 101competent cells, purchased from Yeastern, catalog number FYE607) in ice bath for 5 minutes. Then heat shock was carrited out in a 42°C water bath for 1 minute, and put back on ice for 1 minute, added with 650µl of antibiotic-free SOC medium, resuscitated on a 37°C shaker at 200RPM for 30 minutes. 200µl was taken out to spread on LB solid medium containing antibiotics, and incubated overnight at 37°C in an incubator. The next day, the primers M13F and M13R on the T vector were used to configure a 30µl PCR system to perform colony PCR. A pipette tip was used to dip the colony into the PCR reaction system and pipette, and 0.5µl was aspirated onto another piece of 100nM ampicillin LB solid petri dish to save the strain. After the PCR reaction, 5µl was taken out for agarose gel electrophoresis detection, and the positive samples were sequenced. Wherein, the steps of sequencing can be found in Kabat, Sequences of Proteins of Immunological Interest, National Institutes of Health, Bethesda, Md. (1991). The sequencing results are shown in the sequence information of the present invention.

According to the sequencing results of the previous step, the sequences of the heavy chain variable region and the light chain variable region of the antibody were obtained. Production and preparation of mouse-human chimeric CD47 antibody referred to the procedure in Example 3, that is: 1, preparation of recombinant vector: the heavy chain variable region was directionally cloned into an expression vector comprising the signal peptide and the constant region of the human antibody heavy chain IgG4PE (S228P/L235E) (wherein the expression vector was purchased from Invitrogen, the recombination step was a conventional step), and the light chain variable region was directionally cloned into an expression vector comprising the signal peptide and the kappa constant region of the human antibody light chain (wherein the expression vector was purchased from Invitrogen, the recombination step was a conventional step); 2, cell transfection; 3, purification of antibody. The characteristics of the obtained CD47 chimeric antibody were identified (see Examples 4-7 for the method). Each chimeric antibody was named with the first character "c" before the corresponding lead antibody clone number, for example, the lead antibody clone number corresponding to chimeric antibody c4D10B11 is 4D10B11.

The purified CD47 antibody was tested and analyzed for protein concentration (A280/1. 11), purity and endotoxicity (Lonza kit), and the results were shown in Table 11.

**Table 11 Detection and analysis of chimeric CD47 antibody**

| Clone number | Antibody purity | Protein concentration (mg/ml) | Endotoxin (EU/mg) |
|---|---|---|---|
| c2G9C7 | > 90% | 1.19 | 1.98 |
| c4D10B11 | > 90% | 0.95 | 0.52 |
| c25E3B5 | > 90% | 1.05 | 0.27 |
| c20H4G5 | > 90% | 1.05 | 0.32 |
| c51E2F11 | > 90% | 0.91 | 0.41 |
| c54G8G6 | > 90% | 1.05 | 0.39 |
| c92D9G2 | > 90% | 0.92 | 0.78 |
| c95B8E7 | > 90% | 0.24 | 14.66 |
| c95E2D10 | > 90% | 0.88 | 0.72 |
| c89A4H1 | > 90% | 1.06 | 0.43 |
| c95F7E5 | > 90% | 1.12 | 0.34 |
| c126G2B1 | > 90% | 1.14 | 0.36 |
| c128D8D6 | > 90% | 1.07 | 0.4 |
| c132D1E5 | > 90% | 1.15 | 0.3 |
| c158B3G6 | > 90% | 1.03 | 0.3 |
| c144B4E6 | > 90% | 1.23 | 0.37 |
| c29A03NA | > 90% | 1.08 | 0.4 |

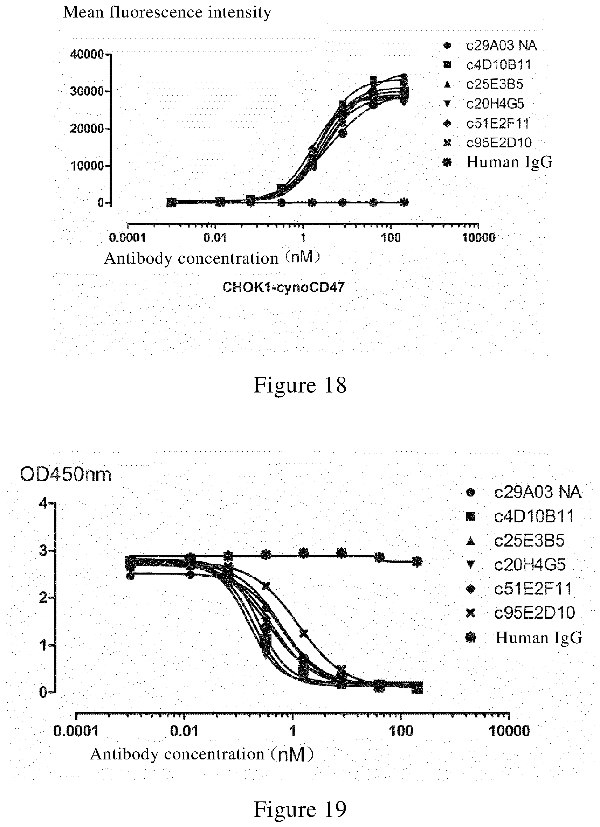

Activity identification of chimeric antibody
A. The binding of antibodies to CD47-expressing cells is detected by Flow cytometry (FACS) (the method is the same as in Example 4), and the results are shown in Figure 17, Figure 18. The results show that the chimeric antibody can bind to human and cynomolgus CD47 expressed on the cell surface.
B. Determination of the affinity constant of CD47 antibody
The affinity constant was determined using an Octet Red 96 instrument (purchased from Fortiebio). The specific operation and method shall be in accordance with the instrument instruction and detailed method provided by the manufacturer. Specifically, the affinity was determined using a streptomycin avidin sensor (SA sensor, purchased from Fortiebio). CD47 chimeric antibody was diluted to 10µg/ml with PBS solution containing 0.1% (w/w) BSA, 0.02% (v/v) Tween, pH 7.4, and then coupled with AHC sensor for reaction. The sensor bound with immunogen A was incubated with CD47-His protein diluted to 100 nM at 30 °C for 3 minutes, and then incubated with PBS solution containing 0.1% (w/v) BSA, 0.02% (v/v) Tween, pH 7.4 at 30 °C for 5 minutes. The change of interference wavelength was detected by Octet instrument to detect the binding and dissociation of antibody and immunogen A, and then the dissociation constant and binding constant were fitted by Octet ® User Software software. The affinity constant was the ratio of the dissociation constant to the binding constant. The results are shown in Table 12:

**Table 12 Affinity constant of CD47 antibody to antigen CD47-His**

| Clone number | Binding constant (1/Ms) | Dissociation constant (1/s) | Affinity constant KD (M) |
|---|---|---|---|
| c2G9C7 | 3.56E+05 | 2.46E-03 | 6.92E-09 |
| c4D10B11 | 2.68E+05 | 2.84E-04 | 1.06E-09 |
| c25E3B5 | 2.34E+05 | 8.67E-04 | 3.70E-09 |
| c20H4G5 | 2.90E+05 | 2.02E-04 | 6.95E-10 |
| c51E2F11 | 4.28E+05 | 1.03E-03 | 2.41E-09 |
| c54G8G6 | 3.43E+05 | 4.78E-04 | 1.39E-09 |
| c92D9G2 | 5.76E+05 | 2.03E-03 | 3.52E-09 |
| c95B8E7 | 5.11E+05 | 1.36E-03 | 2.67E-09 |
| c95E2D10 | 3.50E+05 | 3.58E-03 | 1.02E-08 |
| c89A4H1 | 4.02E+05 | 2.78E-03 | 6.91E-09 |
| c95F7E5 | 4.78E+05 | 3.40E-03 | 7.10E-09 |
| c126G2B1 | 4.53E+05 | 1.56E-03 | 3.44E-09 |
| c128D8D6 | 4.35E+05 | 1.47E-03 | 3.38E-09 |
| c132D1E5 | 3.69E+05 | 3.62E-04 | 9.82E-10 |
| c158B3G6 | 4.52E+05 | 8.87E-04 | 1.96E-09 |
| c144B4E6 | 3.74E+05 | 2.17E-03 | 5.81E-09 |
| c29A03NA | 4.42E+05 | 2.07E-03 | 4.69E-09 |

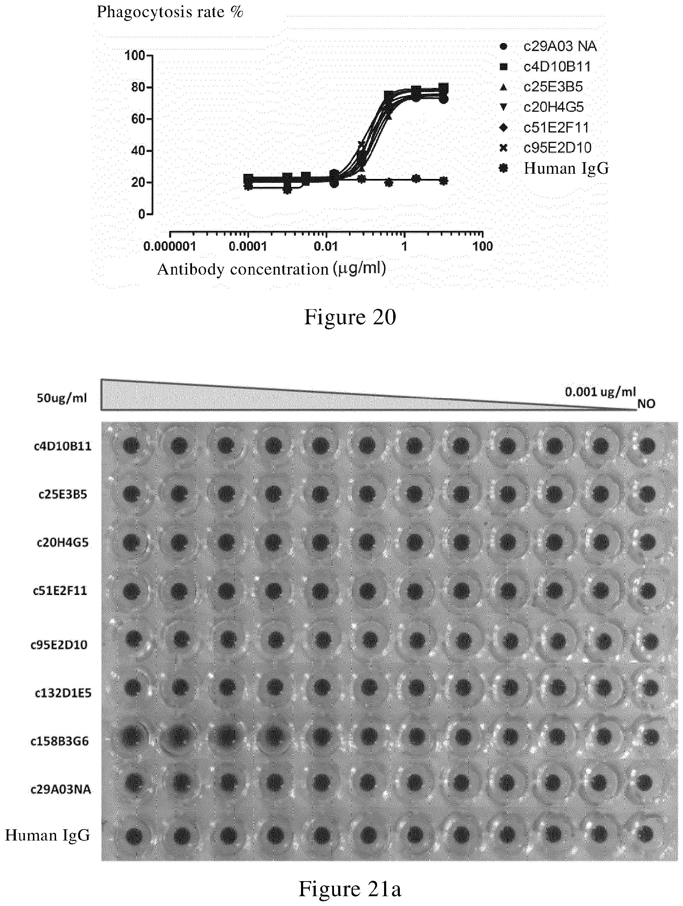

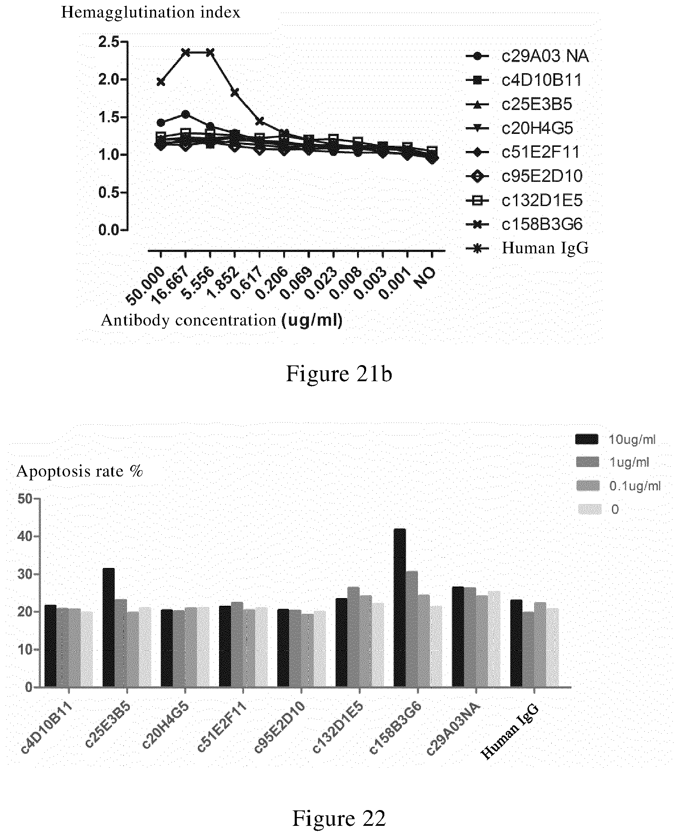

C. CD47 antibody blocks the binding reaction of the CD47 protein to its receptor SIRPα (the method is the same as in Example 5), and the results are shown in Figure 19. The results show that chimeric antibody can block the binding of CD47 protein to SIRPα.
D. CD47 antibody mediates phagocytosis of human peripheral blood primary macrophages on Jurkat cell (the method is the same as in Example 6), and the results are shown in Figure 20. The results show that chimeric antibody can promote macrophage phagocytosis on Jurkat cells.
E. Hemagglutination activity of CD47 antibody (the method is the same as in Example 7), the results are shown in Figure 21a and Figure 21b. The results show that c158B3G6 will cause erythrocyte agglutination, while other antibodies will not cause obvious erythrocyte agglutination.
F. CD47 antibody induces apoptosis of primary CD3+ T cells (the method is the same as in Example 8), and the results are shown in Figure 22. The results show that high concentration of c158B3G6 will cause apoptosis of activated T cells, while other antibodies will not cause obvious apoptosis of T cells.

### EXAMPLE 10 Antibody toxicity assay base on B-hSIRP/hCD47 humanized mouse

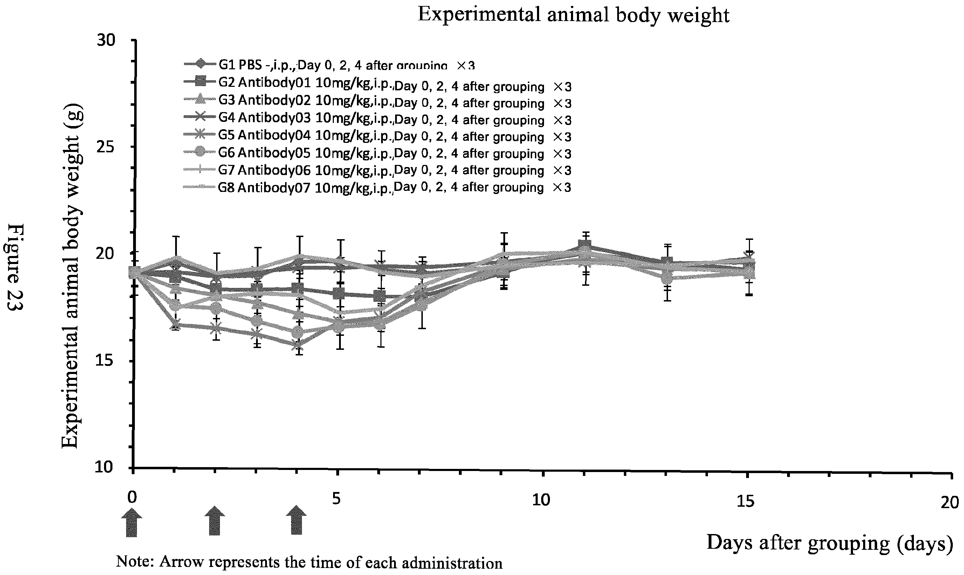

After the quarantine period, 24 mice with moderate individual weight were selected from B-hSIRP/hCD47 humanized mice (provided by Biocytogen), and the animals were randomly assigned to 8 experimental groups according to their weight, with 3 mice in each group. The route of administration in all groups was intraperitoneal injection, which was given once on the 0 th, 2nd and 4th day after grouping, for a total of 3 times. From 0 to 7 days after grouping, the body weight was measured once a day. From 9 to 15 days after grouping, the body weight was measured once every other day, and the body weight data of mice were recorded. On the 1st, 3rd, 6th and 13th day after grouping, 100-150µL of blood was collected from the inner canthus venous plexus of each mouse, and blood routine test was carried out. On the 6th day after grouping, 150-200µL blood was collected from the inner canthus venous plexus of each mouse, and serum was collected for blood biochemical detection. At the end of the experiment, the animals were euthanized and weighed. The results are shown in Table 13 and Figure 23.

**Table 13 Effect of CD47 antibody on body weight of the B-hSIRPα/hCD47 humanized mice**

| Groups | | G1 | G2 | G3 | G4 | G5 | G6 | G7 | G8 |
|---|---|---|---|---|---|---|---|---|---|
| Test drug | | PBS | c4D10B11 | c20H4G5 | c95E2D10 | c158B3G6 | c29A03NA | Hu5F9-G4 | hIgG4 |
| Weight (g) | Day 0 | 19.1±0.0 | 19.1±0.6 | 19.1±0.6 | 19.1±0.2 | 19.1±0.2 | 19.1±0.2 | 19.1±0.6 | 19.1±0.5 |
| | 1st Day | 19.6±0.1 | 18.9±0.5 | 18.4±0.5 | 19.2±0.3 | 16.7±0.1^{∗∗} | 17.6±0.1^{∗∗} | 17.5±0.4 | 19.8±0.5 |
| | 2nd Day | 19.0±0.1 | 18.3±0.3 | 18.1±0.5 | 18.9±0.1 | 16.5±0.5^{∗∗} | 17.5±0.6 | 18.0±0.5 | 19.1±0.4 |
| | 3rd day | 19.0±0.3 | 18.3±0.3 | 17.7±0.4 | 19.2±0.1 | 16.3±0.6^{∗∗} | 16.9±0.4^{∗∗} | 18.2±0.3 | 19.3±0.5 |
| | 4th Day | 19.7±0.2 | 18.4±0.5 | 17.3±0.4 | 19.4±0.1 | 15.8±0.2^{∗∗} | 16.4±0.1^{∗∗} | 18.1±0.5 | 19.9±0.5 |
| | 5th Day | 19.7±0.2 | 18.2±0.6 | 16.9±0.1^{∗} | 19.4±0.2 | 16.9±0.2^{∗∗} | 16.6±0.5^{∗} | 17.3±0.4 | 19.7±0.5 |
| | Day 6 | 19.3±0.2 | 18.1±0.4 | 16.9±0.2 | 19.5±0.2 | 17.1±0.4^{∗∗} | 16.8±0.6^{∗} | 17.5±0.2^{∗} | 19.2±0.5 |
| | Day 7 | 19.2±0.1 | 18.1±0.6 | 17.8±0.2 | 19.5±0.3 | 18.3±0.2^{∗} | 17.6±0.4^{∗} | 18.6±0.4 | 19.0±0.5 |
| | Day 9 | 19.5±0.2 | 19.2±0.7 | 19.4±0.5 | 19.7±0.1 | 19.6±0.1 | 19.6±0.2 | 20.1±0.8 | 19.6±0.6 |
| | Day 11 | 20.2±0.2 | 20.5±0.5 | 19.9±0.6 | 20.2±0.1^{∗} | 19.8±0.1 | 20.2±0.3^{∗} | 20.2±0.8 | 19.7±0.4 |
| | Day 13 | 19.7±0.2 | 19.7±0.4 | 19.6±0.4 | 19.4±0.2 | 19.4±0.1 | 19.0±0.3 | 19.5±0.6 | 19.6±0.4 |
| | Day 15 | 19.8±0.3 | 19.4±0.4 | 19.3±0.3 | 20.0±0.1 | 19.4±0.1 | 19.3±0.3 | 19.3±0.6 | 19.9±0.2 |

| | | | | | | | | | |
|---|---|---|---|---|---|---|---|---|---|
| Note: Weight is shown as mean ± standard error; ^{∗}: After the first administration, the body weight of each administration group was statistically compared with that at the first administration, t-test, P<0.05. ^{∗}: After the first administration, the body weight of each administration group was statistically compared with that at the first administration, t-test, P<0.01. | | | | | | | | | |

The results of Table 13 and Figure 23 show that the average body weight of mice in G1, G2 and G8 groups has no significant change compared with the body weight before the first administration from 0 to 15 days after grouping (P > 0.05). The average body weight of mice in G4 group increases significantly on the day 11 after grouping (P<0.05), which indicates that the experimental animals have good tolerance to c4D10B11 and c95E2D10. In addition, compared with the body weight before the first administration, the average body weight of mice in G3 group decreases significantly from 5 to 6 days after grouping (P<0.05). The average body weight of mice in the G7 group decreases significantly on the day 6 after grouping (P<0.05), indicating that the experimental animals are generally tolerant to the tested products c20H4G5 and Hu5F9-G4. In addition, during the experiment, compared with the body weight before the first administration, the average body weight of the experimental animals in the G5 and G6 groups decreased significantly from 0 to 7 days after grouping (P<0.05), and gradually recovered to the body weight before the first administration from 7 to 15 days after grouping, indicating that the experimental animals have poor tolerance to the tested products c158B3G6 and c29A03NA.

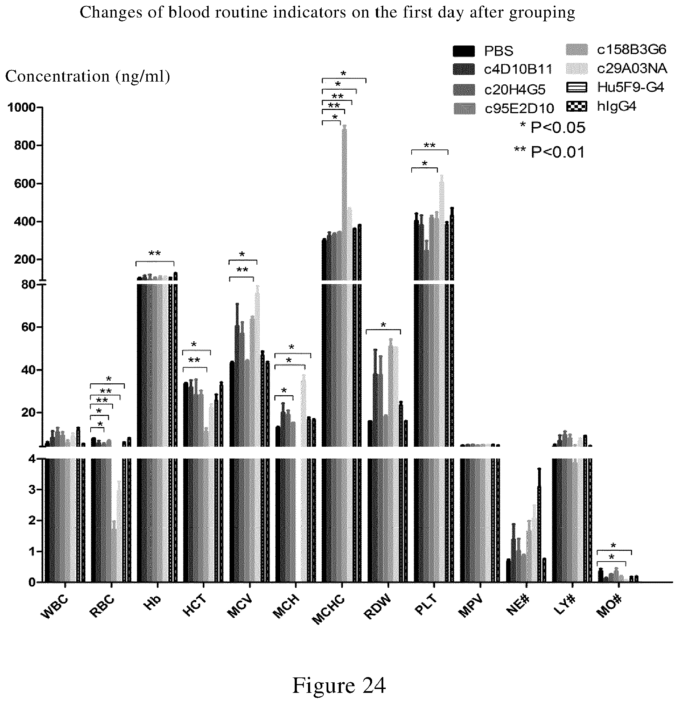

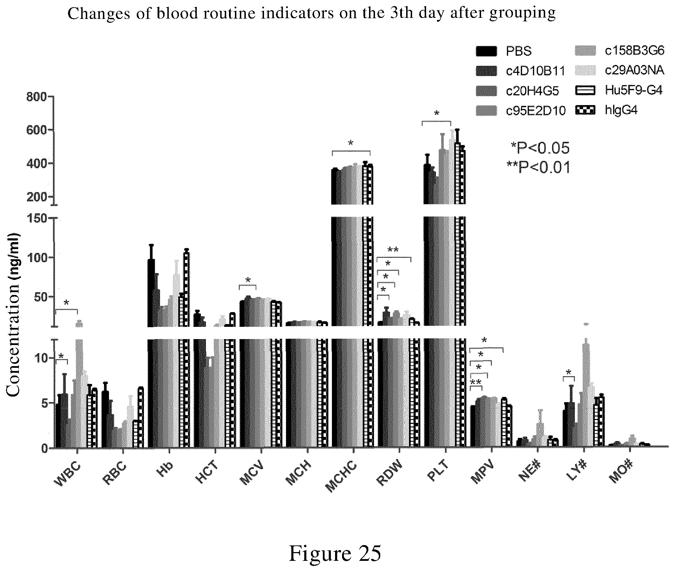

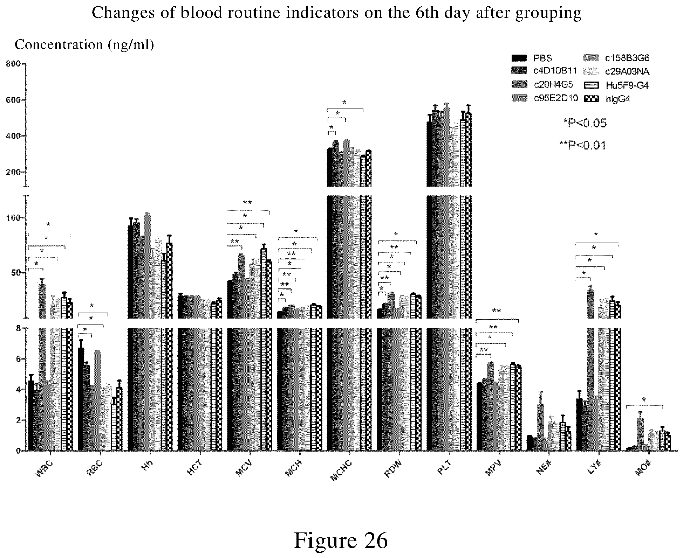

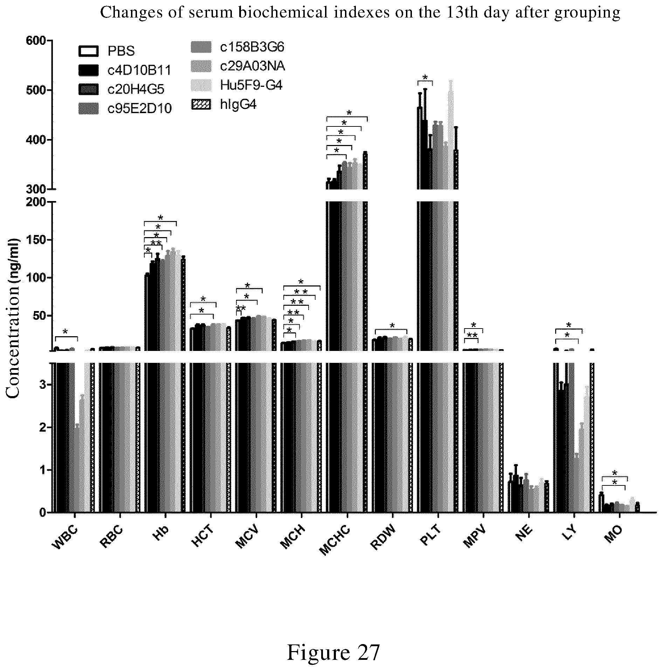

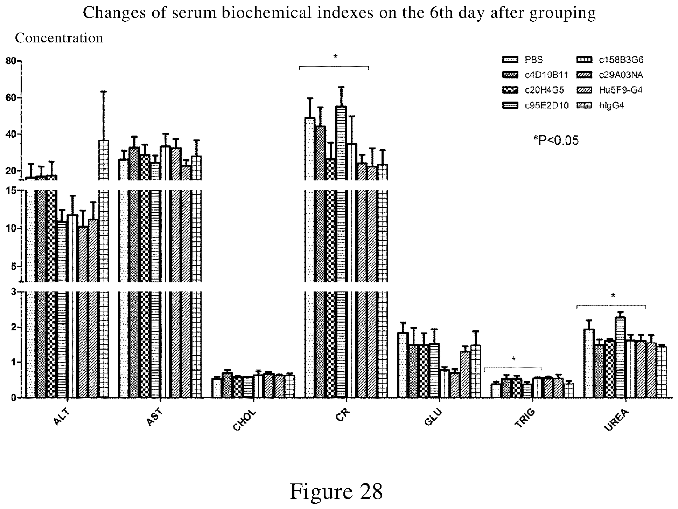

On the 1st, 3rd, 6th and 13th day after grouping, 100-150µL of blood was collected from the inner canthus venous plexus of each mouse, and blood routine test was carried out. See Figure 24, Figure 25, Figure 26 and Figure 27 for the changes of blood routine indexes. On the 6th day after grouping, 150-200µL blood was collected from the inner canthus venous plexus of each mouse, and serum was collected for blood biochemical detection. The results are shown in Figure 28. In this experimental model, the experimental animals have good tolerance to c4D10B11 and c95E2D10.

All literatures mentioned in the present application are incorporated herein by reference, as though each one is individually incorporated by reference. In addition, it should also be understood that, after reading the above teachings of the present invention, those skilled in the art can make various changes or modifications, equivalents of which falls in the scope of claims as defined in the appended claims.

### Sequence information of the present invention

**Table 14 Sequence number (SEQ ID NO.) of VH, VH-CDR1, VH-CDR2, VH-CDR3, VL, VL-CDR1, VL-CDR2, and VL-CDR3 of the antibody of the present invention**

| Clone Number | VH Sequence number | VH-CDR1 Sequence number | VH-CDR2 Sequence number | VH-CDR3 Sequence number | VL Sequence number | VL-CDR1 Sequence number | VL-CDR2 Sequence number | VL-CDR3 Sequence number |
|---|---|---|---|---|---|---|---|---|
| 4D10B11 | 1 | 3 | 4 | 5 | 6 | 8 | 9 | 10 |
| 29A03NA | 11 | 13 | 14 | 15 | 16 | 18 | 19 | 20 |
| 20H4G5 | 21 | 23 | 24 | 25 | 26 | 28 | 29 | 30 |
| 54G8G6 | 31 | 33 | 34 | 35 | 36 | 38 | 39 | 40 |
| 132D1E5 | 41 | 43 | 44 | 45 | 46 | 48 | 49 | 50 |
| 25E3B5 | 51 | 53 | 54 | 55 | 56 | 58 | 59 | 60 |
| 51E2F11 | 61 | 63 | 64 | 65 | 66 | 68 | 69 | 70 |
| 95E2D10 | 71 | 73 | 74 | 75 | 76 | 78 | 79 | 80 |
| Humab004-1 | 81 | 3 | 4 | 5 | 82 | 8 | 9 | 10 |

**Table 15 Sequence information of the present invention**

| SEQ ID NO. | Name | Sequence |
|---|---|---|
| 1 | 4D10B11 VH | QVQLQQSGPELVKPGASVKISCKASGYSFTSYYIHWVKQRPGQGLEWIG WIYPGSGNTKYNEKFKGKATLTADTSSSTADMQLSSLTSEDSAVYYCAR RGDWYFDVWGTGTTVTVSS |
| 2 | 4D10B11 VH | CAGGTCCAGCTGCAGCAGTCTGGACCTGAGCTGGTGAAGCCTGGGGC TTCAGTGAAGATATCCTGCAAGGCATCTGGCTACAGTTTCACAAGCTA CTATATACACTGGGTGAAGCAGAGGCCTGGACAGGGACTTGAGTGGA TTGGATGGATTTATCCTGGAAGTGGTAATACTAAATACAATGAGAAG TTTAAGGGCAAGGCCACACTGACGGCAGACACATCCTCCAGCACTGC CGACATGCAGCTCAGCAGCCTAACATCTGAGGACTCTGCGGTCTATT ACTGTGCAAGAAGGGGGGACTGGTACTTCGATGTCTGGGGCACAGGG ACCACGGTCACCGTCTCCTCA |
| 3 | VH-CDR1 | SYYIH |
| 4 | VH-CDR2 | WIYPGSGNTKYNEKFKG |
| 5 | VH-CDR3 | RGDWYFDV |
| 6 | 4D10B11 VL | DIVMSQSPSSLVVSVGEKVTMNCKSSQSLLFSGNQKSSLAWYQQKPGQS PKLLIYWASTRESGVPDRFTGSGSGTDFTLIISTVKAEDLAVYSCQQYYS YPLTFGAGTKLELK |
| 7 | 4D10B11 VL | GACATTGTGATGTCACAGTCTCCATCCTCCCTAGTTGTGTCAGTTGGA GAAAAGGTTACTATGAACTGCAAGTCCAGTCAGAGCCTTTTATTTAGT GGCAATCAAAAGAGTTCCTTGGCCTGGTACCAGCAGAAACCAGGGCA GTCTCCTAAACTGCTGATTTACTGGGCATCCACTAGGGAATCTGGGGT CCCTGATCGCTTCACAGGCAGTGGATCTGGGACAGATTTCACTCTCAT CATCAGCACTGTGAAGGCTGAAGACCTGGCAGTTTATTCCTGTCAGC AATATTATAGCTATCCGCTCACGTTCGGTGCTGGGACCAAACTGGAG CTGAAA |
| 8 | VL-CDR1 | KSSQSLLFSGNQKSSLA |
| 9 | VL-CDR2 | WASTRES |
| 10 | VL-CDR3 | QQYYSYPLT |
| 11 | 29A03NA VH | |
| 12 | 29A03NA VH | EVQLQQSGAELVKPGASVKMSCKASGYTFTNYWITWVKQRPGQGLEWI GDIYPVSGGTDYNEKFKTRATLTVDTSSSTAYMHLSSLTSEDSAVYYCA RKGRGGVDYWGQGTTLTVSS GAGGTGCAGCTGCAGCAGTCTGGGGCTGAGCTTGTGAAGCCTGGGGC TTCAGTGAAGATGTCCTGCAAGGCTTCTGGCTACACCTTCACCAACTA CTGGATAACCTGGGTGAAGCAGAGGCCTGGACAAGGCCTTGAGTGGA TTGGAGATATTTATCCTGTTAGTGGTGGTACTGACTACAATGAGAAGT TCAAGACCAGGGCCACACTGACTGTAGACACATCCTCCAGCACAGCC TACATGCACCTCAGCAGCCTGACATCTGAGGACTCTGCGGTCTATTAC TGTGCAAGAAAGGGACGTGGAGGAGTTGACTACTGGGGCCAAGGCA CCACTCTCACAGTCTCCTCG |
| 13 | VH-CDR1 | GYTFTNY |
| 14 | VH-CDR2 | YPVSGG |
| 15 | VH-CDR3 | KGRGGVDY |
| 16 | 29A03NA VL | |
| 17 | 29A03NA VL | DILMTQTPLSLPVSLGDQASISCRSSQSIVHSNANTYLEWYLQKPGQSPK LLIYKVSNRFSGVPDRFSGSGSGTDFTLKISRVEAEDLGVYFCSQSTHVPY TFGGGTKLEIK GATATTTTGATGACCCAGACTCCACTCTCCCTGCCTGTCAGTCTTGGA GATCAAGCCTCCATCTCTTGCAGATCTAGTCAGAGCATTGTACATAGT AATGCCAACACCTATTTAGAATGGTACCTGCAGAAACCAGGCCAGTC TCCAAAGCTCCTGATCTACAAAGTTTCCAACCGATTTTCTGGGGTCCC AGACAGGTTCAGTGGCAGTGGATCAGGGACAGATTTCACACTCAAGA TCAGCAGAGTGGAGGCTGAGGATCTGGGAGTTTATTTCTGCTCTCAA AGTACACATGTTCCGTACACGTTCGGAGGGGGGACCAAGCTGGAAAT AAAA |
| 18 | VL-CDR1 | RSSQSIVHSNANTYLE |
| 19 | VL-CDR2 | KVSNRFS |
| 20 | VL-CDR3 | SQSTHVPYT |
| 21 | 20H4G5 VH | |
| 22 | 20H4G5 VH | QVQLQQPGAELVRPGSSVRLSCVTSGYTFTSHWMHWVKQGPIKGLEWI GNIDPSDSETHYNQKFKDKATLTVDKSSSTAYMQLSRLTSDDSAVYYCA RWRHLGIGAMDYWGQGTSVIVSS CAGGTCCAACTGCAGCAGCCTGGGGCTGAACTGGTGAGGCCTGGGTC TTCAGTGAGGCTGTCCTGCGTGACTTCTGGCTACACCTTCACCAGTCA CTGGATGCATTGGGTGAAGCAGGGGCCTATAAAAGGCCTTGAATGGA TTGGTAACATTGACCCTTCTGATAGTGAGACTCACTACAATCAAAAGT TCAAGGACAAGGCCACATTGACTGTAGACAAATCCTCCAGCACAGCC TACATGCAGCTCAGCAGGCTGACATCTGACGACTCTGCGGTCTATTAC TGTGCAAGATGGCGACACCTCGGTATAGGAGCTATGGACTACTGGGG TCAAGGAACCTCAGTCATCGTCTCCTCA |
| 23 | VH-CDR1 | SHWMH |
| 24 | VH-CDR2 | NIDPSDSETHYNQKFKD |
| 25 | VH-CDR3 | WRHLGIGAMDY |
| 26 | 20H4G5 VL | |
| 27 | 20H4G5 VL | DVQITQSPSYLAASPGETITINCRASKNISKFLAWYQAKPGKSNNLLIYSG STLQSGIPSRFSGSGSGTDFTLTISRLEPEDFAMYYCQQHNEYPWTFGGG TKLEIK GATGTCCAGATAACCCAGTCTCCATCTTATCTTGCTGCATCTCCTGGA GAAACCATTACTATTAATTGCAGGGCAAGTAAGAACATTAGCAAATT TTTAGCCTGGTATCAAGCGAAACCTGGGAAAAGTAATAACCTTCTTAT |
| | | CTACTCTGGATCCACTTTGCAATCTGGAATTCCATCAAGGTTCAGTGG CAGTGGATCTGGTACAGATTTCACTCTCACCATCAGTAGACTGGAGCC TGAAGATTTTGCAATGTATTACTGTCAACAGCATAATGAATATCCATG GACGTTCGGTGGAGGCACCAAACTGGAAATCAAA |
| 28 | VL-CDR1 | RASKNISKFLA |
| 29 | VL-CDR2 | SGSTLQS |
| 30 | VL-CDR3 | QQHNEYPWT |
| 31 | 54G8G6 VH | QVQLQQPGADLVKPGASVKMSCKASGYTFTSYWIIWVKQRPGQGLEWI GDINPGRGSPNYNEKFKRKATLTVDTSSSTAYMQLSSLTSEDSAVYYCV RGGLRRFDHWGQGTLVTVSA |
| 32 | 54G8G6 VH | CAGGTCCAACTGCAGCAACCTGGGGCTGACCTTGTGAAGCCTGGGGC TTCAGTGAAAATGTCCTGCAAGGCTTCTGGCTACACCTTCACCAGTTA CTGGATAATCTGGGTGAAGCAGAGGCCTGGACAAGGCCTTGAGTGGA TTGGAGATATTAATCCTGGTCGTGGTAGTCCTAACTACAATGAGAAGT TCAAGAGGAAGGCCACACTGACTGTAGACACATCCTCCAGCACAGCC TACATGCAGCTCAGCAGCCTGACATCTGAGGACTCTGCGGTCTATTAC TGTGTAAGAGGGGGATTGCGACGGTTTGATCACTGGGGCCAAGGAC TCTAGTCACTGTCTCTGCA |
| 33 | VH-CDR1 | SYWII |
| 34 | VH-CDR2 | DINPGRGSPNYNEKFKR |
| 35 | VH-CDR3 | GGLRRFDH |
| 36 | 54G8G6 VL | ENVLTQSPAIMSASPGEKVTMTCRASSSVSSSYLHWYQQKSGASPKLWI YSTSTLASGVPARFSGSGSGTSYSLTISSVEAEDAATYYCQQYSGYPYTF GGGTKLEIK |
| 37 | 54G8G6 VL | GAAAATGTGCTCACCCAGTCTCCAGCAATCATGTCTGCATCTCCAGGG GAAAAGGTCACCATGACCTGCAGGGCCAGTTCAAGTGTAAGTTCCAG TTACTTGCACTGGTACCAGCAGAAGTCAGGTGCCTCCCCCAAACTCTG GATTTATAGCACATCCACCTTGGCTTCTGGAGTCCCTGCTCGCTTCAG TGGCAGTGGGTCTGGGACCTCTTACTCTCTCACAATCAGCAGTGTGGA GGCTGAAGATGCTGCCACTTATTACTGCCAGCAGTACAGTGGTTACCC GTACACGTTCGGAGGGGGGACCAAGCTGGAAATCAAA |
| 38 | VL-CDR1 | RASSSVSSSYLH |
| 39 | VL-CDR2 | STSTLAS |
| 40 | VL-CDR3 | QQYSGYPYT |
| 41 | 132D1E5 VH | QVQLQQPGAELVRPGSSVKLSCKASGYTFTNNWMHWVKQRPLQGLEWI GNIDPSDSETHYNQKFKDKATLTVDKSSTTAYIQLSSLTSEDSAVYYCAR WRGGGRGAMDYWGQGTSVTVSS |
| 42 | 132D1E5 VH | CAGGTCCAACTGCAGCAGCCTGGGGCTGAGCTGGTGAGGCCTGGGTC TTCAGTGAAGCTGTCCTGCAAGGCTTCTGGCTACACCTTCACCAACAA CTGGATGCATTGGGTGAAACAGAGGCCTCTACAAGGCCTTGAATGGA TTGGTAATATTGACCCTTCTGATAGTGAAACTCACTACAATCAAAAAT TCAAAGACAAGGCCACATTGACTGTAGACAAGTCCTCCACCACAGCC TACATTCAGCTCAGCAGCCTGACATCTGAGGACTCTGCGGTCTATTAC TGTGCAAGATGGAGGGGAGGTGGGAGGGGGGCTATGGACTATTGGG GTCAAGGAACCTCAGTCACCGTCTCCTCA |
| 43 | VH-CDR1 | NNWMH |
| 44 | VH-CDR2 | NIDPSDSETHYNQKFKD |
| 45 | VH-CDR3 | WRGGGRGAMDY |
| 46 | 132D1E5 VL | DVQITQSPSYLAASPGETITINCRTSKNISKFLAWYQEKPGKTNKLLIYSG STLQSGIPSRFSGSGSGTDFTLTISNLEPEDFAMYYCQQHNEYPWTFGRG TKLEIK |
| 47 | 132D1E5 VL | GATGTCCAGATAACCCAGTCTCCATCTTATCTTGCTGCATCTCCTGGA GAAACCATTACTATTAATTGCAGGACAAGTAAGAACATTAGCAAATT TTTAGCCTGGTATCAAGAGAAACCTGGGAAAACTAATAAGCTTCTTA TCTACTCTGGATCCACTTTGCAATCTGGAATTCCATCAAGGTTCAGTG GCAGTGGATCTGGTACAGATTTCACTCTCACCATCAGTAACCTGGAGC CTGAGGATTTTGCAATGTATTACTGTCAACAGCATAATGAATACCCGT GGACGTTCGGTAGAGGCACCAAGCTGGAGATCAAA |
| 48 | VL-CDR1 | RTSKNISKFLA |
| 49 | VL-CDR2 | SGSTLQS |
| 50 | VL-CDR3 | QQHNEYPWT |
| 51 | 25E3B5 VH | QVQLQQPGTALVRPGSSVKLSCKASGYTFTSYWMHWVRQRPIKGLEWI GNIDPSDSETHYNQRFKDKVTLTVDKSSNTAYMQLSSLTSEDSAVYYCA RWKYHGIGAMDYWGQGTSVTVSS |
| 52 | 25E3B5 VH | CAGGTCCAACTGCAGCAGCCTGGGACTGCGCTGGTGAGGCCTGGGTC TTCAGTGAAGCTGTCCTGCAAGGCTTCTGGCTACACCTTCACCAGCTA CTGGATGCATTGGGTGAGGCAGAGGCCTATAAAAGGCCTTGAATGGA TTGGTAACATTGACCCTTCTGATAGTGAGACTCACTACAATCAAAGAT TCAAGGACAAGGTCACATTGACTGTAGACAAATCCTCCAACACAGCC TACATGCAGCTCAGCAGCCTGACATCTGAGGACTCTGCGGTCTATTAC TGTGCAAGATGGAAATACCACGGTATAGGAGCTATGGACTACTGGGG TCAAGGAACCTCAGTCACCGTCTCCTCA |
| 53 | VH-CDR1 | SYWMH |
| 54 | VH-CDR2 | NIDPSDSETHYNQRFKD |
| 55 | VH-CDR3 | WKYHGIGAMDY |
| 56 | 25E3B5 VL | DVQITQSPSYLAASPGETVTISCRTNRNISKFLAWYQEKPGKTNKLLIYSG STLHSGIPSRFSGSGSGTDFTLTISRLEPEDFAMYYCQQHNEYPWSFGGG TKLEIK |
| 57 | 25E3B5 VL | GATGTCCAGATAACCCAGTCTCCATCTTATCTTGCTGCATCTCCTGGA GAAACCGTTACTATTAGTTGCAGGACAAATAGGAACATTAGCAAATT TTTAGCCTGGTATCAAGAGAAACCTGGGAAAACTAATAAGCTTCTTA TATACTCTGGATCCACTTTGCATTCTGGAATTCCATCAAGGTTCAGTG GCAGTGGATCTGGTACAGATTTCACTCTCACTATCAGTAGGTTGGAGC CTGAAGATTTTGCAATGTATTACTGTCAACAGCATAATGAATACCCGT GGTCGTTCGGTGGAGGCACCAAGCTGGAAATCAAA |
| 58 | VL-CDR1 | RTNRNISKFLA |
| 59 | VL-CDR2 | SGSTLHS |
| 60 | VL-CDR3 | QQHNEYPWS |
| 61 | 51E2F11 VH | EVQLQQSGAELVKPGASVKLSCTASGFNIKDHYIHWMNQRTEQGLEWI GRIDPDDGETRYAPKFRGKATLTADTSSNTAYLQFSSLTSEDTAVYHCTR ATTITRDWYFDVWGTGTTVTVSS |
| 62 | 51E2F11 VH | GAGGTTCAGCTGCAGCAGTCTGGGGCAGAGCTTGTGAAGCCAGGGGC CTCAGTCAAGTTGTCCTGCACAGCTTCTGGCTTCAACATTAAAGACCA CTATATACACTGGATGAACCAGAGGACTGAACAGGGCCTGGAGTGGA TTGGAAGGATTGATCCTGATGATGGTGAAACTAGATATGCCCCGAAA TTCCGGGGCAAGGCCACATTAACAGCAGACACATCCTCCAACACAGC |
| | | CTACCTGCAGTTCAGCAGCCTGACATCTGAGGACACTGCCGTCTATCA CTGTACTAGGGCTACGACAATAACTAGGGACTGGTACTTCGATGTCT GGGGCACAGGGACCACGGTCACCGTCTCCTCA |
| 63 | VH-CDR1 | DHYIH |
| 64 | VH-CDR2 | RIDPDDGETRYAPKFRG |
| 65 | VH-CDR3 | ATTITRDWYFDV |
| 66 | 51E2F11 VL | DIVMTQSQKFMSTSVGDRVSITCKASQNVRTSVAWYQQKPGQSPKALIY LASNRHTGIPDRFTGSGSGTDFTLTIRNVQSEDLADYFCLQHWDYPLTFG AGTKLELK |
| 67 | 51E2F11 VL | GACATTGTGATGACCCAGTCTCAAAAATTCATGTCCACATCAGTAGG AGACAGGGTCAGCATCACCTGCAAGGCCAGTCAGAATGTTCGAACTA GTGTAGCCTGGTATCAACAGAAACCTGGGCAGTCTCCTAAAGCACTG ATTTACTTGGCATCCAACCGGCACACTGGAATCCCTGATCGCTTCACA GGCAGTGGATCTGGGACAGATTTCACTCTCACCATTAGGAATGTGCA ATCTGAAGACCTGGCAGATTATTTCTGTCTGCAACATTGGGATTATCC TCTCACGTTCGGTGCTGGGACCAAGCTGGAACTGAAA |
| 68 | VL-CDR1 | KASQNVRTSVA |
| 69 | VL-CDR2 | LASNRHT |
| 70 | VL-CDR3 | LQHWDYPLT |
| 71 | 95E2D10 VH | QIQLQQSGPELVKPGASMKISCKASGYPFTDYYIHWVKQKPGQGLEWIG WIYPGSGNNKYNEKFKGKATLTVDTSPSTVYMQLSSLTSEDTAVYFCAR RAGNYFDYWGQGTTLTVSS |
| 72 | 95E2D10 VH | CAGATCCAGCTGCAGCAGTCTGGACCTGAGCTGGTGAAGCCTGGGGC TTCAATGAAGATATCCTGCAAGGCTTCTGGCTACCCCTTCACTGACTA CTATATACATTGGGTGAAGCAGAAGCCTGGACAGGGACTTGAGTGGA TTGGATGGATTTATCCTGGAAGCGGTAATAATAAGTACAATGAGAAG TTCAAGGGCAAGGCCACATTGACTGTAGACACATCCCCCAGCACAGT CTACATGCAACTCAGCAGCCTGACATCTGAGGACACTGCTGTCTATTT CTGTGCAAGACGCGCTGGAAACTACTTTGACTACTGGGGCCAAGGCA CCACTCTCACAGTCTCCTCA |
| 73 | VH-CDR1 | DYYIH |
| 74 | VH-CDR2 | WIYPGSGNNKYNEKFKG |
| 75 | VH-CDR3 | RAGNYFDY |
| 76 | 95E2D10 VL | NIVMTQSPKSMSRSVGERVTLSCRASENVRTYVFWYQQKPGLSPKLLIY GASNRYIGVPDRFTGSGSATDFTLTISSVQAEDLADYHCGQSYSYPLTFG AGTKLELK |
| 77 | 95E2D10 VL | AACATTGTAATGACCCAATCTCCCAAATCCATGTCCAGGTCAGTAGG AGAGAGGGTCACCTTGAGCTGCAGGGCCAGTGAGAATGTGCGTACTT ATGTATTCTGGTATCAACAGAAACCGGGGCTGTCTCCTAAACTGCTGA TATACGGGGCATCCAACCGGTACATTGGGGTCCCCGATCGCTTCACA GGCAGTGGATCTGCAACAGATTTCACTCTGACCATCAGCAGTGTGCA GGCTGAAGACCTTGCAGATTATCACTGTGGACAGAGTTATAGTTATCC TCTCACGTTCGGTGCTGGGACCAAGCTGGAGCTGAAA |
| 78 | VL-CDR1 | RASENVRTYVF |
| 79 | VL-CDR2 | GASNRYI |
| 80 | VL-CDR3 | GQSYSYPLT |
| 81 | Humab004-1 VH | QVQLVQSGAEVKKPGASVKVSCKASGYTFTSYYIHWVRQAPGQGLEWMG WIYPGSGNTKYNEKFKGRVTMTRDTSTSTVYMELSSLRSEDTAVYYCARRG |
| | | DWYFDVWGQGTTVTVSS |
| 82 | Humab004-1 VL | DIVMTQSPDSLAVSLGERATINCKSSQSLLFSGNQKSSLAWYQQKPGQPPKL LIYWASTRESGVPDRFSGSGSGTDFTLTISSLQAEDVAVYYCQQYYSYPLTF GQGTKLEIK |

## Claims

1. A heavy chain variable region of an antibody, wherein the heavy chain variable region has a complementarity determining region CDR selected from the group consisting of:
VH-CDR1 shown in SEQ ID NO.10n+3,
VH-CDR2 shown in SEQ ID NO.10n+4, and
VH-CDR3 shown in SEQ ID NO.10n+5;
wherein, each n is independently 0, 1, 2, 3, 4, 5, 6, or 7;
wherein any one of the above amino acid sequences further comprises a derivative sequence which is obtained through optional addition, deletion, modification and/or substitution of at least one amino acid and is capable of retaining CD47 binding affinity.

2. A heavy chain of an antibody, wherein the heavy chain has the heavy chain variable region of claim 1.

3. A light chain variable region of an antibody, wherein the light chain variable region has a complementarity determining region CDR selected from the group consisting of:
VL-CDR1 shown in SEQ ID NO. 10n +8,
VL-CDR2 shown in SEQ ID NO. 10n +9, and
VL-CDR3 shown in SEQ ID NO. 10n +10;
wherein, each n is independently 0, 1, 2, 3, 4, 5, 6, or 7;
wherein any one of the above amino acid sequences further comprises a derivative sequence which is obtained through optional addition, deletion, modification and/or substitution of at least one amino acid and is capable of retaining CD47 binding affinity.

4. A light chain of an antibody, wherein the light chain has the light chain variable region of claim 3.

5. An antibody, wherein the antibody has:
(1) the heavy chain variable region of claim 1; and/or
(2) the light chain variable region of claim 3;
or the antibody has: the heavy chain of claim 2; and/or the light chain of claim 4,
wherein any one of the above amino acid sequences further comprises a derivative sequence which is obtained through optional addition, deletion, modification and/or substitution of at least one amino acid and is capable of retaining CD47 binding affinity.

6. The antibody of claim 5, wherein the antibody has the heavy chain variable region of claim 1 and the light chain variable region of claim 3;
wherein, the heavy chain variable region and the light chain variable region comprise CDRs selected from the group consisting of:
| VH-CDR1 Sequence number | VH-CDR2 Sequence number | VH-CDR3 Sequence number | VL-CDR1 Sequence number | VL-CDR2 Sequence number | VL-CDR3 Sequence number |
|---|---|---|---|---|---|
| 3 | 4 | 5 | 8 | 9 | 10 |
| 13 | 14 | 15 | 18 | 19 | 20 |
| 23 | 24 | 25 | 28 | 29 | 30 |
| 33 | 34 | 35 | 38 | 39 | 40 |
| 43 | 44 | 45 | 48 | 49 | 50 |
| 53 | 54 | 55 | 58 | 59 | 60 |
| 63 | 64 | 65 | 68 | 69 | 70 |
| 73 | 74 | 75 | 78 | 79 | 80 |
wherein any one of the above amino acid sequences further comprises a derivative sequence which is obtained through optional addition, deletion, modification and/or substitution of at least one amino acid and is capable of retaining CD47 binding affinity.

7. The antibody of claim 5, wherein the heavy chain variable region of the antibody comprises the amino acid sequence shown in SEQ ID NO. 1, 11, 21, 31, 41, 51, 61, or 71; and/or the light chain variable region of the antibody contains the amino acid sequence shown in SEQ ID NO. 6, 16, 26, 36, 46, 56, 66, or 76.

8. The antibody of claim 6, wherein the antibody is selected from the group consisting of:
| Antibody number | Clone Number | VH Sequence number | VL Sequence number |
|---|---|---|---|
| 1 | 4D10B11 | 1 | 6 |
| 2 | 29A03NA | 11 | 16 |
| 3 | 20H4G5 | 21 | 26 |
| 4 | 54G8G6 | 31 | 36 |
| 5 | 132D1E5 | 41 | 46 |
| 6 | 25E3B5 | 51 | 56 |
| 7 | 51E2F11 | 61 | 66 |
| 8 | 95E2D10 | 71 | 76. |

9. A recombinant protein, wherein the recombinant protein comprises:
(i) the heavy chain variable region of claim 1, the heavy chain of claim 2, the light chain variable region of claim 3, the light chain of claim 4, or the antibody of any one of claims 5-8; and
(ii) an optional tag sequence that assists expression and/or purification.

10. A polynucleotide, wherein the polynucleotide encodes a polypeptide selected from group consisting of:
(1) the heavy chain variable region of claim 1, the heavy chain of claim 2, the light chain variable region of claim 3, the light chain of claim 4, or the antibody of any one of claims 5-8; and
(2) the recombinant protein of claim 9.

11. The polynucleotide of claim 10, wherein the polynucleotide encoding the heavy chain variable region is as shown in SEQ ID NO. 2, 12, 22, 32, 42, 52, 62, or 72; and/or, the polynucleotide encoding the light chain variable region is as SEQ ID NO. 7, 17, 27, 37, 47, 57, 67, or 77.

12. The polynucleotide of claim 11, wherein the polynucleotide encoding the heavy chain variable region and the polynucleotide encoding the light chain variable region are selected from the group consisting of:
| Clone Number | Sequence number of the polynucleotide encoding VH | Sequence number of the polynucleotide encoding VL |
|---|---|---|
| 4D10B11 | 2 | 7 |
| 29A03NA | 12 | 17 |
| 20H4G5 | 22 | 27 |
| 54G8G6 | 32 | 37 |
| 132D1E5 | 42 | 47 |
| 25E3B5 | 52 | 57 |
| 51E2F11 | 62 | 67 |
| 95E2D10 | 72 | 77. |

13. A vector, wherein the vector comprises the polynucleotide according to any one of claims 10-12.

14. A genetically engineered host cell, wherein the host cell contains the vector of claim 13 or the genome thereof is integrated with the polynucleotide of any one of claims 10-12.

15. An antibody conjugate, wherein the antibody conjugate comprises:
(a) an antibody moiety, which is selected from the group consisting of: the heavy chain variable region of claim 1, the heavy chain of claim 2, the light chain variable region of claim 3, the light chain of claim 4, the antibody of any one of claims 5-8, and a combination thereof; and
(b) a coupling moiety coupled to the antibody moiety, and the coupling moiety is selected from the group consisting of a detectable label, a drug, a toxin, a cytokine, a radionuclide, an enzyme, and a combination thereof.

16. An immune cell, which expresses or is exposed outside the cell membrane with the antibody of any one of claims 5-8.

17. A pharmaceutical composition, wherein the pharmaceutical composition comprises:
(i) an active ingredient, wherein the active ingredient is selected from the group consisting of: the heavy chain variable region of claim 1, the heavy chain of claim 2, the light chain variable region of claim 3, the light chain of claim 4, and the antibody of any one of claims 5-8, the recombinant protein of claim 9, the antibody conjugate of claim 15, the immune cell of claim 16, and combinations thereof; and
(ii) a pharmaceutically acceptable carrier.
